(19) Europäisches Patentamt
European Patent Office
Office européen des brevets

(11) EP 2 476 668 B1

(12) # EUROPEAN PATENT SPECIFICATION

(45) Date of publication and mention of the grant of the patent:
**06.11.2013 Bulletin 2013/45**

(21) Application number: **10815458.4**

(22) Date of filing: **10.09.2010**

(51) Int Cl.:
***C07D 213/74*** *(2006.01)* ***A61K 31/444*** *(2006.01)*
***A61P 11/00*** *(2006.01)* ***A61P 11/06*** *(2006.01)*
***A61P 43/00*** *(2006.01)*

(86) International application number:
**PCT/JP2010/065649**

(87) International publication number:
**WO 2011/030864 (17.03.2011 Gazette 2011/11)**

(54) **ANILINE COMPOUNDS**

ANILINVERBINDUNGEN

COMPOSÉS ANILINE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **11.09.2009 JP 2009210790**

(43) Date of publication of application:
**18.07.2012 Bulletin 2012/29**

(73) Proprietor: **Ube Industries, Ltd.**
**Ube-shi**
**Yamaguchi 755-8633 (JP)**

(72) Inventors:
- **IWAMURA, Ryo**
**Yamaguchi 755-8633 (JP)**
- **MURAKAMI, Yoko**
**Yamaguchi 755-8633 (JP)**
- **HAGIHARA, Masahiko**
**Yamaguchi 755-8633 (JP)**
- **OKANARI, Eiji**
**Yamaguchi 755-8633 (JP)**

(74) Representative: **Goddar, Heinz J.**
**Boehmert & Boehmert**
**Pettenkoferstrasse 20-22**
**80336 München (DE)**

(56) References cited:

| | |
|---|---|
| **WO-A1-98/27053** | **WO-A1-2009/113600** |
| **WO-A2-2008/015517** | **JP-A- 2006 519 250** |
| **JP-A- 2007 186 424** | **JP-A- 2007 515 467** |
| **JP-A- 2009 502 982** | **US-A1- 2007 049 625** |

- **CAMERON, K. 0.: 'Discovery of CP-533536: An EP2 receptor selective prostaglandin E2 (PGE2) agonist that induces local bone formation' BIOORGANIC & MEDICINAL CHEMISTRY LETTERS vol. 19, no. 7, 2009, pages 2075 - 2078, XP008153979**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### TECHNICAL FIELD

The present invention relates to a novel aniline compound or pharmaceutically acceptable salt thereof, that is useful as a pharmaceutical. More particularly, the aniline compound as related to the present invention has EP2 agonistic action and is therefore useful as a therapeutic and/or prophylactic agent for respiratory diseases such as asthma or chronic obstructive pulmonary disease (hereinafter abbreviated as COPD).

### BACKGROUND ART

Prostaglandin $E_2$ (hereinafter abbreviated as $PGE_2$), which is administered by inhalation, has been reported to inhibit immediate-type and late-type asthmatic responses in asthma patients (see Non-Patent Literature 1). In addition, $PGE_2$ is known to act as an agonist against receptors such as EP1, EP2, EP3 and EP4, and its agonistic action against EP2 receptor in particular has been suggested to be intimately involved with bronchodilatory action (see Non-Patent Literature 2).

Sulfonamide compounds, which have a structure that resembles the compound of the present invention, have been previously found to have EP2 agonistic action (see Patent Literatures 1 to 4). In particular, the compound described as Example 14e in Patent Literature 2 has been reported to increase concentration of cyclic adenosine monophosphate (hereinafter abbreviated as cAMP) due to its EP2 agonistic action, and have an action that accelerates healing of fractures (see Non-Patent Literature 3). However, there are no specific descriptions regarding bronchodilatory action based on EP2 agonistic action of these compounds described in Patent Literatures 1 to 4, and there are no specific disclosures in any of these publications regarding a sulfonamide compound related to the present invention having the pyridylaminoacetic acid or ester therof as a partial structure.

### PRIOR ART LITERATURES

[Patent Literatures]

[Patent Literature 1] WO 98/28264A
[Patent Literature 2] WO 99/19300A
[Patent Literature 3] WO 2004/078169A
[Patent Literature 4] WO 2008/015517A
[Non-Patent Literatures]

[Non-Patent Literature 1] American Journal of Respiratory and Critical Care Medicine, 159, 31(1999)
[Non-Patent Literature 2] American Journal of Physiology-Lung Cellular and Molecular Physiology, 284, L599 (2003)
[Non-Patent Literature 3] Proceedings of the National Academy of Sciences of the United States of America, 100, 6736 (2003)

### SUMMARY OF THE INVENTION

### PROBLEMS TO BE SOLVED BY THE INVENTION

The present inventors have carried out intensive studies on various sulfonamide compounds to develop a superior therapeutic agent or prophylactic agent for respiratory diseases, and as a result, they have found that a novel aniline compound having a specific structure has superior bronchodilatory action based on potent EP2 agonistic action, while also having superior properties in terms of tissue distribution, bioavailability (BA), fast-acting pharmacological effect, sustained pharmacological effect, solubility, physical stability, drug interaction, toxicity and the like, and is particularly useful as a therapeutic and/or prophylactic agent (and preferably a therapeutic agent) for respiratory diseases such as asthma or COPD, thereby leading to completion of the present invention.
The present invention is to provide a novel aniline compound or a pharmaceutically acceptable salt thereof, that has superior bronchodilatory action based on potent EP2 agonistic action, and is particularly useful as a therapeutic and/or prophylactic agent (and preferably a therapeutic agent) for respiratory diseases such as asthma or COPD.

MEANS TO SOLVE THE PROBLEMS

The "aniline compound" in the present invention means a compound represented by the following formula (I):

[Formula 1]

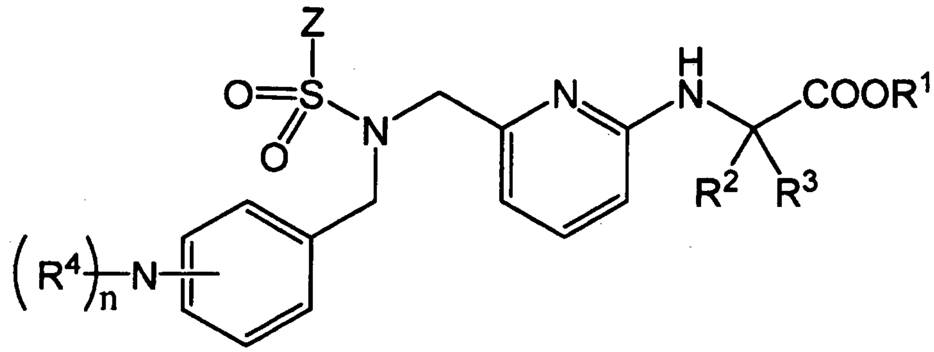

(I)

[wherein

$R^1$ represents a hydrogen atom or a $C_1$-$C_6$ alkyl group which may be substituted by a halogeno group,
$R^2$ and $R^3$ each independently represent a hydrogen atom or a $C_1$-$C_6$ alkyl group,
$R^4$ represents a hydrogen atom, or a $C_1$-$C_6$ alkyl group, a $C_2$-$C_6$ alkenyl group, a $C_2$-$C_6$ alkynyl group, a $C_3$-$C_8$ cycloalkyl group, a $C_2$-$C_6$ alkanoyl group, an arylcarbonyl group, a heteroarylcarbonyl group, a $C_1$-$C_6$ alkylsulfonyl group, an arylsulfonyl group, a heteroarylsulfonyl group, an aminosulfonyl group, a $C_1$-$C_6$ alkylaminosulfonyl group, an arylaminosulfonyl group, a heteroarylaminosulfonyl group, an aminocarbonyl group, a $C_1$-$C_6$ alkylaminocarbonyl group, an arylaminocarbonyl group, a heteroarylaminocarbonyl group, a $C_1$-$C_6$ alkoxycarbonyl group, an aryloxycarbonyl group, a heteroaryloxycarbonyl group, an aryl group, a heteroaryl group or a heterocyclic group, each of which may be substituted by the same or different 1 to 3 groups selected from the substituent group $\alpha$,
n is an integer of 2 or 3, $R^4$s may be the same or different from each other,
when n is 3, $R^4$s are $C_1$-$C_6$ alkyl groups,
Z represents a $C_1$-$C_6$ alkyl group, a $C_2$-$C_6$ alkenyl group, a $C_2$-$C_6$ alkynyl group, a $C_3$-$C_8$ cycloalkyl group, a $C_5$-$C_8$ cycloalkenyl group, an aryl group, a heteroaryl group or a heterocyclic group, each of which may be substituted by the same or different 1 to 3 groups selected from the substituent group $\alpha$,
a substituent contained in the substituent group $\alpha$ includes a hydroxy group, an oxo group, a halogeno group, a cyano group, a nitro group, a formyl group, a $C_1$-$C_6$ alkyl group, a $C_7$-$C_{12}$ aralkyl group, a $C_2$-$C_6$ alkenyl group, a $C_2$-$C_6$ alkynyl group, a $C_1$-$C_6$ alkoxy group, a $C_1$-$C_6$ alkoxy-$C_1$-$C_6$ alkyl group, a $C_7$-$C_{12}$ aralkyloxy group, a $C_3$-$C_8$ cycloalkyl group, a $C_3$-$C_8$ cycloalkyl-$C_1$-$C_6$ alkyl group, a $C_5$-$C_8$ cycloalkenyl group, a $C_2$-$C_6$ alkanoyl group, a $C_3$-$C_8$ cycloalkyl-$C_2$-$C_6$ alkanoyl group, a $C_2$-$C_6$ alkanoyl-$C_1$-$C_6$ alkyl group, an arylcarbonyl group, a heteroarylcarbonyl group, a carboxy group, a $C_1$-$C_6$ alkoxycarbonyl group, a $C_1$-$C_6$ alkylthio group, an arylthio group, a heteroarylthio group, a $C_1$-$C_6$ alkylsulfinyl group, an arylsulfinyl group, a heteroarylsulfinyl group, a $C_1$-$C_6$ alkylsulfonyl group, an arylsulfonyl group, a heteroarylsulfonyl group, an aminosulfonyl group, a $C_1$-$C_6$ alkylaminosulfonyl group, a di-($C_1$-$C_6$ alkyl)aminosulfonyl group (two alkyl groups may form a 4- to 8-membered ring in combination), an arylaminosulfonyl group, a heteroarylaminosulfonyl group, an amino group, a $C_1$-$C_6$ alkylamino group, a di-($C_1$-$C_6$ alkyl)amino group (two alkyl groups may form a 4- to 8-membered ring in combination), a tri-($C_1$-$C_6$ alkyl)ammonium group (two of three alkyl groups may form a 4- to 8-membered ring in combination), a $C_2$-$C_6$ alkenylamino group, a $C_2$-$C_6$ alkynylamino group, a $C_3$-$C_8$ cycloalkylamino group, an arylamino group, a heteroarylamino group, a $C_1$-$C_6$ alkylsulfonylamino group, an arylsulfonylamino group, a heteroarylsulfonylamino group, an aminosulfonylamino group, a $C_1$-$C_6$ alkylaminosulfonylamino group, a di-($C_1$-$C_6$ alkyl)aminosulfonylamino group (two alkyl groups may form a 4- to 8-membered ring in combination), an arylaminosulfonyl-amino group, a heteroarylaminosulfonylamino group, a $C_2$-$C_6$ alkanoylamino group, an arylcarbonylamino group, a heteroarylcarbonylamino group, an aminocarbonylamino group, a $C_1$-$C_6$ alkylaminocarbonylamino group, a di-($C_1$-$C_6$ alkyl)aminocarbonylamino group (two alkyl groups may form a 4- to 8-membered ring in combination), an aryl-aminocarbonylamino group, a heteroarylaminocarbonylamino group, a $C_1$-$C_6$ alkoxycarbonylamino group, an aryloxycarbonylamino group, a heteroaryloxycarbonylamino group, a carbamoyl group, a $C_1$-$C_6$ alkylcarbamoyl group, a di-($C_1$-$C_6$ alkyl)carbamoyl group (two alkyl groups may form a 4- to 8-membered ring in combination), an arylcarbamoyl group, a heteroarylcarbamoyl group, an aryl group, a

heteroaryl group, an aryloxy group, a heteroaryloxy group and a heterocyclic group, and
in the substituents in the substituent group $\alpha$, when it contains an alkyl group, an alkenyl group, an alkynyl group, a cycloalkyl group, a cycloalkenyl group, an aryl group, a heteroaryl group or a heterocyclic group portion, it may be further substituted by the same or different 1 to 3 substituents selected from the substituent group $\alpha$.]

or a pharmaceutically acceptable salt thereof.

EFFECTS OF THE INVENTION

The aniline compound represented by the formula (I) or a pharmaceutically acceptable salt thereof of the present invention demonstrates superior bronchodilatory action based on potent EP2 agonistic action, and also has superior properties in terms of tissue distribution, bioavailability (BA), fast-acting pharmaceutically effect, sustained pharmaceutically effect, solubility, physical stability, drug interaction, toxicity and the like. Thus, the present invention is able to provide a novel compound having superior properties as a therapeutic and/or prophylactic agent for respiratory diseases (such as asthma, COPD, bronchitis, emphysema, pulmonary fibrosis, acute respiratory distress syndrome (ARDS), cystic fibrosis and pulmonary hypertension). Moreover, the compound represented by the formula (1) of the present invention is also useful as a therapeutic and/or prophylactic agent for diseases for which EP2 agonistic action is thought to be useful (such as bone diseases, gastric ulcer, hypertension and glaucoma).

EMBODIMENT TO CARRY OUT THE INVENTION

In the compound represented by the above-mentioned formula (I), the "$C_1$-$C_6$ alkyl group" shown by the respective substituents or the "$C_1$-$C_6$ alkyl group" portion in the respective substituents each means a "$C_1$-$C_6$ alkyl group" having the same meanings, and such a "$C_1$-$C_6$ alkyl group" may be mentioned, for example, a linear or branched $C_1$-$C_6$ alkyl group such as a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a 1-methylbutyl group, a 2-methylbutyl group, a 1-ethylpropyl group, a 1,2-dimethylpropyl group, a hexyl group, a 1-methylpentyl group, a 2-methylpentyl group, a 3-methylpentyl group, a 4-methylpentyl group, a 1-ethylbutyl group, a 2-ethylbutyl group, a 1,1-dimethylbutyl group, a 1,2-dimethylbutyl group, a 1,3-dimethylbutyl group, a 2,2-dimethylbutyl group, a 2,3-dimethylbutyl group, a 3,3-dimethylbutyl group, a 1-ethyl-1-methylpropyl group, a 1-ethyl-2-methylpropyl group, a 1,1,2-trimethylpropyl group or a 1,2,2-trimethylpropyl group, etc., preferably a $C_1$-$C_4$ alkyl group, more preferably a methyl group, an ethyl group, a propyl group, an isopropyl group, a butyl group or a tert-butyl group, and particularly preferably a methyl group, an ethyl group, a propyl group or a butyl group.

The "$C_2$-$C_6$ alkenyl group" shown by the respective substituents or the "$C_2$-$C_6$ alkenyl group" portion in the respective substituents each means a "$C_2$-$C_6$ alkenyl group" having the same meanings, and such a "$C_2$-$C_6$ alkenyl group" may be mentioned, for example, a linear or branched $C_2$-$C_6$ alkenyl group such as a vinyl group, a 1-propenyl group, a 2-propenyl group, an isopropenyl group, a 1-butenyl group, a 2-butenyl group, a 3-butenyl group, a 1-methyl-1-propenyl group, a 2-methyl-1-propenyl group, a 1-methyl-2-propenyl group, a 2-methyl-2-propenyl group, a 1-ethylvinyl group, a 1-pentenyl group, a 2-pentenyl group, a 3-pentenyl group, a 4-pentenyl group, a 1-methyl-1-butenyl group, a 2-methyl-1-butenyl group, a 3-methyl-1-butenyl group, a 1-methyl-2-butenyl group, a 2-methyl-2-butenyl group, a 3-methyl-2-butenyl group, a 1-methyl-3-butenyl group, a 2-methyl-3-butenyl group, a 3-methyl-3-butenyl group, a 1-ethyl-1-propenyl group, a 1-ethyl-2-propenyl group, a 1,1-dimethyl-2-propenyl group, a 1-hexenyl group, a 2-hexenyl group, a 3-hexenyl group, a 4-hexenyl group, a 5-hexenyl group, a 1-methyl-1-pentenyl group, a 2-methyl-1-pentenyl group, a 3-methyl-1-pentenyl group, a 4-methyl-1-pentenyl group, a 1-methyl-2-pentenyl group, a 2-methyl-2-pentenyl group, a 3-methyl-2-pentenyl group, a 4-methyl-2-pentenyl group, a 1-methyl-3-pentenyl group, a 2-methyl-3-pentenyl group, a 3-methyl-3-pentenyl group, a 4-methyl-3-pentenyl group, a 1-methyl-4-pentenyl group, a 2-methyl-4-pentenyl group, a 3-methyl-4-pentenyl group, a 4-methyl-4-pentenyl group, a 1-ethyl-1-butenyl group, a 2-ethyl-1-butenyl group, a 1-ethyl-2-butenyl group, a 2-ethyl-2-butenyl group, a 1-ethyl-3-butenyl group, a 2-ethyl-3-butenyl group, a 1,1-dimethyl-2-butenyl group and a 1,1-dimethyl-3-butenyl group, etc., preferably a $C_3$-$C_5$ alkenyl group, more preferably a 1-propenyl group, a 2-propenyl group, an isopropenyl group, a 1-butenyl group, a 2-butenyl group, a 3-butenyl group or a 2-methyl-1-propenyl group, and particularly preferably a 1-propenyl group, a 2-propenyl group or a 1-butenyl group.

The "$C_2$-$C_6$ alkynyl group" shown by the respective substituents each means a "$C_2$-$C_6$ alkynyl group" having the same meanings, and such a "$C_2$-$C_6$ alkynyl group" may be mentioned, for example, a linear or branched $C_2$-$C_6$ alkynyl group such as an ethynyl group, a 1-propynyl group, a 2-propynyl group, a 1-butynyl group, a 2-butynyl group, a 3-butynyl group, a 1-methyl-2-propynyl group, a 1-pentynyl group, a 2-pentynyl group, a 3-pentynyl group, a 4-pentynyl group, a 3-methyl-1-butynyl group, a 1-methyl-2-butynyl group, a 1-methyl-3-butynyl group, a 2-methyl-3-butynyl group, a 1-ethyl-2-propynyl group, a 1-hexynyl group, a 2-hexynyl group, a 3-hexynyl group, a 4-hexynyl group, a 5-hexynyl group, a 3-methyl-1-pentynyl group, a 4-methyl-1-pentynyl group, a 1-methyl-2-pentynyl group, a 4-methyl-2-pentynyl

group, a 1-methyl-3-pentynyl group, a 2-methyl-3-pentynyl group, a 1-methyl-4-pentynyl group, a 2-methyl-4-pentynyl group, a 3-methyl-4-pentynyl group, a 3,3-dimethyl-1-butynyl group, a 1,1-dimethyl-2-butynyl group, a 1-ethyl-2-butynyl group, a 1,1-dimethyl-3-butynyl group, a 1,2-dimethyl-3-butynyl group, a 2,2-dimethyl-3-butynyl group, a 1-ethyl-3-butynyl group, a 2-ethyl-3-butynyl group, a 1-propyl-2-propynyl group, a 1-isopropyl-2-propynyl group and a 1-ethyl-1-methyl-2-propynyl group, etc., preferably a $C_3$-$C_5$ alkynyl group, more preferably a 2-propynyl group, a 2-butynyl group, a 3-butynyl group, a 2-pentynyl group, a 3-pentynyl group or a 4-pentynyl group, and particularly preferably a 2-propynyl group or a 2-butynyl group.

The "$C_3$-$C_8$ cycloalkyl group" shown by the respective substituents or the "$C_3$-$C_8$ cycloalkyl group" portion in the respective substituents each means a "$C_3$-$C_8$ cycloalkyl group" having the same meanings, and such a "$C_3$-$C_8$ cycloalkyl group" may be mentioned, for example, a $C_3$-$C_8$ cycloalkyl group such as a cyclopropyl group, a cyclobutyl group, a cyclopentyl group, a cyclohexyl group, a cycloheptyl group or a cyclooctyl group, etc., preferably a $C_3$-$C_6$ cycloalkyl group, more preferably a cyclopropyl group, a cyclobutyl group or a cyclopentyl group, and particularly preferably a cyclopropyl group or a cyclobutyl group.

The "$C_5$-$C_8$ cycloalkenyl group" shown by the respective substituents each means a "$C_5$-$C_8$ cycloalkenyl group" having the same meanings, and such a "$C_5$-$C_8$ cycloalkenyl group" may be mentioned, for example, a $C_5$-$C_8$ cycloalkenyl group such as a cyclopentenyl group, a cyclopenten-3-yl group, a cyclohexenyl group, a cyclohexen-3-yl group, a cyclohexen-4-yl group, a cycloheptenyl group, a cyclohepten-3-yl group, a cyclohepten-4-yl group, a cyclohepten-5-yl group, a cycloctenyl group, a cyclocten-3-yl group, a cyclocten-4-yl group or a cyclocten-5-yl group, etc., preferably a $C_5$-$C_6$ cycloalkenyl group, more preferably a cyclopentenyl group, a cyclopenten-3-yl group or a cyclohexenyl group, and particularly preferably a cyclopentenyl group or a cyclohexenyl group.

The "$C_1$-$C_6$ alkoxy group" shown by the respective substituents or the "$C_1$-$C_6$ alkoxy group" portion in the respective substituents each means a "$C_1$-$C_6$ alkoxy group" having the same meanings, and such a "$C_1$-$C_6$ alkoxy group" may be mentioned, for example, a linear or branched $C_1$-$C_6$ alkoxy group such as a methoxy group, an ethoxy group, a propoxy group, an isopropoxy group, a butoxy group, an isobutoxy group, a sec-butoxy group, a tert-butoxy group, a pentyloxy group, an isopentyloxy group, a neopentyloxy group, a tert-pentyloxy group, a 1-methylbutoxy group, a 2-methylbutoxy group, a 1-ethylpropoxy group, a 1,2-dimethylpropoxy group, a hexyloxy group, a 1-methylpentyloxy group, a 2-methylpentyloxy group, a 3-methylpentyloxy group, a 4-methylpentyloxy group, a 1-ethylbutoxy group, a 2-ethylbutoxy group, a 1,1-dimethylbutoxy group, a 1,2-dimethylbutoxy group, a 1,3-dimethylbutoxy group, a 2,2-dimethylbutoxy group, a 2,3-dimethylbutoxy group, a 3,3-dimethylbutoxy group, a 1-ethyl-1-methylpropoxy group, a 1-ethyl-2-methylpropoxy group, a 1,1,2-trimethylpropoxy group or a 1,2,2-trimethylpropoxy group, etc., preferably a $C_1$-$C_4$ alkoxy group, more preferably a methoxy group, an ethoxy group, a propoxy group or an isopropoxy group, and particularly preferably a methoxy group.

The "$C_2$-$C_6$ alkanoyl group" shown by the respective substituents or the "$C_2$-$C_6$ alkanoyl group" portion in the respective substituents each means a "$C_2$-$C_6$ alkanoyl group" having the same meanings, and such a "$C_2$-$C_6$ alkanoyl group" may be mentioned, for example, a linear or branched $C_2$-$C_6$ alkanoyl group such as an acetyl group, a propanoyl group, a butanoyl group, a 2-methylpropanoyl group, a pentanoyl group, a 2-methylbutanoyl group, a 3-methylbutanoyl group, a pivaloyl group, a hexanoyl group, a 2-methylpentanoyl group, a 3-methylpentanoyl group, a 4-methylpentanoyl group, a 2-ethylbutanoyl group, a 2,2-dimethylbutanoyl group, a 2,3-dimethylbutanoyl group or a 3,3-dimethylbutanoyl group, etc., preferably a $C_2$-$C_4$ alkanoyl group, and more preferably an acetyl group, a propanoyl group or a butanoyl group.

The "$C_7$-$C_{12}$ aralkyl group" in the substituent group $\alpha$ may be mentioned, for example, a benzyl group, a 1-phenylethyl group, a 2-phenylethyl group, a 1-phenylpropyl group, a 2-phenylpropyl group, a 3-phenylpropyl group, a phenylbutyl group, a phenylpentyl group, a phenylhexyl group, a naphthalen-1-ylmethyl group, a naphthalen-2-ylmethyl group, a 1-(naphthalen-1-yl)ethyl group, a 2-(naphthalen-1-yl)ethyl group, a 1-(naphthalen-2-yl)ethyl group or a 2-(naphthalen-2-yl)ethyl group, etc., preferably a benzyl group, a 1-phenylethyl group, a 2-phenylethyl group or a 1-methyl-2-phenylethyl group, more preferably a benzyl group, a 1-phenylethyl group or a 2-phenylethyl group, and particularly preferably a benzyl group or a 2-phenylethyl group.

The "$C_7$-$C_{12}$ aralkyloxy group" in the substituent group $\alpha$ may be mentioned, for example, benzyloxy group, a 1-phenylethyloxy group, a 2-phenylethyloxy group, a 1-phenylpropyloxy group, a 2-phenylpropyloxy group, a 3-phenylpropyloxy group, a phenylbutyloxy group, a phenylpentyloxy group, a phenylhexyloxy group, a naphthalen-1-ylmethyloxy group, a naphthalen-2-ylmethyloxy group, a 1-(naphthalen-1-yl)-ethyloxy group, a 2-(naphthalen-1-yl)ethyloxy group, a 1-(naphthalen-2-yl)ethyloxy group or a 2-(naphthalen-2-yl)ethyloxy group, etc., preferably a benzyloxy group, a 1-phenylethyloxy group, a 2-phenylethyloxy group or a 1-methyl-2-phenylethyloxy group, more preferably a benzyloxy group, a 1-phenylethyloxy group or a 2-phenylethyloxy group, and particularly preferably a benzyloxy group or a 2-phenylethyloxy group.

The "aryl group" shown by the respective substituents or the "aryl group" portion in the respective substituents each means an "aryl group" having the same meanings, and such an "aryl group" may be mentioned, for example, a phenyl group or a naphthyl group, etc., and preferably a phenyl group.

The "heteroaryl group" shown by the respective substituents or the "heteroaryl group" portion in the respective

substituents each means a "heteroaryl group" having the same meanings, and such a "heteroaryl group" may be mentioned, for example, a pyrrolyl group, a furyl group, a thienyl group, a pyrazolyl group, an imidazolyl group, an oxazolyl group, a thiazolyl group, a pyridyl group, a pyridazinyl group, a pyrimidinyl group, a pyradinyl group, a benzofuryl group, a benzothienyl group, a benzoxazolyl group, a benzothiazolyl group, an isoindolyl group, an indolyl group, an indazolyl group, a benzimidazolyl group, an isoquinolyl group or a quinolyl group, etc., preferably a thienyl group, a pyrazolyl group, an imidazolyl group, a thiazolyl group, a pyridyl group, a pyridazinyl group, a pyrimidinyl group, a benzofuryl group, a benzothienyl group or a quinolyl group, more preferably a thienyl group, a pyrazolyl group, a thiazolyl group, a pyridyl group, a benzofuryl group or a benzothienyl group, and particularly preferably a thienyl group or a pyridyl group.

The "heterocyclic group" shown by the respective substituents each means a "heterocyclic group" having the same meanings, and such a "heterocyclic group" means a partially unsaturated or completely saturated monocyclic group or bicyclic group containing 1 to 4 hetero atoms (in case of a plural number, each independently) selected from the group consisting of an oxygen atom, a nitrogen atom and a sulfur atom as a constitutional element(s) of the ring. The partially unsaturated heterocyclic group may be mentioned, for example, a 4,5-dihydro-1H-imidazolyl group, a 4,5-dihydroxazolyl group, a 4,5-dihydrothiazolyl group, a 1,4,5,6-tetrahydropyrimidinyl group, a 5,6-dihydro-4H-1,3-oxazinyl group or a 5,6-dihydro-4H-1,3-thiazinyl group, etc., and the completely saturated heterocyclic group may be mentioned, for example, a pyrrolidinyl group, a tetrahydrofuryl group, a 1,3-dioxolanyl group, a piperidinyl group, a tetrahydropyranyl group, a piperazinyl group, a morpholinyl group, a thiomorpholinyl group, a 1,3-dioxanyl group or a 1,4-dioxanyl group, etc. The "heterocyclic group" shown by the respective substituents may be preferably mentioned a 4,5-dihydro-1H-imidazolyl group, an azetidinyl group, a pyrrolidinyl group, a piperidinyl group, a piperazinyl group, a morpholinyl group or a thiomorpholinyl group, more preferably an azetidinyl group, a pyrrolidinyl group, a piperidinyl group, a piperazinyl group or a morpholinyl group, and particularly preferably a pyrrolidinyl group or a piperidinyl group.

The "a halogeno group" shown by the respective substituents each means a "halogeno group" having the same meanings, and such a "halogeno group" may be mentioned, for example, a fluoro group, a chloro group, a bromo group or an iodo group, and preferably a fluoro group, a chloro group or a bromo group.

In the formula (I), n is an integer of 2 or 3, and $R^4$s may be the same or different from each other. When n is 3, $R^4$s are $C_1$-$C_6$ alkyl groups, and the nitrogen atom to which $R^4$s are substituted is quaternarized.

$R^1$ is preferably a hydrogen atom or a $C_1$-$C_4$ alkyl group, more preferably a hydrogen atom, a methyl group, an ethyl group, a propyl group, an isopropyl group or a tert-butyl group, and particularly preferably a hydrogen atom, a methyl group, an ethyl group or an isopropyl group.

$R^2$ is preferably a hydrogen atom or a $C_1$-$C_4$ alkyl group, more preferably a hydrogen atom or a methyl group, and particularly preferably a hydrogen atom.

$R^3$ is preferably a hydrogen atom or a $C_1$-$C_4$ alkyl group, more preferably a hydrogen atom or a methyl group, and particularly preferably a hydrogen atom.

$R^4$ is preferably a hydrogen atom, or, a $C_1$-$C_6$ alkyl group, a $C_3$-$C_5$ alkenyl group, a $C_3$-$C_5$ alkynyl group, a $C_3$-$C_6$ cycloalkyl group, a $C_2$-$C_4$ alkanoyl group, a benzoyl group, a $C_1$-$C_4$ alkylsulfonyl group, a phenylsulfonyl group, an aminosulfonyl group, a $C_1$-$C_4$ alkylaminosulfonyl group, a phenylsulfamoyl group, a $C_1$-$C_4$ alkylaminocarbonyl group, a phenylcarbamoyl group, a $C_1$-$C_4$ alkoxycarbonyl group, a phenoxycarbonyl group, a phenyl group, a thienyl group, a pyrazolyl group, a thiazolyl group, a pyridyl group, a benzofuryl group, a benzothienyl group or a completely saturated heterocyclic group, each of which may be substituted by a group(s) selected from the group consisting of a halogeno group, a $C_1$-$C_4$ alkoxy group, a $C_3$-$C_6$ cycloalkyl group, a $C_1$-$C_4$ alkoxycarbonyl group, a $C_1$-$C_4$ alkylthio group, a $C_1$-$C_4$ alkylsulfinyl group, a $C_1$-$C_4$ alkylsulfonyl group, a $C_1$-$C_4$ alkylamino group, a di-($C_1$-$C_4$ alkyl)amino group, a $C_2$-$C_4$ alkanoylamino group, a $C_1$-$C_4$ alkoxycarbonylamino group, an aryl group, a heteroaryl group and a completely saturated heterocyclic group, more preferably a hydrogen atom, or, a $C_1$-$C_4$ alkyl group, a 2-propenyl group, a 2-propynyl group, a cyclopropyl group, a $C_2$-$C_4$ alkanoyl group, a benzoyl group, a $C_1$-$C_4$ alkylsulfonyl group, a $C_1$-$C_4$ alkylaminosulfonyl group, a $C_1$-$C_4$ alkylaminocarbonyl group, a phenylcarbamoyl group, a $C_1$-$C_4$ alkoxycarbonyl group, a phenoxycarbonyl group, a phenyl group, a thienyl group, a pyridyl group, a pyrrolidinyl group or a piperidinyl group, each of which may be substituted by a group(s) selected from the group consisting of a fluoro group, a chloro group, a methoxy group, a cyclopropyl group, a methoxycarbonyl group, a $C_1$-$C_4$ alkylsulfonyl group, a dimethylamino group, a $C_2$-$C_4$ alkanoylamino group, a methoxycarbonylamino group, a phenyl group, a thienyl group, a pyridyl group, a pyrrolidinyl group and a piperidinyl group, further more preferably a hydrogen atom, a trifluoromethyl group, a 2,2,2-trifluoroethyl group, a benzyl group, a 1-phenylethyl group, a 2-phenylethyl group, a methyl group, an ethyl group, a propyl group, a butyl group, a cyclopropyl group, an acetyl group, a propanoyl group, a butanoyl group, a benzoyl group, a methylsulfonyl group, an ethylsulfonyl group, a propylsulfonyl group, a methylaminosulfonyl group, a methylaminocarbonyl group, a methoxycarbonyl group, a phenoxycarbonyl group, a phenyl group or a piperidinyl group, and particularly preferably a hydrogen atom, a methyl group, an ethyl group, a propyl group, a butyl group, a 2,2,2-trifluoroethyl group, a benzyl group, a butanoyl group, a propylsulfonyl group or a phenyl group.

Z is preferably a $C_1$-$C_4$ alkyl group, a $C_3$-$C_6$ cycloalkyl group, a $C_5$-$C_6$ cycloalkenyl group, a $C_3$-$C_5$ alkenyl group, a $C_3$-$C_5$ alkynyl group, a completely saturated heterocyclic group, each of which may be substituted by a group(s) selected from the group consisting of a halogeno group, a $C_7$-$C_{12}$ aralkyl group, a $C_1$-$C_4$ alkoxy group, a $C_7$-$C_{12}$ aralkyloxy group, a $C_3$-$C_6$ cycloalkyl group, a $C_5$-$C_6$ cycloalkenyl group, an arylcarbonyl group, a $C_1$-$C_4$ alkylamino group, a di-($C_1$-$C_4$ alkyl)amino group, an arylamino group, an aryl group, a heteroaryl group, an aryloxy group and a completely saturated heterocyclic group, or, a phenyl group or a heteroaryl group, each of which may be substituted by a group(s) selected from the group consisting of a halogeno group, a cyano group, a nitro group, a formyl group, a $C_1$-$C_4$ alkyl group, a halogeno-$C_1$-$C_4$ alkyl group, a $C_7$-$C_{12}$ aralkyl group, a $C_7$-$C_{12}$ aralkyloxy group, a $C_3$-$C_6$ cycloalkyl group, a $C_1$-$C_4$ alkoxy group, a $C_1$-$C_4$ alkoxycarbonyl group, a $C_1$-$C_4$ alkylthio group, a $C_1$-$C_4$ alkylamino group, a di-($C_1$-$C_4$ alkyl) amino group, an arylamino group, a $C_2$-$C_4$ alkanoylamino group, a $C_1$-$C_4$ alkoxycarbonylamino group, an aryl group, a heteroaryl group, an aryloxy group and a completely saturated heterocyclic group; more preferably a $C_1$-$C_4$ alkyl group, a 2-propynyl group, a 2-butynyl group, a cyclopropyl group, a cyclobutyl group, a cyclopentenyl group, each of which may be substituted by a group(s) selected from the group consisting of [a fluoro group, a chloro group, a benzyl group, a methoxy group, a benzyloxy group, a cyclopropyl group, a cyclopentenyl group, a benzoyl group, a methylamino group, a dimethylamino group, an anilino group, a phenyl group, a thienyl group, a pyridyl group, a phenoxy group, a pyrrolidinyl group and a piperidinyl group], or, a phenyl group, a thienyl group, a pyrazolyl group, a thiazolyl group or a pyridyl group, each of which may be substituted by a group(s) selected from the group consisting of [a fluoro group, a chloro group, a cyano group, a nitro group, a methyl group, an ethyl group, a trifluoromethyl group, a benzyl group, a benzyloxy group, a cyclopropyl group, a methoxy group, a methoxycarbonyl group, a methylthio group, a methylamino group, a dimethylamino group, an anilino group, an acetylamino group, a methoxycarbonylamino group, a phenyl group, a phenoxy group, an azetidinyl group, a pyrrolidinyl group and a piperidinyl group]; further more preferably a methyl group, an ethyl group, a trifluoromethyl group, a 2-phenylethyl group, a cyclopropyl group, a phenyl group, a 2-fluorophenyl group, a 3-fluorophenyl group, a 4-fluorophenyl group, a 2-chlorophenyl group, a 3-chlorophenyl group, a 4-chlorophenyl group, a 4-cyanophenyl group, a 4-cyclopropylphenyl group, a 4-methoxyphenyl group, a thiophen-2-yl group, a thiophen-3-yl group, a pyridin-2-yl group, a pyridin-3-yl group or a pyridin-4-yl group; and particularly preferably a phenyl group, a 4-fluorophenyl group, a pyridin-2-yl group or a pyridin-3-yl group.

When there is an optical isomer, geometric isomer or rotational isomer in the compound represented by the formula (I) of the present invention, such isomers are also included in the scope of the present invention, and when there is a proton tautomer, such tautomer is also included in the present invention.

The compound represented by the formula (I) of the present invention is easily converted into a pharmaceutically acceptable salt by treating it with an acid. Examples of such a salt include, for example, inorganic acid salts such as hydrochloride, hydrobromide, hydroiodide, nitrate, sulfate and phosphate; or organic acid salts such as acetate, trifluoroacetate, benzoate, oxalate, malonate, succinate, maleate, fumarate, tartrate, citrate, methanesulfonate, ethanesulfonate, trifluoromethanesulfonate, benzenesulfonate, p-toluenesulfonate, glutamate and aspartate.

The compound represented by the formula (I) of the present invention is easily converted into a pharmaceutically acceptable basic salt by treating it with a base when $R^1$ is a hydrogen atom. Examples of such a salt include, for example, metal salts such as a sodium salt, potassium salt, calcium salt or magnesium salt: inorganic salts such as an ammonium salt: or organic amine salts such as a triethylamine salt and guanidine salt.

Further, the compound or pharmaceutically acceptable salt thereof represented by the formula (I) of the present invention can be present as a hydrate or solvate, and they are also included in the present invention.

The compound represented by the formula (I) of the present invention is, preferably,

(1) a compound wherein $R^1$ is a hydrogen atom or a $C_1$-$C_4$ alkyl group,

(2) a compound wherein $R^1$ is a hydrogen atom, a methyl group, an ethyl group, a propyl group, an isopropyl group or a tert-butyl group,

(3) a compound wherein $R^1$ is a hydrogen atom, a methyl group, an ethyl group or an isopropyl group,

(4) a compound wherein $R^2$ and $R^3$ each independently represents a hydrogen atom or a $C_1$-$C_4$ alkyl group,

(5) a compound wherein $R^2$ and $R^3$ each independently represent a hydrogen atom or a methyl group,

(6) a compound wherein $R^2$ and $R^3$ are both hydrogen atoms,

(7) a compound wherein $R^4$ is a hydrogen atom, or, a $C_1$-$C_6$ alkyl group, a $C_3$-$C_5$ alkenyl group, a $C_3$-$C_5$ alkynyl group, a $C_3$-$C_6$ cycloalkyl group, a $C_2$-$C_4$ alkanoyl group, a benzoyl group, a $C_1$-$C_4$ alkylsulfonyl group, a phenylsulfonyl group, an aminosulfonyl group, a $C_1$-$C_4$ alkylaminosulfonyl group, a phenylsulfamoyl group, a $C_1$-$C_4$ alkylaminocarbonyl group, a phenylcarbamoyl group, a $C_1$-$C_4$ alkoxycarbonyl group, a phenoxycarbonyl group, a phenyl group, a thienyl group, a pyrazolyl group, a thiazolyl group, a pyridyl group, a benzofuryl group, a benzothienyl group or a completely saturated heterocyclic group, each of which may be substituted by a group(s) selected from the group consisting of a halogeno group, a $C_1$-$C_4$ alkoxy group, a $C_3$-$C_6$ cycloalkyl group, a $C_1$-$C_4$ alkoxycarbonyl group, a $C_1$-$C_4$ alkylthio group, a $C_1$-$C_4$ alkylsulfinyl group, a $C_1$-$C_4$ alkylsulfonyl group, a $C_1$-$C_4$ alkylamino group, a di-($C_1$-$C_4$ alkyl)amino group, a $C_2$-$C_4$ alkanoylamino group, a $C_1$-$C_4$ alkoxycarbonylamino group, an aryl group, a heteroaryl group and a completely saturated heterocyclic group,

(8) a compound wherein $R^4$ is a hydrogen atom, or, a $C_1$-$C_4$ alkyl group, a 2-propenyl group, a 2-propynyl group, a

cyclopropyl group, a $C_2$-$C_4$ alkanoyl group, a benzoyl group, a $C_1$-$C_4$ alkylsulfonyl group, a $C_1$-$C_4$ alkylaminosulfonyl group, a $C_1$-$C_4$ alkylaminocarbonyl group, a phenylcarbamoyl group, a $C_1$-$C_4$ alkoxycarbonyl group, a phenoxycarbonyl group, a phenyl group, a thienyl group, a pyridyl group, a pyrrolidinyl group or piperidinyl group, each of which may be substituted by a group(s) selected from the group consisting of a fluoro group, a chloro group, a methoxy group, a cyclopropyl group, a methoxycarbonyl group, a $C_1$-$C_4$ alkylsulfonyl group, a dimethylamino group, a $C_2$-$C_4$ alkanoylamino group, a methoxycarbonylamino group, a phenyl group, a thienyl group, a pyridyl group, a pyrrolidinyl group and a piperidinyl group,

(9) a compound wherein $R^4$ is a hydrogen atom, a trifluoromethyl group, a 2,2,2-trifluoroethyl group, a benzyl group, a 1-phenylethyl group, a 2-phenylethyl group, a methyl group, an ethyl group, a propyl group, a butyl group, a cyclopropyl group, an acetyl group, a propanoyl group, a butanoyl group, a benzoyl group, a methylsulfonyl group, an ethylsulfonyl group, a propylsulfonyl group, a methylaminosulfonyl group, a methylaminocarbonyl group, a methoxycarbonyl group, a phenoxycarbonyl group, a phenyl group or a piperidinyl group,

(10) a compound wherein $R^4$ is a hydrogen atom, a methyl group, an ethyl group, a propyl group, a butyl group, a 2,2,2-trifluoroethyl group, a benzyl group, a butanoyl group, a propylsulfonyl group or a phenyl group,

(11) a compound wherein Z is a $C_1$-$C_4$ alkyl group, a $C_3$-$C_6$ cycloalkyl group, a $C_5$-$C_6$ cycloalkenyl group, a $C_3$-$C_5$ alkenyl group, a $C_3$-$C_5$ alkynyl group, a completely saturated heterocyclic group, each of which may be substituted by a group(s) selected from the group consisting of a halogeno group, a $C_7$-$C_{12}$ aralkyl group, a $C_1$-$C_4$ alkoxy group, a $C_7$-$C_{12}$ aralkyloxy group, a $C_3$-$C_6$ cycloalkyl group, a $C_5$-$C_6$ cycloalkenyl group, an arylcarbonyl group, a $C_1$-$C_4$ alkylamino group, a di-($C_1$-$C_4$ alkyl)amino group, an arylamino group, an aryl group, a heteroaryl group, aryloxy group and a completely saturated heterocyclic group, or, a phenyl group or a heteroaryl group, each of which may be substituted by a group(s) selected from the group consisting of a halogeno group, a cyano group, a nitro group, a formyl group, a $C_1$-$C_4$ alkyl group, a halogeno-$C_1$-$C_4$ alkyl group, a $C_7$-$C_{12}$ aralkyl group, a $C_7$-$C_{12}$ aralkyloxy group, a $C_3$-$C_6$ cycloalkyl group, a $C_1$-$C_4$ alkoxy group, a $C_1$-$C_4$ alkoxycarbonyl group, a $C_1$-$C_4$ alkylthio group, a $C_1$-$C_4$ alkylamino group, a di-($C_1$-$C_4$ alkyl)amino group, an arylamino group, a $C_2$-$C_4$ alkanoylamino group, a $C_1$-$C_4$ alkoxycarbonylamino group, an aryl group, a heteroaryl group, an aryloxy group and a completely saturated heterocyclic group,

(12) a compound wherein Z is a $C_1$-$C_4$ alkyl group, a 2-propynyl group, a 2-butynyl group, a cyclopropyl group, a cyclobutyl group, a cyclopentenyl group, each which may be substituted by a group(s) selected from the group consisting of [a fluoro group, a chloro group, a benzyl group, a methoxy group, a benzyloxy group, a cyclopropyl group, a cyclopentenyl group, a benzoyl group, a methylamino group, a dimethylamino group, an anilino group, a phenyl group, a thienyl group, a pyridyl group, a phenoxy group, a pyrrolidinyl group and a piperidinyl group], or, a phenyl group, a thienyl group, a pyrazolyl group, a thiazolyl group or a pyridyl group, each of which may be substituted by a group(s) selected from the group consisting of [a fluoro group, a chloro group, a cyano group, a nitro group, a methyl group, an ethyl group, a trifluoromethyl group, a benzyl group, a benzyloxy group, a cyclopropyl group, a methoxy group, a methoxycarbonyl group, a methylthio group, a methylamino group, a dimethylamino group, an anilino group, an acetylamino group, a methoxycarbonylamino group, a phenyl group, a phenoxy group, an azetidinyl group, a pyrrolidinyl group and a piperidinyl group],

(13) a compound wherein Z is a methyl group, an ethyl group, a trifluoromethyl group, a 2-phenylethyl group, a cyclopropyl group, a phenyl group, a 2-fluorophenyl group, a 3-fluorophenyl group, a 4-fluorophenyl group, a 2-chlorophenyl group, a 3-chlorophenyl group, a 4-chlorophenyl group, a 4-cyanophenyl group, a 4-cyclopropylphenyl group, a 4-methoxyphenyl group, a thiophen-2-yl group, a thiophen-3-yl group, a pyridin-2-yl group, a pyridin-3-yl group or a pyridin-4-yl group,

(14) a compound wherein Z is a phenyl group, a 4-fluorophenyl group, a pyridin-2-yl group or a pyridin-3-yl group.

Further, in the above-mentioned groups of (1)-(3), (4)-(6), (7)-(10) and (11)-(14), as the number becomes larger, a more preferred compound is indicated, and a compound obtained by arbitrarily selecting $R^1$ from the groups (1)-(3), $R^2$ and $R^3$ from the groups (4)-(6), $R^4$ from the groups (7)-(10), and Z from the groups (11)-(14), or by arbitrarily combining them is also a preferred compound.
Examples of such compound include:

(15) a compound wherein $R^1$ is a hydrogen atom, a methyl group, an ethyl group, a propyl group, an isopropyl group or a tert-butyl group,

$R^2$ and $R^3$ each independently is a hydrogen atom or a methyl group,
$R^4$ is a hydrogen atom, or, a $C_1$-$C_6$ alkyl group, a $C_3$-$C_5$ alkenyl group, a $C_3$-$C_5$ alkynyl group, a $C_3$-$C_6$ cycloalkyl group, a $C_2$-$C_4$ alkanoyl group, a benzoyl group, a $C_1$-$C_4$ alkylsulfonyl group, a phenylsulfonyl group, an aminosulfonyl group, a $C_1$-$C_4$ alkylaminosulfonyl group, a phenylsulfamoyl group, a $C_1$-$C_4$ alkylaminocarbonyl group, a phenylcarbamoyl group, a $C_1$-$C_4$ alkoxycarbonyl group, a phenoxycarbonyl group, a phenyl group, a

thienyl group, a pyrazolyl group, a thiazolyl group, a pyridyl group, a benzofuryl group, a benzothienyl group or a completely saturated heterocyclic group, each of which may be substituted by a group(s) selected from the group consisting of a halogeno group, a $C_1$-$C_4$ alkoxy group, a $C_3$-$C_6$ cycloalkyl group, a $C_1$-$C_4$ alkoxycarbonyl group, a $C_1$-$C_4$ alkylthio group, a $C_1$-$C_4$ alkylsulfinyl group, a $C_1$-$C_4$ alkylsulfonyl group, a $C_1$-$C_4$ alkylamino group, a di-($C_1$-$C_4$ alkyl)amino group, a $C_2$-$C_4$ alkanoylamino group, a $C_1$-$C_4$ alkoxycarbonylamino group, an aryl group, a heteroaryl group and a completely saturated heterocyclic group,

Z is a $C_1$-$C_4$ alkyl group, a $C_3$-$C_6$ cycloalkyl group, a $C_5$-$C_6$ cycloalkenyl group, a $C_3$-$C_5$ alkenyl group, a $C_3$-$C_5$ alkynyl group or a completely saturated heterocyclic group, each of which may be substituted by a group(s) selected from the group consisting of a halogeno group, a $C_7$-$C_{12}$ aralkyl group, a $C_1$-$C_4$ alkoxy group, a $C_7$-$C_{12}$ aralkyloxy group, a $C_3$-$C_6$ cycloalkyl group, a $C_5$-$C_6$ cycloalkenyl group, an arylcarbonyl group, a $C_1$-$C_4$ alkylamino group, a di-($C_1$-$C_4$ alkyl)amino group, an arylamino group, an aryl group, a heteroaryl group, an aryloxy group and a completely saturated heterocyclic group, or, a phenyl group or a heteroaryl group, each of which may be substituted by a group(s) selected from the group consisting of a halogeno group, a cyano group, a nitro group, a formyl group, a $C_1$-$C_4$ alkyl group, a halogeno-$C_1$-$C_4$ alkyl group, a $C_7$-$C_{12}$ aralkyl group, a $C_7$-$C_{12}$ aralkyloxy group, a $C_3$-$C_6$ cycloalkyl group, a $C_1$-$C_4$ alkoxy group, a $C_1$-$C_4$ alkoxycarbonyl group, a $C_1$-$C_4$ alkylthio group, a $C_1$-$C_4$ alkylamino group, a di-($C_1$-$C_4$ alkyl)amino group, an arylamino group, a $C_2$-$C_4$ alkanoylamino group, a $C_1$-$C_4$ alkoxycarbonylamino group, an aryl group, a heteroaryl group, an aryloxy group and a completely saturated heterocyclic group, (16) a compound wherein $R^1$ is a hydrogen atom, a methyl group, an ethyl group, a propyl group, an isopropyl group or a tert-butyl group,

$R^2$ and $R^3$ each independently is a hydrogen atom or a methyl group,

$R^4$ is a hydrogen atom, or, a $C_1$-$C_4$ alkyl group, a 2-propenyl group, a 2-propynyl group, a cyclopropyl group, a $C_2$-$C_4$ alkanoyl group, a benzoyl group, a $C_1$-$C_4$ alkylsulfonyl group, a $C_1$-$C_4$ alkylaminosulfonyl group, a $C_1$-$C_4$ alkylaminocarbonyl group, a phenylcarbamoyl group, a $C_1$-$C_4$ alkoxycarbonyl group, a phenoxycarbonyl group, a phenyl group, a thienyl group, a pyridyl group, a pyrrolidinyl group or a piperidinyl group, each of which may be substituted by a group(s) selected from the group consisting of a fluoro group, a chloro group, a methoxy group, a cyclopropyl group, a methoxycarbonyl group, a $C_1$-$C_4$ alkylsulfonyl group, a dimethylamino group, a $C_2$-$C_4$ alkanoylamino group, a methoxycarbonylamino group, a phenyl group, a thienyl group, a pyridyl group, a pyrrolidinyl group and a piperidinyl group,

Z is a $C_1$-$C_4$ alkyl group, a 2-propynyl group, a 2-butynyl group, a cyclopropyl group, a cyclobutyl group, a cyclopentenyl group, each of which may be substituted by a group(s) selected from the group consisting of [a fluoro group, a chloro group, a benzyl group, a methoxy group, a benzyloxy group, a cyclopropyl group, a cyclopentenyl group, a benzoyl group, a methylamino group, a dimethylamino group, an anilino group, a phenyl group, a thienyl group, a pyridyl group, a phenoxy group, a pyrrolidinyl group and a piperidinyl group], or, a phenyl group, a thienyl group, a pyrazolyl group, a thiazolyl group or a pyridyl group, each of which may be substituted by a group(s) selected from the group consisting of [a fluoro group, a chloro group, a cyano group, a nitro group, a methyl group, an ethyl group, a trifluoromethyl group, a benzyl group, a benzyloxy group, a cyclopropyl group, a methoxy group, a methoxycarbonyl group, a methylthio group, a methylamino group, a dimethylamino group, an anilino group, an acetylamino group, a methoxycarbonylamino group, a phenyl group, a phenoxy group, an azetidinyl group, a pyrrolidinyl group and a piperidinyl group], (17) a compound wherein $R^1$ is a hydrogen atom, a methyl group, an ethyl group or an isopropyl group,

$R^2$ and $R^3$ are both hydrogen atoms,

$R^4$ is a hydrogen atom, a trifluoromethyl group, a 2,2,2-trifluoroethyl group, a benzyl group, a 1-phenylethyl group, a 2-phenylethyl group, a methyl group, an ethyl group, a propyl group, a butyl group, a cyclopropyl group, an acetyl group, a propanoyl group, a butanoyl group, a benzoyl group, a methylsulfonyl group, an ethylsulfonyl group, a propylsulfonyl group, a methylaminosulfonyl group, a methylaminocarbonyl group, a methoxycarbonyl group, a phenoxycarbonyl group, a phenyl group or a piperidinyl group,

Z is a methyl group, an ethyl group, a trifluoromethyl group, a 2-phenylethyl group, a cyclopropyl group, a phenyl group, a 2-fluorophenyl group, a 3-fluorophenyl group, a 4-fluorophenyl group, a 2-chlorophenyl group, a 3-chlorophenyl group, a 4-chlorophenyl group, a 4-cyanophenyl group, a 4-cyclopropylphenyl group, a 4-methoxyphenyl group, a thiophen-2-yl group, a thiophen-3-yl group, a pyridin-2-yl group, a pyridin-3-yl group or a pyridin-4-yl group, or,

(18) a compound wherein $R^1$ is a hydrogen atom, a methyl group, an ethyl group or an isopropyl group,

$R^2$ and $R^3$ are both hydrogen atoms,

$R^4$ is a hydrogen atom, a methyl group, an ethyl group, a propyl group, a butyl group, a 2,2,2-trifluoroethyl group, a benzyl group, a butanoyl group, a propylsulfonyl group or a phenyl group,

Z is a phenyl group, a 4-fluorophenyl group, a pyridin-2-yl group or a pyridin-3-yl group.

(19) as the aniline compound, it is preferably mentioned a compound wherein
{6-[(4-methylaminobenzyl)(pyridin-3-ylsulfonyl)aminomethyl]pyridin-2-ylamino}-acetic acid,
[6-({4-[methyl(propylsulfonyl)amino]benzyl}(pyridin-3-ylsulfonyl)aminomethyl)-pyridin-2-ylamino]acetic acid,
{6-[(4-diphenylaminobenzyl)(pyridin-3-ylsulfonyl)aminomethyl]pyridin-2-ylamino}-acetic acid,
[6-({4-[diethyl(methyl)ammonioammonio]benzyl}(pyridin-3-ylsulfonyl)aminomethyl)-pyridin-2-ylamino]acetic acid,
[6-({4-[butyrylbutyryl(methyl)amino]benzyl}(pyridin-3-ylsulfonyl)aminomethyl)-pyridin-2-ylamino]acetic acid,
[6-({4-[benzyl(methyl)amino]benzyl}(pyridin-3-ylsulfonyl)aminomethyl)pyridin-2-yl-amino]acetic acid,
[6-({4-[butyl(methyl)amino]benzyl}(pyridin-2-ylsulfonyl)aminomethyl)pyridin-2-yl-amino]acetic acid,
(6- {[4-(phenylamino)benzyl](pyridin-2-ylsulfonyl)aminomethyl}pyridin-2-ylamino)-acetic acid, or
[6-({4-[butyl(2,2,2-trifluoroethyl)amino]benzyl}(pyridin-2-ylsulfonyl)aminomethyl)-pyridin-2-ylamino]acetic acid, etc.

Further, the present invention also provides:

(20) a pharmaceutical composition containing as an active ingredient the above-mentioned compound represented by the formula (I), an aniline compound according to any one of (1) to (19) or a pharmaceutically acceptable salt thereof, and
(21) a pharmaceutical composition according to (20) for the prevention or treatment of respiratory diseases.

Representative preparation method of the compound of the present invention is shown below. With regard to the respective specific preparation method of the compounds of the present invention, they are explained in detail in the below-mentioned Examples.
[Formula 2]

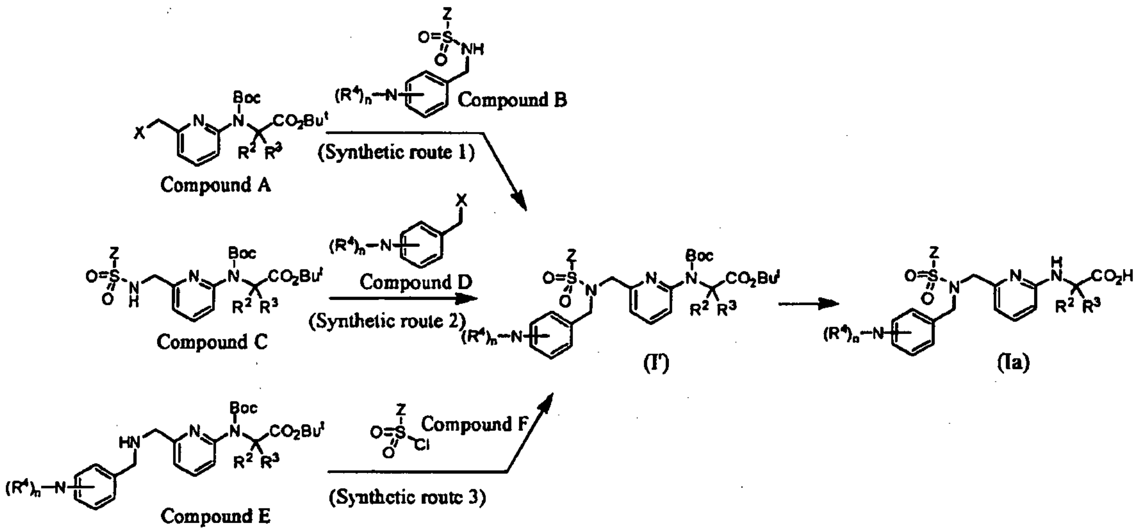


[wherein $R^2$, $R^3$, $R^4$, Z and n have the same meanings as defined above, X represents a hydroxy group, a chloro group, a bromo group, an iodo group, a methanesulfonyloxy group, a benzenesulfonyloxy group, a p-toluenesulfonyloxy group or a trifluoromethanesulfonyloxy group, Boc represents a tert-butoxycarbonyl group, and $Bu^t$ represents a tert-butyl group.]

Synthetic routes 1 to 3: Compound A and Compound B, or, Compound C and Compound D, or, Compound E and Compound F are each reacted in an organic solvent, in the presence of a condensing agent or a base, respectively, to prepare Compound (I') which is a precursor of the compound of the present invention.
Compound (Ia) wherein $R^1$ is a hydrogen atom of the present invention can be obtained by deprotecting the Boc group and $Bu^t$ group of the precursor compound (I') by an acid treatment.
The substituent(s) on the substituent $R^4$ and/or the substituent Z may be previously introduced before the preparation, or, after preparing a basic skeleton according to the above-mentioned process, a desired substituent(s) may be introduced into the basic skeleton by using the conventionally used synthetic method using an oxidation, reduction, alkylation, esterification, amidation, dehydration, deprotection, acetylation, hydrolysis, coupling reaction, cyclization and/or an optional combination thereof.
The preparation method of the synthetic intermediates of the compound according to the present invention will be

mentioned in the following Examples in detail.

The objective compounds formed in each of the respective reactions can be obtained from a reaction mixture in accordance with the conventional methods. For example, after suitably neutralizing the reaction mixture, or removing insolubles by filtration in the case such insolubles are present, an organic solvent such as ethyl acetate that is not miscible with water is added followed by rinsing with water, separating the organic layer containing the objective compound, drying with a drying agent such as anhydrous magnesium sulfate and anhydrous sodium sulfate, and distilling off the solvent to obtain the objective compound.

The resulting objective compound can be separated and purified as necessary by suitably combining the conventional methods, examples of which include recrystallization; reprecipitation; or a method commonly used to separate and purify ordinary organic compounds (such as adsorption column chromatography using a carrier such as silica gel and alkylated silica gel; ion exchange chromatography; or normal or reverse phase column chromatography using silica gel or alkylated silica gel (and preferably, high-performance liquid chromatography)).

Although the compound represented by the formula (I) of the present invention can be converted into a pharmaceutically acceptable salt in accordance with ordinary methods as necessary, it can also be separated directly from the reaction mixture as a salt.

In the case of using the compound represented by the formula (I), or a pharmaceutically acceptable salt thereof of the present invention, as a pharmaceutical, the compound, or pharmaceutically acceptable salt thereof, per se can be administered (as a bulk powder), or can be administered orally or parenterally (such as intravenous administration, intramuscular administration, intraperitoneal administration, transcutaneous administration, transtracheal administration, intracutaneous administration and subcutaneous administration) in a form such as a tablet, capsule, powder, syrup, granule, fine particles, pill, suspension, emulsion, transdermal preparation, suppository, ointment, lotion, inhalant and injection, which is prepared by mixing with a suitable pharmaceutically acceptable vehicle or diluent and the like.

These preparations are prepared by commonly known methods using additives such as vehicles, lubricants, binders, disintegrators, emulsifiers, stabilizers, corrigents or diluents and the like.

Examples of vehicles include organic vehicles and inorganic vehicles. Examples of organic vehicles include sugar derivatives such as lactose, sucrose, glucose, mannitol and sorbitol; starch derivatives such as cornstarch, potato starch, $\alpha$-starch and dextrin; cellulose derivatives such as crystalline cellulose; gum Arabic; dextran; and pullulan. Examples of inorganic vehicles include light silicic acid anhydride; and sulfates such as calcium sulfate.

Examples of lubricants include stearic acid; stearic acid metal salts such as calcium stearate and magnesium stearate; talc; colloidal silica; waxes such as beeswax and spermaceti; boric acid; adipic acid; sulfates such as sodium sulfate; glycol; fumaric acid; sodium benzoate; D,L-leucine; sodium lauryl sulfate; silicic acids such as silicic acid anhydride and silicic acid hydrate; and the above-mentioned starch derivatives listed as examples of the vehicles.

Examples of binders include hydroxypropyl cellulose, hydroxypropyl methylcellulose, polyvinyl pyrrolidone, Macrogol and the above-mentioned compounds listed as examples of the vehicles.

Examples of disintegrators include cellulose derivatives such as low substitution-degree hydroxypropyl cellulose, carboxymethyl cellulose, calcium carboxymethyl cellulose and internally-crosslinked calcium carboxymethyl cellulose; crosslinked polyvinyl pyrrolidone; and chemically modified starch or cellulose derivatives such as carboxymethyl starch and sodium carboxymethyl starch.

Examples of emulsifiers include colloidal clays such as bentonite and bee gum; anionic surfactants such as sodium lauryl sulfate; cationic surfactants such as benzalkonium chloride; and nonionic surfactants such as polyoxyethylene alkyl ethers, polyoxyethylene sorbitan fatty acid esters and sucrose fatty acid esters.

Examples of stabilizers include para-hydroxybenzoic acid esters such as methylparaben and propylparaben; alcohols such as chlorobutanol, benzyl alcohol and phenylethyl alcohol; benzalkonium chloride; phenols such as phenol and cresol; thimerosal; acetic anhydride; and sorbic acid.

Examples of corrigents include sweeteners such as sodium saccharin and aspartame; sour flavorings such as citric acid, malic acid and tartaric acid; and flavorings such as menthol, lemon extract and orange extract.

Examples of diluents include compounds ordinarily used as diluents, such as lactose, mannitol, glucose, sucrose, calcium sulfate, hydroxypropyl cellulose, microcrystalline cellulose, water, ethanol, polyethylene glycol, propylene glycol, glycerol, starch, polyvinyl pyrrolidone and mixtures thereof.

Although the dosage of a compound represented by the formula (I) or a pharmaceutically acceptable salt thereof of the present invention can be varied according to conditions such as patient symptoms, age or body weight, the adult dosage per administration in the case of oral administration has a lower limit of 0.001 mg/Kg (preferably 0.01 mg/Kg) and an upper limit of 20 mg/Kg (preferably 10 mg/Kg), while the adult dosage per administration in the case of parenteral administration has a lower limit of 0.0001 mg/Kg (preferably 0.0005 mg/Kg) and an upper limit of 10 mg/kg (preferably 5 mg/Kg), administered corresponding to symptoms from 1 to 6 times per day.

EXAMPLES

In the following, the present invention is explained in more detail by referring to Examples, Reference examples and Test examples, but the scope of the present invention is not limited to these ranges. Incidentally, the Rf value in Examples is a value measured by using a thin-layer chromatography (available from Merck, TLC plate silica gel $60F_{254}$ (Trade name)), and the description in the parentheses represents an eluent(s) (volume ratio).

[Example 1]

{6-[(4-Methylaminobenzyl)(pyridin-3-ylsulfonyl)aminomethyl]pyridin-2-ylamino}-acetic acid

1-(a): tert-Butyl (tert-butoxycarbonyl{6-[(4-methylaminobenzyl)(pyridin-3-ylsulfonyl)-aminomethyl]pyridin-2-yl}amino)acetate

To 20 ml of a tetrahydrofuran solution containing 2.02 g (4.22 mmol) of tert-butyl (tert-butoxycarbonyl {6-[(pyridin-3-ylsulfonyl)aminomethyl]pyridin-2-yl}amino)-acetate obtained in the same manner as in Reference example 1-(f) were added 1.30 g (9.48 mmol) of 4-methylaminobenzyl alcohol (see Organic Letters, 9, 671 (2007)), 3.4 ml (14 mmol) oftri-n-butylphosphine and 2.35 g (13.6 mmol) ofN,N,N',N'-tetramethylazodicarboxamide, and the mixture was stirred at room temperature for 8.5 hours. After completion of the reaction, water was added to the reaction mixture, and the mixture was extracted with ethyl acetate. The organic layer was washed with a saturated aqueous sodium chloride solution, dried over anhydrous sodium sulfate and concentrated under reduced pressure. The obtained residue was applied to silica gel column chromatography (eluent; n-hexane:ethyl acetate=2:1→1:1 (VV)), and the fractions containing the objective material were concentrated under reduced pressure. Then, the obtained crude product was purified by reverse phase C18 column chromatography (eluent; acetonitrile:water=1:1→ 1:0 (V/V)) to obtain 1.83 g of the title compound as pale yellow foam. (Yield: 73%)
Mass spectrum (FAB, m/z): 598 ($M^+$+1).
$^1$H-NMR spectrum ($CDCl_3$, δ ppm): 8.93 (dd, J=2.3, 0.6 Hz, 1H), 8.69 (dd, J=4.9, 1.7 Hz, 1H), 7.86 (ddd, J=8.1, 2.3, 1.7 Hz, 1H), 7.70 (d, J=8.2 Hz, 1H), 7.52 (dd, J=8.2, 7.3 Hz, 1H), 7.30 (ddd, J=8.1, 4.9, 0.6 Hz, 1H), 7.04-6.98 (m, 2H), 6.87 (d, J=7.3 Hz, 1H), 6.52-6.45 (m, 2H), 4.45 (s, 2H), 4.39 (s, 2H), 4.35 (s, 2H), 3.73 (s, 1H), 2.81 (s, 3H), 1.52 (s, 9H), 1.43 (s, 9H).

1-(b): {6-[(4-Methylaminobenzyl)(pyridin-3-ylsulfonyl)aminomethyl]pyridin-2-yl-amino}acetic acid

To 3.4 ml of a methylene chloride solution containing 100 mg (0.167 mmol) of tert-butyl (tert-butoxycarbonyl {6-[(4-methylaminobenzyl)(pyridin-3-ylsulfonyl)amino-methyl]pyridin-2-yl}amino)acetate obtained in Example 1-(a) was added 1.7 ml (6.8 mmol) of 4N hydrogen chloride/1,4-dioxane solution, and the mixture was stirred at room temperature for 8 hours. After completion of the reaction, the reaction mixture was concentrated under reduced pressure, water was added to the residue, and the mixture was adjusted to pH 5.0 with 1N aqueous sodium hydroxide solution and 1N hydrochloric acid. The precipitated solid was collected by filtration and dried under reduced pressure to obtain 55 mg of the title compound as pale brownish solid. (Yield: 74%)
Mass spectrum (FAB, m/z): 442($M^+$+1).
$^1$H-NMR spectrum (DMSO-$d_6$, δ ppm): 8.78 (dd, J=2.3, 0.8 Hz, 1H), 8.70 (dd, J=4.9, 1.6 Hz, 1H), 7.99 (ddd, J=8.0, 2.3, 1.6 Hz, 1H), 7.46 (ddd, J=8.0, 4.9, 0.8 Hz, 1H), 7.24 (dd, J=8.3, 7.0 Hz, 1H), 7.00-6.95 (m, 2H), 6.62 (brs, 0.9H), 6.48-6.43 (m, 2H), 6.35 (d, J=8.3 Hz, 1H), 6.30 (d, J=7.0 Hz, 1H), 5.65 (brs, 0.5H), 4.47 (s, 2H), 4.10 (s, 2H), 3.66 (d, J=4.8 Hz, 2H), 2.64 (s, 3H).
Rf value: 0.51 (n-butanol: acetic acid:water=3:1:1).

[Example 2]

[6-({4-[Methyl(propylsulfonyl)amino]benzyl}(pyridin-3-ylsulfonyl)aminomethyl)-pyridin-2-ylamino]acetic acid

2-(a): tert-Butyl {tert-butoxycarbonyl[6-({4-[methyl(propylsulfonyl)amino]benzyl}-(pyridin-3-ylsulfonyl)aminomethyl)pyridin-2-yl]amino}acetate

To 1 ml of a methylene chloride solution containing 151 mg (0.253 mmol) of tert-butyl (tert-butoxycarbonyl {6-[(4-methylaminobenzyl)(pyridin-3-ylsulfonyl)aminomethyl]pyridin-2-yl}amino)acetate obtained in Example 1-(a) were added 70 μl (0.50 mmol) of triethylamine and 34 μl (0.30 mmol) of 1-propanesulfonyl chloride under ice-cooling, and the mixture was stirred at room temperature for 2 hours. Further, 70 μl (0.50 mmol) of triethylamine and 34 μl (0.30

mmol) of 1-propanesulfonyl chloride were additionally added to the mixture, and the mixture was stirred at room temperature for 30 minutes. After completion of the reaction, 1 ml of water and 0.5 ml of a saturated aqueous sodium hydrogen carbonate solution were added to the reaction mixture, and the mixture was extracted with ethyl acetate. After the organic layer was dried over anhydrous sodium sulfate, the mixture was concentrated under reduced pressure. The obtained residue was applied to silica gel column chromatography (eluent; n-hexane: ethyl acetate=2:1→1:2 (V/V)), and the fractions containing the objective material were concentrated under reduced pressure to obtain 163 mg of the title compound as pale brownish foam. (Yield: 92%)
Mass spectrum (FAB, m/z): 704 ($M^+$+1).
H-NMR spectrum ($CDCl_3$, δ ppm): 8.94-8.90 (m, 1H), 8.71 (dd, J=4.9, 1.7 Hz, 1H), 7.86 (ddd, J=8.1, 2.4, 1.7 Hz, 1H), 7.70 (d, J=8.2 Hz, 1H), 7.50 (dd, J=8.2, 7.2 Hz, 1H),7.36-7.21 (m, 5H), 6.84 (d, J=7.2 Hz, 1H), 4.57 (s, 2H), 4.39 (s, 2H), 4.35 (s, 2H), 3.31 (s, 3H), 2.99-2.91 (m, 2H), 1.92-1.78 (m, 2H), 1.53 (s, 9H), 1.43 (s, 9H), 1.04 (t, J=7.4 Hz, 3H).

2-(b): <u>[6-({4-[Methyl(propylsulfonyl)amino]benzyl}(pyridin-3-ylsulfonyl)amino-methyl)pyridin-2-ylamino]acetic acid</u>

To 2.3 ml of a methylene chloride solution containing 160 mg (0.227 mmol) of tert-butyl {tert-butoxycarbonyl [6-({4-[methyl(propylsulfonyl)amino]benzyl}(pyridin-3-ylsulfonyl)aminomethyl)pyridin-2-yl]amino}acetate obtained in Example 2-(a) was added 2.3 ml (9.2 mmol) of 4N hydrogen chloride/1,4-dioxane solution, and the mixture was allowed to stand at room temperature for 7 hours. After completion of the reaction, the reaction mixture was concentrated under reduced pressure, water was added to the residue, and the mixture was adjusted to pH 4.6 with 1N aqueous sodium hydroxide solution and 1N hydrochloric acid. The precipitated solid was collected by filtration and dried under reduced pressure to obtain 107 mg of the title compound as pale bluish-grey solid. (Yield: 86%)
Mass spectrum (FAB, m/z): 548 ($M^+$+1).
H-NMR spectrum (DMSO-$d_6$, δ ppm): 8.80 (dd, J=2.4, 0.8 Hz, 1H), 8.71 (dd, J=4.8, 1.5 Hz, 1H), 8.01 (ddd, J=8.1, 2.4, 1.5 Hz, 1H), 7.47 (ddd, J=8.1, 4.8, 0.8 Hz, 1H), 7.37-7.34 (m, 2H), 7.31-7.28 (m, 2H), 7.23 (dd, J=8.3, 7.1 Hz, 1H), 6.75-6.66 (m, 1H), 6.35 (d, J=8.3 Hz, 1H), 6.31 (d, J=7.1 Hz, 1H), 4.66 (s, 2H), 4.20 (s, 2H), 3.68 (d, J=5.5 Hz, 2H), 3.24 (s, 3H), 3.10-3.05 (m, 2H), 1.71-1.63 (m, 2H), 0.96 (t, J=7.4 Hz, 3H). Rf value: 0.66 (n-butanol:acetic acid:water=3:1:1).

[Example 3]

<u>{6-[(4-Diphenylaminobenzyl)(pyridin-3-ylsulfonyl)aminomethyl]pyridin-2-ylamino}-acetic acid</u>

3-(a): <u>tert-Butyl (tert-butoxycarbonyl{6-[(4-diphenylaminobenzyl)(pyridin-3-yl-sulfonyl)aminomethyl]pyridin-2-yl}amino) acetate</u>

To 1.6 ml of a tetrahydrofuran solution containing 106 mg (0.385 mmol) of 4-diphenylaminobenzyl alcohol (see US2003/87127A) were added 158 mg (0.330 mmol) of tert-butyl (tert-butoxycarbonyl {6-[(pyridin-3-ylsulfonyl)aminomethyl]pyridin-2-yl}-amino)acetate obtained in the same manner as in Reference example 1-(f), 0.25 ml (1.0 mmol) of tri-n-butylphosphine and 170 mg (0.987 mmol) of N,N,N',N'-tetramethylazodicarboxamide, and the mixture was stirred at room temperature for 15 hours. After completion of the reaction, water was added to the reaction mixture, and the mixture was extracted with ethyl acetate. The organic layer was washed with a saturated aqueous sodium chloride solution, dried over anhydrous sodium sulfate and concentrated under reduced pressure. The obtained residue was applied to silica gel column chromatography (eluent; n-hexane:ethyl acetate=1:0→3:2 (V/V)), and the fractions containing the objective material were concentrated under reduced pressure to obtain 166 mg of the title compound as pale yellow foam. (Yield: 68%)
Mass spectrum (FAB, m/z): 736 ($M^+$+1).
$^1$H-NMR spectrum ($CDCl_3$, δ ppm): 8.94-8.92 (m, 1H), 8.71 (dd, J=4.9, 1.5 Hz, 1H), 7.91-7.86 (m, 1H), 7.72 (d, J=8.4 Hz, 1H), 7.53 (dd, J=8.4, 7.4 Hz, 1H), 7.32 (ddd, J=8.1, 4.9, 0.7 Hz, 1H), 7.29-6.90 (m, 14H), 6.90 (d, J=7.4 Hz, 1H), 4.50 (s, 2H), 4.41 (s, 2H), 4.38 (s, 2H), 1.52 (s, 9H), 1.40 (s, 9H).

3-(b): <u>{6-[(4-Diphenylaminobenzyl)(pyridin-3-ylsulfonyl)aminomethyl]pyridin-2-ylamino}acetic acid</u>

To 1.4 ml of a tetrahydrofuran solution containing 160 mg (0.218 mmol) of tert-butyl (tert-butoxycarbonyl {6-[(4-diphenylaminobenzyl)(pyridin-3-ylsulfonyl)-aminomethyl]pyridin-2-yl}amino)acetate obtained in Example 3-(a) were added 1 ml of water and 0.25 ml of concentrated hydrochloric acid, and the mixture was stirred at 65°C for 4 hours. After completion of the reaction, the reaction mixture was concentrated under reduced pressure, water was added to the residue, and the mixture was adjusted to pH 6.8 with 1N aqueous sodium hydroxide solution and 1N hydrochloric acid. The mixture was extracted with ethyl acetate, the organic layer was washed with a saturated aqueous sodium chloride solution, and the mixture was concentrated under reduced pressure. To the residue were added tert-butyl methyl ether

and diisopropyl ether, the precipitated solid was collected by filtration, and dried under reduced pressure to obtain 79 mg of the title compound as pale brown solid.
(Yield: 63%)
Mass spectrum (FAB, m/z): 580 ($M^+$+1).
$^1$H-NMR spectrum (DMSO-$d_6$, δ ppm): 8.81 (dd, J=2.4, 0.6 Hz, 1H), 8.73 (dd, J=4.8, 1.6 Hz, 1H), 8.10-8.06 (m, 1H), 7.50 (ddd, J=8.1, 4.8, 0.6 Hz, 1H), 7.32-7.27 (m, 4H), 7.23 (dd, J=8.5, 7.2 Hz, 1H), 7.20-7.15 (m, 2H), 7.05-7.01 (m, 2H), 6.99-6.94 (m, 4H), 6.91-6.87 (m, 2H), 6.32 (d, J=8.5 Hz, 1H), 6.30 (d, J=7.2 Hz, 1H), 6.24 (brs, 1H),4.56 (s, 2H), 4.23 (s, 2H), 3.48 (brs, 2H).
Rf value: 0.77 (n-butanol:acetic acid:water=3:1:1).

[Example 4]

[6-({4-[Diethyl(methyl)ammonio]benzyl}(pyridin-3-ylsulfonyl)aminomethyl)pyridin-2-ylamino]acetic acid hydrochloride

4-(a): 4-({6-[tert-Butoxycarbonyl(2-tert-butoxy-2-oxoethyl)amino]pyridin-2-ylmethyl}-(pyridin-3-ylsulfonyl)aminomethyl)-N,N-diethyl-N-methylbenzene aminium triflate

To 1.9 ml of a tetrahydrofuran solution containing 163 mg (0.474 mmol) of N,N-diethyl-4-hydroxymethyl-N-methylbenzene aminium triflate obtained in Reference example 2 were added 190 mg (0.397 mmol) oftert-butyl (tert-butoxycarbonyl{6-[(pyridin-3-ylsulfonyl)aminomethyl]pyridin-2-yl}amino)acetate obtained by the same manner as in Reference example 1-(f), 0.35 ml (1.4 mmol) oftri-n-butylphosphine and 239 mg (1.39 mmol) of N,N,N',N'-tetramethylazodicarboxamide, and the mixture was stirred at room temperature for 15 hours. After completion of the reaction, water was added to the reaction mixture, and the mixture was extracted with ethyl acetate. The organic layer was washed with a saturated aqueous sodium chloride solution, dried over anhydrous sodium sulfate and concentrated under reduced pressure. The obtained residue was applied to silica gel column chromatography (eluent; chloroform:methanol: triethylamine=90:10:1 (V/V/V)), and the fractions containing the objective material were concentrated under reduced pressure to obtain 135 mg of the title compound as pale yellow foam. (Yield: 42%)
Mass spectrum (FAB, m/z): 654 ($M^+$).
$^1$H-NMR spectrum (DMSO-$d_6$, δ ppm): 8.96 (dd, J=2.4, 0.7 Hz, 1H), 8.82 (dd, J=4.8, 1.6 Hz, 1H), 8.20 (ddd, J=8.1, 2.4, 1.6 Hz, 1H), 7.69-7.50 (m, 5H), 7.45-7.38 (m, 2H), 6.92 (dd, J=6.3, 1.7 Hz, 1H), 4.59 (s, 2H), 4.44 (s, 2H), 4.31 (s, 2H), 4.00-3.68 (m, 4H), 3.40 (s, 3H), 1.45 (s, 9H), 1.39 (s, 9H), 0.93 (t, J=7.1 Hz, 6H).

4-(b): [6-({4-[Diethyl(methyl)ammonio]benzyl}(pyridin-3-ylsulfonyl)aminomethyl)-pyridin-2-ylamino]acetic acid hydrochloride

To 0.2 ml of a tetrahydrofuran solution containing 43 mg (0.53 mmol) of 4-({6-[tert-butoxycarbonyl(2-tert-butoxy-2-oxoethyl)amino]pyridin-2-ylmethyl}(pyridin-3-ylsulfonyl)aminomethyl)-N,N-diethyl-N-methylbenzene aminium triflate obtained in Example 4-(a) was added 50 μl of concentrated hydrochloric acid, and the mixture was stirred at 65°C for 5 hours. After completion of the reaction, the reaction mixture was concentrated under reduced pressure. The obtained residue was applied to reverse phase C18 column chromatography (eluent; acetonitrile:water=1:20→3:7 (V/V)), and the fractions containing the objective material were concentrated under reduced pressure to obtain 15 mg of the title compound as yellow oil. (Yield: 46% as trihydrochloride)
Mass spectrum (FAB, m/z): 498 ($M^+$+1).
$^1$H-NMR spectrum (DMSO-$d_6$, δ ppm): 8.93-8.92 (m, 1H), 8.79 (dd, J=4.8, 1.5 Hz, 1H), 8.21 (ddd, J=8.1, 2.3, 1.5 Hz, 1H), 7.72-7.69 (m, 2H), 7.58 (ddd, J=8.1, 4.8, 0.7 Hz, 1H), 7.52-7.49 (m, 2H), 7.15 (dd, J=8.3, 7.2 Hz, 1H), 6.23 (d, J=7.2 Hz, 1H), 6.19 (d, J=8.3 Hz, 1H), 5.71 (t, J=3.9 Hz, 1H), 4.64 (s, 2H), 4.23 (s, 2H), 4.02-3.92 (m, 2H), 3.82-3.72 (m, 2H), 3.44 (s, 3H), 3.17 (d, J=3.9 Hz, 2H), 0.96 (t, J=7.2 Hz, 6H).
Rf value: 0.07 (n-butanol:acetic acid:water=3:1:1).

[Example 5]

[6-({4-[Butyryl(methyl)amino]benzyl}(pyridin-3-ylsulfonyl)aminomethyl)pyridin-2-ylamino]acetic acid

5-(a): tert-Butyl{tert-butoxycarbonyl[6-({4-[butyryl(methyl)amino]benzyl}(pyridin-3-ylsulfonyl)aminomethyl)pvridin-2-yl]amino}acetate

To 1.3 ml of a methylene chloride solution containing 150 mg (0.251 mmol) of tert-butyl (tert-butoxycarbonyl {6-[(4-methylaminobenzyl)(pyridin-3-ylsulfonyl)amino-methyl]pyridin-2-yl}amino)acetate obtained in Example 1-(a) were

added 70 μl (0.50 mmol) of triethylamine and 31 μl (0.30 mmol) of butyryl chloride under ice-cooling, and the mixture was stirred at room temperature for 3 hours. After completion of the reaction, 1 ml of water and 0.5 ml of a saturated aqueous sodium hydrogen carbonate solution were added to the reaction mixture, and the mixture was extracted with ethyl acetate. After the organic layer was dried over anhydrous sodium sulfate, the mixture was concentrated under reduced pressure. The obtained residue was applied to silica gel column chromatography (eluent; n-hexane:ethyl acetate= 1:1→1:2 (V/V)), and the fractions containing the objective material were concentrated under reduced pressure to obtain 166 mg of the title compound as white foam. (Yield: 99%)
Mass spectrum (FAB, m/z): 668 ($M^+$+1).
$^1$H-NMR spectrum ($CDCl_3$, δ ppm): 8.95-8.93 (m, 1H), 8.72 (dd, J=4.8, 1.6 Hz, 1H), 7.92-7.86 (m, 1H), 7.71 (d, J=8.3 Hz, 1H), 7.49 (dd, J=8.3, 7.2 Hz, 1H), 7.37-7.27 (m, 3H), 7.11-7.05 (m, 2H), 6.85 (d, J=7.2 Hz, 1H), 4.60 (s, 2H), 4.41 (s, 2H), 4.34 (s, 2H), 3.23 (s, 3H), 2.01 (t, J=6.8 Hz, 2H), 1.65-1.53 (m, 2H), 1.52 (s, 9H), 1.43 (s, 9H), 0.84 (t, J=7.3 Hz, 3H).

5-(b): <u>[6-({4-[Butyryl(methyl)amino]benzyl}(pyridin-3-ylsulfonyl)aminomethyl)-pyridin-2-ylamino]acetic acid</u>

To 4.8 ml of a methylene chloride solution containing 162 mg (0.243 mmol) of tert-butyl {tert-butoxycarbonyl [6-({4-[butyryl(methyl)amino]benzyl}(pyridin-3-yl-sulfonyl)aminomethyl)pyridin-2-yl]amino}acetate obtained in Example 5-(a) was added 2.4 ml (9.6 mmol) of 4N hydrogen chloride/1,4-dioxane solution, and the mixture was stirred at room temperature for 7 hours. After completion of the reaction, the reaction mixture was concentrated under reduced pressure, water was added to the obtained residue, and the mixture was adjusted to pH 4.5 with 1N aqueous sodium hydroxide solution and 1N hydrochloric acid. The mixture was extracted with ethyl acetate, the organic layer was dried over anhydrous sodium sulfate and concentrated under reduced pressure to obtain 123 mg of the title compound as pale brown foam. (Yield: 99%) Mass spectrum (FAB, m/z): 512 ($M^+$+1).
$^1$H-NMR spectrum (DMSO-$d_6$, δ ppm): 8.83 (dd, J=2.3, 0.7 Hz, 1H), 8.72 (dd, J=4.9, 1.6 Hz, 1H), 8.06-8.02 (m, 1H), 7.48 (ddd, J=8.1, 4.9, 0.7 Hz, 1H), 7.33 (d, J=8.3 Hz, 2H), 7.24-7.19 (m, 3H), 6.76 (t, J=5.7 Hz, 1H), 6.35 (d, J=8.3 Hz, 1H), 6.32 (d, J=7.2 Hz, 1H), 4.66 (s, 2H), 4.22 (s, 2H), 3.71 (d, J=5.7 Hz, 2H), 3.13 (s, 3H), 1.95 (brs, 2H), 1.50-1.41 (m, 2H), 0.77 (t, J=6.6 Hz, 3H).
Rf value: 0.65 (n-butanol:acetic acid:water=3:1:1).

[Example 6]

<u>[6-({4-[Benzyl(methyl)amino]benzyl}(pyridin-3-ylsulfonyl)aminomethyl)pyridin-2-ylamino]acetic acid</u>

6-(a): <u>tert-Butyl {[6-({4-[benzyl(methyl)amino]benzyl}(pyridin-3-ylsulfonyl)aminomethyl)pyridinn-2-yl]tert-butoxycarbonylamino}acetate</u>

To 1.7 ml of an acetonitrile solution containing 200 mg (0.335 mmol) oftert-butyl (tert-butoxycarbonyl {6-[(4-methylaminobenzyl)(pyridin-3-ylsulfonyl)aminomethyl]pyridin-2-yl}amino)acetate obtained in Example 1-(a) were added 93 mg (0.67 mmol) of potassium carbonate and 48 μl (0.40 mmol) of benzyl bromide, and the mixture was stirred at room temperature for 15 hours. After completion of the reaction, water was added to the reaction mixture, and the mixture was extracted with ethyl acetate. After the organic layer was dried over anhydrous sodium sulfate, the mixture was concentrated under reduced pressure. The obtained residue was applied to silica gel column chromatography (eluent; n-hexane:ethyl acetate=4:1→1:1 (V/V)), and the fractions containing the objective material were concentrated under reduced pressure to obtain 156 mg of the title compound as yellow foam. (Yield: 68%)
Mass spectrum (FAB, m/z): 688 ($M^+$+1).
H-NMR spectrum ($CDCl_3$, δ ppm): 8.93 (dd, J=2.3, 0.6 Hz, 1H), 8.67 (dd, J=4.9, 1.7 Hz, 1H), 7.84 (ddd, J=8.1, 2.3, 1.7 Hz, 1H), 7.70 (d, J=8.5 Hz, 1H), 7.51 (dd, J=8.4, 7.4 Hz, 1H), 7.36-7.16 (m, 6H), 7.06-6.98 (m, 2H), 6.87 (d, J=7.4 Hz, 1H), 6.65-6.58 (m, 2H), 4.51 (s, 2H), 4.45 (s, 2H), 4.39 (s, 2H), 4.36 (s, 2H), 3.00 (s, 3H), 1.52 (s, 9H), 1.41 (s, 9H).

6-(b): <u>[6-({4-[Benzyl(methyl)amino]benzyl}(pyridin-3-ylsulfonyl)aminomethyl-pyridin-2-ylamino]acetic acid</u>

To 4.4 ml of a methylene chloride solution containing 150 mg (0.218 mmol) of tert-butyl {[6-({4-[benzyl(methyl) amino]benzyl}(pyridin-3-ylsulfonyl)aminomethyl)-pyridin-2-yl]tert-butoxycarbonylamino}acetate obtained in Example 6-(a) was added 2.2 ml (8.8 mmol) of 4N hydrogen chloride/1,4-dioxane solution, and the mixture was stirred at room temperature for 12 hours. After completion of the reaction, the reaction mixture was concentrated under reduced pressure, water was added to the obtained residue, and the mixture was adjusted to pH 4.6 with 1N aqueous sodium hydroxide solution and 1N hydrochloric acid. The precipitated solid was collected by filtration and dried under reduced pressure to obtain 111 mg of the title compound as pale orange solid. (Yield: 96%)
Mass spectrum (FAB, m/z): 532 ($M^+$+1).

$^1$H-NMR spectrum (DMSO-$d_6$, δ ppm): 8.77 (dd, J=2.4, 0.7 Hz, 1H), 8.69 (dd, J=4.8, 1.6 Hz, 1H), 7.97 (ddd, J=8.1, 2.4, 1.6 Hz, 1H), 7.44 (ddd, J=8.1, 4.8, 0.7 Hz, 1H), 7.34-7.28 (m, 2H), 7.26-7.17 (m, 4H), 7.06-7.02 (m, 2H), 6.71-6.62 (m, 3H), 6.35 (d, J=8.3 Hz, 1H), 6.30 (d, J=7.0 Hz, 1H), 4.55 (s, 2H), 4.50 (s, 2H), 4.11 (s, 2H), 3.68 (d, J=5.3 Hz, 2H), 2.98 (s, 3H).
Rf value: 0.69 (n-butanol:acetic acid:water=3:1:1).

[Example 7]

[6-({4-[Butyl(methyl)amino]benzyl}(pyridin-2-ylsulfonyl)aminomethyl)pyridin-2-ylamino]acetic acid

7-(a): tert-Butyl (tert-butoxycarbonyl[6-({4-[butyl(methyl)amino]benzyl}(pyridin-2-ylsulfonyl)aminomethyl)pyridin-2-yl]amino)acetate

To 60 ml of a tetrahydrofuran solution containing 1.10 g (5.67 mmol) of 4-[butyl(methyl)amino]benzyl alcohol (see Farmaco, 44, 1167 (1989)), 2.68 g (5.60 mmol) of tert-butyl (tert-butoxycarbonyl {6-[(pyridin-2-ylsulfonyl)aminomethyl]-pyridin-2-yl}amino)acetate obtained in the same manner as in Reference example 3 and 1.50 g (8.71 mmol) of N,N,N',N'-tetramethylazodicarboxamide was added dropwise 2.7 ml (11 mmol) of tri-n-butylphosphine at room temperature over 15 minutes, and the mixture was stirred at the same temperature for 18.5 hours. After completion of the reaction, a saturated aqueous sodium chloride solution was added to the reaction mixture, and the mixture was extracted with ethyl acetate. The organic layer was dried over anhydrous magnesium sulfate, and the mixture was concentrated under reduced pressure. The obtained residue was applied to silica gel column chromatography (eluent; n-hexane:ethyl acetate=2:1 (V/V)), and the fractions containing the objective material were concentrated under reduced pressure to obtain 2.47 g of the title compound as pale yellow oil. (Yield: 68%)
Mass Spectrum (CI, m/z): 654 ($M^+$+1)
$^1$H-NMR spectrum ($CDCl_3$, δ ppm): 8.61-8.57 (m, 1H), 7.83-7.78 (m, 1H), 7.75 (ddd, J=7.6, 7.4, 1.7 Hz, 1H), 7.64 (d, J=8.1 Hz, 1H), 7.46 (dd, J=8.1, 7.7 Hz, 1H), 7.36 (ddd, J=7.4, 4.8, 1.6 Hz, 1H), 7.06-6.99 (m, 2H), 6.91 (d, J=7.7 Hz, 1H), 6.54-6.48 (m, 2H), 4.56 (s, 2H), 4.48 (s, 2H), 4.46 (s, 2H), 3.26 (t, J=7.4 Hz, 2H), 2.88 (s, 3H), 1.52 (s, 9H), 1.48-1.22 (m, 4H), 1.43 (s, 9H), 0.94 (t, J=7.2 Hz, 3H).

7-(b): [6-({4-[Butyl(methyl)amino]benzyl}(pyridin-2-ylsulfonyl)aminomethyl)pyridin-2-ylamino]acetic acid

To 20 ml of a methylene chloride solution containing 2.47 g (3.78 mmol) of tert-butyl (tert-butoxycarbonyl[6-({4-[butyl(methyl)amino]benzyl}(pyridin-2-ylsulfonyl)aminomethyl)pyridin-2-yl]amino)acetate obtained in Example 7-(a) was added 20 ml (80 mmol) of 4N hydrogen chloride/1,4-dioxane solution, and the mixture was stirred at room temperature for 28.5 hours. After completion of the reaction, the reaction mixture was concentrated under reduced pressure, and 20 ml of water was added to the obtained residue. The mixture was adjusted to pH 5.1 with 2N aqueous sodium chloride solution and 1N hydrochloric acid, and the mixture was extracted with ethyl acetate. The organic layer was washed with water, dried over anhydrous sodium sulfate, and concentrated under reduced pressure to obtain 1.64 g of the title compound as yellow foam. (Yield: 87%)
Mass spectrum (FAB, m/z): 498 ($M^+$+1).
H-NMR spectrum (DMSO-$d_6$, δ ppm): 8.62 (ddd, J=4.8, 1.8, 0.9 Hz, 1H), 7.93 (ddd, J=7.7, 7.7, 1.8 Hz, 1H), 7.79-7.76 (m, 1H), 7.56 (ddd, J=7.7, 4.8, 1.1 Hz, 1H), 7.21 (dd, J=8.3, 7.3 Hz, 1H), 7.01-6.96 (m, 2H), 6.73 (t, J=5.7 Hz, 1H), 6.56-6.51 (m, 2H), 6.34 (d, J=8.3 Hz, 1H), 6.28 (d, J=7.3 Hz, 1H), 4.52 (s, 2H), 4.17 (s, 2H), 3.83 (d, J=5.7 Hz, 2H), 3.26 (t, J=7.3 Hz, 2H), 2.83 (s, 3H), 1.48-1.40 (m, 2H), 1.33-1.23 (m, 2H), 0.90 (t, J=7.3 Hz, 3H).
Rf value: 0.56 (n-butanol:acetic acid:water=3:1:1).

[Example 8]

(6-{[4-(Phenylamino)benzyl](pyridin-2-ylsulfonyl)aminomethyl}pyridin-2-ylamino)-acetic acid

8-(a): tert-Butyl ({6-[(4-bromobenzyl)(pyridin-2-ylsulfonyl)aminomethyl]pyridin-2-yl}tert-butoxycarbonylamino)acetate

To 10 ml of an acetonitrile solution containing 1.44 g (3.01 mmol) of tert-butyl (tert-butoxycarbonyl {6-[(pyridin-2-ylsulfonyl)aminomethyl]pyridin-2-yl}amino)acetate obtained in the same manner as in Reference example 3 were added 787 mg (3.15 mmol) of 4-bromobenzyl bromide and 830 mg (6.01 mmol) of potassium carbonate, and the mixture was stirred at room temperature for 16 hours. After completion of the reaction, the reaction mixture was added to 60 ml of water, and the mixture was extracted with toluene. The organic layer was successively washed with water, and then, with a saturated aqueous sodium chloride solution, dried over anhydrous magnesium sulfate, and the mixture was

concentrated under reduced pressure. The obtained residue was applied to silica gel column chromatography (eluent; n-hexane:ethyl acetate=4:1→2:1 (V/V)), and the fractions containing the objective material were concentrated under reduced pressure to obtain 1.77 g of the title compound as pale yellow oil. (Yield: 91%)
Mass Spectrum (CI, m/z): 647($M^{+}$+1).
$^{1}$H-NMR spectrum ($CDCl_3$, δ ppm): 8.59 (ddd, J=4.6, 1.6, 1.0 Hz, 1H), 7.83-7.74 (m, 2H), 7.68-7.62 (m, 1H), 7.48-7.32 (m, 4H), 7.15-7.10 (m, 2H), 6.87 (d, J=7.8 Hz, 1H), 4.66 (s, 2H), 4.44 (s, 2H), 4.43 (s, 2H), 1.52 (s, 9H), 1.42 (s, 9H).

8-(b): tert-Butyl [tert-butoxycarbonyl(6-{[4-(phenylamino)benzyl](pyridin-2-yl-sulfonyl)aminomethyl}pyridin-2-yl)amino] acetate

To 1.0 ml of a toluene solution containing 389 mg (0.601 mmol) of tert-butyl ({6-[(4-bromobenzyl)(pyridin-2-ylsulfonyl)aminomethyl]pyridin-2-yl}tert-butoxy-carbonylamino)acetate obtained in Example 8-(a) were added 66 μl (0.73 mmol) of aniline, 28 mg (0.031 mmol) oftris(dibenzylideneacetone)dipalladium, 36 mg (0.12 mmol) of o-biphenyl-di-tert-butylphosphine and 81 mg (0.84 mmol) of sodium tert-butoxide, and the mixture was stirred at 80°C for 1 hour. After completion of the reaction, to the reaction mixture were added 20 ml of a saturated aqueous ammonium chloride solution and 20 ml of water, and the mixture was extracted with ethyl acetate. The organic layer was washed with a saturated sodium chloride solution, dried over anhydrous magnesium sulfate and concentrated under reduced pressure. The obtained residue was applied to silica gel column chromatography (eluent; n-hexane:ethyl acetate=3:1→2:1 (V/V)), and the fractions containing the objective material were concentrated under reduced pressure to obtain 302 mg of the title compound as pale yellow foam. (Yield: 76%)
Mass spectrum (FAB, m/z): 660 ($M^{+}$+1).
$^{1}$H-NMR spectrum ($CDCl_3$, δ ppm): 8.61 (ddd, J=4.7, 1.7, 1.0 Hz, 1H), 7.83 (ddd, J=7.8, 1.5, 1.0 Hz, 1H), 7.78 (ddd, J=7.8, 7.4, 1.7 Hz, 1H), 7.64 (d, J=8.2 Hz, 1H), 7.46 (dd, J=8.2, 7.6 Hz, 1H), 7.39 (ddd, J=7.4, 4.7, 1.5 Hz, 1H), 7.30-7.22 (m, 3H), 7.13-7.07 (m, 2H), 7.05-7.00 (m, 2H), 6.97-6.87 (m, 4H), 5.68 (s, 1H), 4.62 (s, 2H), 4.49 (s, 2H), 4.46 (s, 2H), 1.52 (s, 9H), 1.43 (s, 9H).

8-(c): (6-{[4-(Phenylamino)benzyl](pyridin-2-ylsulfonyl)aminomethyl}pyridin-2-ylamino)acetic acid

To 297 mg (0.450 mmol) of tert-butyl [tert-butoxycarbonyl(6-{[4-(phenyl-amino)benzyl](pyridin-2-ylsulfonyl)aminomethyl}pyridin-2-yl)amino]acetate obtained in Example 8-(b) was added 2.5 ml (10 mmol) of 4N hydrogen chloride/ 1,4-dioxane solution, and the mixture was stirred at room temperature for 16 hours. After completion of the reaction, the reaction mixture was concentrated under reduced pressure, 20 ml of water was added to the obtained residue, and a pH of the mixture was adjusted to 5.8 with 1N aqueous sodium hydroxide solution and 1N hydrochloric acid. The precipitated solid was collected by filtration, and stirred in 4 ml of tert-butyl methyl ether for 2 hours. The precipitated solid was collected by filtration, and then, dried under reduced pressure to obtain 181 mg of the title compound as pale yellow solid.
(Yield: 80%)
Mass spectrum (FAB, m/z): 504 ($M^{+}$+1).
H-NMR spectrum (DMSO-$d_6$, δ ppm): 8.63 (ddd, J=4.8, 1.7, 0.7 Hz, 1H), 8.14 (s, 1H), 7.95 (ddd, J=7.8, 7.7, 1.7 Hz, 1H), 7.82-7.77 (m, 1H), 7.57 (ddd, J=7.7, 4.8, 1.2 Hz, 1H), 7.26-7.17 (m, 3H), 7.11-6.99 (m, 4H), 6.98-6.90 (m, 2H), 6.85-6.78 (m, 1H), 6.71-6.59 (m, 1H), 6.33 (d, J=8.3 Hz, 1H), 6.29 (d, J=7.3 Hz, 1H), 4.57 (s, 2H), 4.22 (s, 2H), 3.78 (d, J=4.6 Hz, 2H).
Rf value: 0.65(n-butanol:acetic acid:water=3:1:1).

[Example 9]

[6-({4-[Butyl(2,2,2-trifluoroethyl)amino]benzyl}(pyridin-2-ylsulfonyl)aminomethyl)-pyridin-2-ylamino]acetic acid

9-(a): tert-Butyl(tert-butoxycarbonyl[6-({4-[butyl(2,2,2-trifluoroethyl)amino]benzyl}-(pyridin-2-ylsulfonyl)aminomethyl)pyridin-2-yl]amino)acetate

To 14 ml of a tetrahydrofuran solution containing 672 mg (1.40 mmol) of tert-butyl (tert-butoxycarbonyl {6-[(pyridin-2-ylsulfonyl)aminomethyl]pyridin-2-yl}amino)-acetateacetate obtained by the same manner as in Reference example 3 were added 367 mg (1.40 mmol) of 4-[butyl(2,2,2-trifluoroethyl)amino]benzyl alcohol obtained in Reference example 4-(c), 0.88 ml (3.5 mmol) of tri-n-butylphosphine and 363 mg (2.11 mmol) of N,N,N',N'-tetramethylazodicarboxamide, and the mixture was stirred at room temperature for 16 hours. After completion of the reaction, water was added to the reaction mixture, and the mixture was extracted with ethyl acetate. The organic layer was dried over anhydrous magnesium sulfate, and the mixture was concentrated under reduced pressure. The obtained residue was applied to silica gel column chromatography (eluent; n-hexane:ethyl acetate=9:1→7:3 (V/V)), and the fractions containing the

objective material were concentrated under reduced pressure to obtain 468 mg of the title compound as brown oil. (Yield: 53%)

Mass Spectrum (CI, m/z): 722 ($M^+$+1).

$^1$H-NMR spectrum ($CDCl_3$, δ ppm): 8.60-8.57 (m, 1H), 7.82-7.72 (m, 2H), 7.68-7.60 (m, 1H), 7.45 (dd, J=8.3, 7.6 Hz, 1H), 7.36 (ddd, J=7.2, 4.8, 1.6 Hz, 1H), 7.10-7.03 (m, 2H), 6.91 (d, J=7.6 Hz, 1H), 6.62-6.55 (m, 2H), 4.61-4.54 (m, 2H), 4.49-4.46 (m, 4H), 3.81 (q, J=8.9 Hz, 2H), 3.37-3.30 (m, 2H), 1.62-1.48 (m, 2H), 1.52 (s, 9H), 1.43 (s, 9H), 1.42-1.29 (m, 2H), 0.96 (t, J=7.2 Hz, 3H).

9-(b): [6-({4-[Butyl](2,2,2-trifluoroethyl)amino]benzyl}(pyridin-2-ylsulfonyl)aminomethyl)pyridin-2-ylamino]acetic acid

To 4.0 ml of a methylene chloride solution containing 252 mg (0.396 mmol) of tert-butyl (tert-butoxycarbonyl [6-({4-[butyl(2,2,2-trifluoroethyl)amino]benzyl}(pyridin-2-ylsulfonyl)aminomethyl)pyridin-2-yl]amino)acetate obtained in Example 9-(a) was added 4.0 ml (52 mmol) oftrifluoroacetic acid, and the mixture was stirred at room temperature for 4.5 hours. After completion of the reaction, the reaction mixture was concentrated under reduced pressure, 10 ml of water was added to the residue, and a pH of the mixture was adjusted to 4.4 with 1N aqueous sodium hydroxide solution and 0.1N hydrochloric acid. The precipitated solid was collected by filtration, and then, dried under reduced pressure to obtain 165 mg of the title compound as pale brown solid. (Yield: 74%)

Mass spectrum (FAB, m/z): 566 ($M^+$+1).

H-NMR spectrum (DMSO-$d_6$, δ ppm): 8.65-8.58 (m, 1H), 7.93 (ddd, J=7.7, 7.7, 1.7 Hz, 1H), 7.80-7.74 (m, 1H), 7.55 (ddd, J=7.7, 4.6, 1.0 Hz, 1H), 7.19 (dd, J=8.2, 7.2 Hz, 1H), 7.09-7.00 (m, 2H), 6.77-6.59 (m, 3H), 6.33 (d, J=8.2 Hz, 1H), 6.27 (d, J=7.2 Hz, 1H), 4.55 (s, 2H), 4.25-4.03 (m, 2H), 4.18 (s, 2H), 3.79 (d, J=3.4 Hz, 2H), 3.37-3.29 (m, 2H), 1.56-1.40 (m, 2H), 1.37-1.22 (m, 2H), 0.91 (t, J=7.2 Hz, 3H).

Rf value: 0.69 (n-butanol:acetic acid:water=3:1:1).

The compounds used in Examples were synthesized as follows.

[Reference example 1]

tert-Butyl (tert-butoxycarbonyl{6-[(pyridin-3-ylsulfonyl)aminomethyl]pyridin-2-yl}-amino)acetate

1-(a): tert-Butyl [tert-butoxycarbonyl(6-ethoxycarbonylpyridin-2-yl)amino]acetate

To 362 ml of an N,N-dimethylformamide solution containing 15.7 g (0.360 mol) of sodium hydride (55% dispersed material in mineral oil) was added dropwise 300 ml of an N,N-dimethylformamide solution containing 81.2 g (0.305 mol) of ethyl 6-tert-butoxycarbonylaminopyridin-2-carboxylate (see WO2006/074884A) under argon atmosphere and under ice-cooling over 20 minutes, and the mixture was stirred at room temperature for 1 hour. Then, 54.0 ml (0.366 mol) of tert-butyl bromoacetate was added dropwise under ice-cooling over 10 minutes to the mixture, and the mixture was further stirred at room temperature for 1 hour. After completion of the reaction, to the reaction mixture was added an aqueous solution in which 1.77 g (33.0 mmol) of ammonium chloride had been dissolved in 300 ml of water, and the mixture was extracted with toluene. The organic layer was washed with a saturated sodium chloride solution, dried over anhydrous magnesium sulfate and concentrated under reduced pressure. The obtained residue was applied to silica gel column chromatography (eluent; n-hexane:ethyl acetate=9:1→4:1 (V/V)), and the fractions containing the objective material were concentrated under reduced pressure to obtain 108 g of the title compound as pale yellowish oily product. (Yield: 93%)

Mass Spectrum (CI, m/z): 381($M^+$+1).

H-NMR spectrum ($CDCl_3$, δ ppm): 8.04 (d, J=7.8 Hz, 1H), 7.81 (dd, J=7.6, 1.5 Hz, 1H), 7.76 (dd, J=7.8, 7.6 Hz, 1H), 4.67 (s, 2H), 4.40 (q, J=7.1 Hz, 2H), 1.52 (s, 9H), 1.45 (s, 9H), 1.40 (t, J=7.1 Hz, 3H).

1-(b): tert-Butyl [tert-butoxycarbonyl(6-hydroxymethylpyridin-2-yl)amino]acetate

To 195 ml of an ethanol solution containing 98.8 g (0.260 mol) oftert-butyl [tert-butoxycarbonyl(6-ethoxycarbonylpyridin-2-yl)amino]acetate obtained in Reference example 1-(a) was added dropwise 195 ml of an ethanol solution containing 34.6 g (0.312 mol) of calcium chloride under ice-cooling over 20 minutes. After completion of the dropwise addition, 105 ml (0.315 mol) of 3M sodium borohydride/- tetraethylene glycol dimethyl ether solution was added dropwise to the mixture at 35°C or lower over 20 minutes, and the mixture was further stirred at room temperature for 15 minutes. After completion of the reaction, the reaction mixture was added dropwise to 195 ml of an aqueous solution containing 17.8 ml of acetic acid in water under ice-cooling over 10 minutes, and the mixture was stirred at room temperature for 1 hour. Then, 315 ml of water was added to the mixture, and the mixture was extracted with toluene. The organic layer was successively washed with a saturated aqueous sodium hydrogen carbonate solution, water, and then, a saturated

aqueous sodium chloride solution, and the mixture was concentrated under reduced pressure. The obtained residue was applied to silica gel column chromatography (eluent; n-hexane:ethyl acetate=4:1→3:2 (V/V)), and the fractions containing the objective material were concentrated under reduced pressure to obtain 81.1 g of the title compound as pale yellow oil. (Yield: 92%)
Mass Spectrum (CI, m/z): 339 ($M^+$+1).
H-NMR spectrum ($CDCl_3$, δ ppm): 7.74 (d, J=8.2 Hz, 1H), 7.63 (dd, J=8.2, 7.4 Hz, 1H), 6.93-6.98 (m, 1H), 4.68-4.65 (m, 2H), 4.54 (s, 2H), 3.39 (t, J=5.3 Hz, 1H), 1.54 (s, 9H), 1.46 (s, 9H).

1-(c): tert-Butyl [tert-butoxycarbonyl(6-formylpyridin-2-yl)amino]acetate

To 130 ml of a methylene chloride solution containing 12.9 g (30.4 mmol) of Dess-martin reagent were added dropwise 50 ml of a methylene chloride solution containing 10.0 g (29.6 mmol) of tert-butyl [tert-butoxycarbonyl(6-hydroxymethyl-pyridin-2-yl)amino]acetate obtained in Reference example 1-(b) under argon atmosphere and under ice-cooling over 20 minutes. After completion of the dropwise addition, the mixture was stirred at room temperature for 2 hours. After completion of the reaction, to the reaction mixture was added 305 ml of 0.1 % aqueous sodium thiosulfate solution, and the mixture was extracted with methylene chloride. The organic layer was successively washed with 0.5N aqueous sodium hydroxide solution, and then, a saturated aqueous sodium chloride solution, dried over anhydrous magnesium sulfate, and concentrated under reduced pressure to obtain 9.61 g of the title compound as pale yellow oil substantially quantitatively.
Mass spectrum (EI, m/z): 336 ($M^+$).
H-NMR spectrum (DMSO-$d_6$, δ ppm): 9.82 (s, 1H), 8.11-7.99 (m, 2H), 7.68 (dd, J=6.6, 1.5 Hz, 1H), 4.58 (s, 2H), 1.48 (s, 9H), 1.42 (s, 9H).

1-(d): tert-Butyl [tert-butoxycarbonyl(6-hydroxyiminomethylpyridin-2-yl)amino]acetate

To 29 ml of a methanol solution containing 2.88 g (8.56 mmol) oftert-butyl [tert-butoxycarbonyl(6-formylpyridin-2-yl)amino]acetate obtained in Reference example 1-(c) were added 0.650 g (9.35 mmol) of hydroxyl ammonium chloride and 3.5 ml (43 mmol) of pyridine, and the mixture was stirred at room temperature for 1 hour. After completion of the reaction, the reaction mixture was concentrated under reduced pressure. Ethyl acetate was added to the obtained residue, and the mixture was successively washed with a 5% aqueous potassium hydrogen sulfate solution, a saturated aqueous sodium hydrogen carbonate solution, and then, a saturated aqueous sodium chloride solution, dried over anhydrous magnesium sulfate, and concentrated under reduced pressure. The obtained residue was applied to silica gel column chromatography (eluent; n-hexane:ethyl acetate=3:2 (V/V)), and the fractions containing the objective material were concentrated under reduced pressure to obtain 2.76 g of the title compound as colorless oil. (Yield: 92%)
Mass spectrum (EI, m/z): 351 ($M^+$).
$^1$H-NMR spectrum ($CDCl_3$, δ ppm): 8.06 (s, 1H), 7.91 (s, 1H), 7.85 (d, J=8.2 Hz, 1H), 7.65 (dd, J=8.2, 7.6 Hz, 1H), 7.47 (dd, J=7.6, 0.7 Hz, 1H), 4.59 (s, 2H), 1.53 (s, 9H), 1.45 (s, 9H).

1-(e): tert-Butyl [(6-aminomethylpyridin-2-yl)tert-butoxycarbonylamino]acetate

To 49 ml of an ethanol solution containing 2.75 g (7.83 mmol) of tert-butyl [tert-butoxycarbonyl(6-hydroxyiminomethylpyridin-2-yl)amino]acetate obtained in Reference example 1-(d) was added 0.98 g of 10% palladium-active carbon (50% hydrate), and the mixture was stirred under 1 atm hydrogen atmosphere at room temperature for 1 hour. After completion of the reaction, insoluble materials were filtered off, and the filtrate was concentrated under reduced pressure to obtain 2.48 g of the title compound as colorless oil. (Yield: 94%)
Mass Spectrum (CI, m/z): 338 ($M^+$+1).
$^1$H-NMR spectrum ($CDCl_3$, δ ppm): 7.68 (d, J=8.3 Hz, 1H), 7.58 (dd, J=8.3, 7.4 Hz, 1H), 6.91 (d, J=7.4 Hz, 1H), 4.57 (s, 2H), 3.85 (s, 2H), 1.53 (s, 9H), 1.46 (s, 9H).

1-(f): tert-Butyl (tert-butoxycarbonyl{6-[(pyridin-3-ylsulfonyl)aminomethyl]pyridin-2-yl}amino)acetate

To 14 ml of a methylene chloride solution containing 0.640 g (3.60 mmol) of 3-pyridylsulfonyl chloride were added 1.20 g (3.56 mmol) of tert-butyl [(6-aminomethylpyridin-2-yl)tert-butoxycarbonylamino]acetate obtained in Reference example 1-(e) and 2.24 ml (16.2 mmol) of triethylamine, and the mixture was stirred at room temperature for 1 hour. After completion of the reaction, to the reaction mixture was added 5% aqueous potassium hydrogen sulfate solution, and the mixture was extracted with chloroform. The organic layer was successively washed with a saturated aqueous sodium hydrogen carbonate solution, and then, a saturated aqueous sodium chloride solution, dried over anhydrous magnesium sulfate, and concentrated under reduced pressure. The obtained residue was applied to silica

gel column chromatography (eluent; n-hexane:ethyl acetate=1:1→1:2 (V/V)), and the fractions containing the objective material were concentrated under reduced pressure to obtain 1.45 g of the title compound as colorless oil. (Yield: 85%)
Mass Spectrum (CI, m/z): 479 ($M^+$+1).
H-NMR spectrum ($CDCl_3$, δ ppm): 9.06 (d, J=2.2 Hz, 1H), 8.71 (dd, J=4.6, 1.5 Hz, 1H), 8.13-8.08 (m, 1H), 7.68 (d, J=8.2 Hz, 1H), 7.52 (dd, J=8.2, 7.4 Hz, 1H), 7.38-7.32 (m, 1H), 6.77 (d, J=7.4 Hz, 1H), 5.80 (t, J=5.1 Hz, 1H), 4.40 (s, 2H), 4.24 (d, J=5.1 Hz, 2H), 1.53 (s, 9H), 1.46 (s, 9H).

[Reference example 2]

N,N-Diethyl-4-hydroxymethyl-N-methylbenzene aminium triflate

To 7.4 ml of a methylene chloride solution containing 331 mg (1.85 mmol) of 4-diethylaminobenzyl alcohol (see Journal of Molecular Structure, 829, 202 (2007)) was added 0.20 ml (3.7 mmol) of methyl trifluoromethanesulfonate under ice-water cooling, and the mixture was stirred at the same temperature for 15 hours. After completion of the reaction, the reaction mixture was concentrated under reduced pressure to obtain 646 mg of the title compound as yellow oil quantitatively.
Mass spectrum (FAB, m/z): 194 ($M^+$).
$^1$H-NMR spectrum (DMSO-$d_6$, δ ppm): 7.78-7.71 (m, 2H), 7.60-7.52 (m, 2H), 4.58 (s, 2H), 4.08-3.93 (m, 2H), 3.87-3.73 (m, 2H), 3.45 (s, 3H), 0.99 (t, J=7.1 Hz, 6H).

[Reference example 3]

tert-Butyl (tert-butoxycarbonyl{6-[(pyridin-2-ylsulfonyl)aminomethyl]pyridin-2-yl}-amino)acetate

Reaction and post-treatment were carried out in accordance with the procedures of Reference example 1-(f) except for using 1.20 g (3.56 mmol) of tert-butyl [(6-aminomethylpyridin-2-yl)tert-butoxycarbonylamino]acetate obtained in Reference example 1-(e), and using 640 mg (3.60 mmol) of 2-pyridylsulfonyl chloride in place of 3-pyridylsulfonyl chloride, to obtain 1.46 g of the title compound as white solid. (Yield: 86%)
Mass Spectrum (APCI, m/z): 479 ($M^+$+1).
$^1$H-NMR spectrum ($CDCl_3$, δ ppm): 8.56 (ddd, J=4.7, 1.7, 0.9 Hz, 1H), 7.97 (ddd, J=7.8, 1.1, 0.9 Hz, 1H), 7.84 (ddd, J=7.8, 7.7, 1.7 Hz, 1H), 7.68 (d, J=8.4 Hz, 1H), 7.52 (dd, J=8.4, 7.4 Hz, 1H), 7.40 (ddd, J=7.7, 4.7, 1.1 Hz, 1H), 6.84 (dd, J=7.4, 0.5 Hz, 1H), 5.86 (t, J=5.6 Hz, 1H), 4.48 (s, 2H), 4.36 (d, J=5.6 Hz, 2H), 1.53 (s, 9H), 1.45 (s, 9H).

[Reference example 4]

4-[Butyl(2,2,2-trifluoroethyl)amino]benzyl alcohol

4-(a): Ethyl 4-(N-butyl-2,2,2-trifluoroacetamide)benzoate

To 27 ml of a methylene chloride solution containing 4.43 g (20.0 mmol) of ethyl 4-(butylamino)benzoate was added 3.69 ml (26.5 mmol) of triethylamine under ice-cooling, and then, 3.54 ml (25.3 mmol) of trifluoroacetic anhydride was added dropwise to the mixture, and the mixture was stirred at room temperature for 3 hours. After completion of the reaction, water was added to the reaction mixture, and the mixture was extracted with methylene chloride. The organic layer was washed with a saturated aqueous sodium chloride solution, dried over anhydrous magnesium sulfate, and concentrated under reduced pressure to obtain 6.37 g of the title compound as colorless oil substantially quantitatively.
Mass Spectrum (CI, m/z): 318 ($M^+$+1).
$^1$H-NMR spectrum ($CDCl_3$, δ ppm): 8.15-8.09 (m, 2H), 7.32-7.26 (m, 2H), 4.41 (q, J=7.2 Hz, 2H), 3.79-3.71 (m, 2H), 1.59-1.48 (m, 2H), 1.41 (t, J=7.2 Hz, 3H), 1.38-1.28 (m, 2H), 0.90 (t, J=7.2 Hz, 3H).

4-(b): Ethyl 4-[butyl(2,2,2-trifluoroethyl)amino]benzoate

To 6.8 ml of a tetrahydrofuran solution containing 2.01 g (6.62 mmol) of ethyl 4-(N-butyl-2,2,2-trifluoroacetamide) benzoate obtained in Reference example 4-(a) was added 1.88 ml (19.8 mmol) of borane·dimethylsulfide complex, and the mixture was stirred at 50°C for 5.5 hours. After completion of the reaction, methanol was added dropwise to the mixture under ice-cooling, then, water was added to the same, and the mixture was extracted with ethyl acetate. The organic layer was washed with a saturated sodium chloride solution, dried over anhydrous magnesium sulfate and concentrated under reduced pressure. The obtained residue was applied to silica gel column chromatography (eluent; n-hexane:ethyl acetate=49:1 (V/V)), and the fractions containing the objective material were concentrated under reduced

pressure to obtain 760 mg of the title compound as colorless oil. (Yield: 38%)
Mass Spectrum (CI, m/z): 304 ($M^+$+1).
$^1$H-NMR spectrum ($CDCl_3$, δ ppm):7.97-7.89 (m, 2H), 6.78-6.70 (m, 2H), 4.33 (q, J=7.2 Hz, 2H), 3.93 (q, J=8.8 Hz, 2H), 3.48-3.41 (m, 2H), 1.68-1.56 (m, 2H), 1.43-1.31 (m, 2H), 1.36 (t, J=7.2 Hz, 3H), 0.97 (t, J=7.3 Hz, 3H).

4-(c): 4-[Butyl(2,2,2-trifluoroethyl)amino]benzyl alcohol

To 6.6 ml of a tetrahydrofuran solution containing 458 mg (1.51 mmol) of ethyl 4-[butyl(2,2,2-trifluoroethyl) amino]benzoate obtained in 4-(b) was added dropwise 2.51 ml (2.51 mmol) of 1M lithium aluminum hydride/tetrahydrofuran solution at room temperature, and the mixture was stirred at the same temperature for 1 hour. After completion of the reaction, to the reaction mixture were added dropwise under ice-cooling, 95 μl of water, 95 μl of a 4N aqueous sodium chloride solution, and 286 μl of water successively in this order, and the mixture was filtered. The filtrate was concentrated under reduced pressure to obtain 367 mg of the title compound as colorless oil. (Yield: 93%)
Mass Spectrum (CI, m/z): 262 ($M^+$+1).
$^1$H-NMR spectrum ($CDCl_3$, δ ppm):7.28-7.22 (m, 2H), 6.80-6.72 (m, 2H), 4.58 (d, J=5.9 Hz, 2H), 3.86 (q, J=9.0 Hz, 2H), 3.42-3.35 (m, 2H), 1.66-1.56 (m, 2H), 1.43-1.29 (m, 2H), 0.96 (t, J=7.3 Hz, 3H).

[Test example 1]

Measurement of EP2 receptor binding action

Measurement of EP2 receptor binding action was carried out in compliance with the method of Abramovitz et al. (Biochimica et Biophysica Acta, 1483, 285 (2000)). A test compound dissolved in dimethylsulfoxide and [$^3$H]prostaglandin $E_2$ (NET-428, PerkinElmer) (final concentration: 10 nM) were added to a buffer solution (10 mM MES-KOH (pH 6.0), 10 mM $MgCl_2$, 1 mM EDTA) in which was suspended 10 μg of a membrane fraction of HEK293 cells expressing human EP2 receptor (ES-562-M, available from Euroscreen) followed by incubating at 30°C for 60 minutes. The membrane fraction was recovered on glass fiber filter paper (GF/B, Whatmann) using a cell harvester (M30R, Brandel), and after washing with buffer solution (10 mM MES-KOH (pH 6.0), 10 mM $MgCl_2$), radioactivity was measured with a liquid scintillation analyzer (2000CA, Packard). The concentration of test compound required to replace 50% of the [$^3$H]prostaglandin $E_2$ bound to the receptor ($IC_{50}$ value) was calculated using EXSAS (Ver. 7.1.6, Arm Systex), and the inhibition constant (Ki value) was determined using the formula indicated below.

$$Ki=IC_{50}/(1+([^3H]\text{prostaglandin } E_2 \text{ concentration}/Kd))$$

The dissociation constant (Kd value) was calculated by Scatchard analysis.
The test result is shown in Table 1.
[Table 1]

| Test compound Example No. | Ki value (nM) of EP2 Receptor Binding Action |
|---|---|
| Example 7 | 10 |

In this test, the compound of the present invention demonstrated superior EP2 receptor binding action.

[Test example 2]

Measurement of EP2 Agonist Activity

Measurement of EP2 agonist activity was carried out in compliance with the method of Wilson et al. (European Journal of Pharmacology, 501, 49 (2004)). HEK293 cells expressing human EP2 receptor (ES-562-C, available from Euroscreen) were cultured in MEM medium containing 10% FBS and seeded at 2 x $10^4$ cells per well of a 96-well plate. On the following day, the medium was replaced with serum-free MEM medium containing 3-isobutyl-1-methylxanthine (final concentration: 500 μM) and after culturing for 30 minutes, a test compound dissolved in dimethylsulfoxide was added followed by allowing to stand undisturbed in a carbon dioxide incubator. After 30 minutes, the amount of cAMP in the cells was measured with a cAMP Biotrak EIA System kit (available from GE Healthcare Biosciences). The concentration of test compound required to increase the amount of cAMP to 50% of the maximum increase ($EC_{50}$ value) was calculated by non-linear regression of the test compound concentration and amount of cAMP using EXSAS.
The test result is shown in Table 2.

[Table 2]

| Test compound Example No. | EP2 agonist activity $EC_{50}$ value (nM) |
|---|---|
| Example 7 | 1.2 |

In this test, the compound of the present invention demonstrated superior EP2 agonist activity.

[Test example 3]

Isolated guinea pig trachea relaxation test

The tracheas were isolated from guinea pigs (Hartley, male, age 7 to 9 weeks, supplier: Nippon SLC) followed by cutting as rings containing cartilage. The trachea specimens were suspended in Krebs solution containing 3 μM indomethacin while applying a load of 1.0 g, and changes in tension were measured through an FD pickup (TB-611T, Nippon Kohden). The trachea specimens were then warmed to 37°C and perfused with a mixed gas consisting of 95% oxygen and 5% carbon dioxide. After causing the trachea specimen to contract by electrical field stimulation (SEN-3401, Nippon Kohden) and the contractive reaction being stable, a test compound dissolved in dimethylsulfoxide was added to cause the trachea specimen to relax. The inhibiting rate to the contractive reaction before adding the compound was calculated.
The test results are shown in Table 3.

[Table 3]

| Test compound Example No. | Contraction-inhibiting rate (%) |
|---|---|
| Example 1 | 95 (10 μM) |
| Example 2 | 86 (10 μM) |
| Example 4 | 81 (10 μM) |
| Example 5 | 92 (10 μM) |
| Example 6 | 94 (10 μM) |
| Example 7 | 84 (0.03 μM) |
| (Numeral in the parentheses shows a test compound concentration.) | |

In this test, compounds of the present invention demonstrated superior trachea contraction-inhibiting activity.

Preparation example

(Preparation example 1) (Hard capsule preparation)

50 mg of powdered compound of Example 7, 128.7 mg of lactose, 70 mg of cellulose and 1.3 mg of magnesium stearate are mixed and passed through a 60 mesh sieve followed by placing 250 mg of the powder in a No. 3 gelatin capsule to obtain a capsule preparation.

(Preparation example 2) (Tablet preparation)

50 mg of the compound of Example 7, 124 mg of lactose, 25 mg of cellulose and 1 mg of magnesium stearate are mixed and formed into a tablet with a tablet-making machine to obtain a tablet preparation weighing 200 mg of the mixture per tablet. This tablet preparation can be provided with a sugar coating as necessary.

INDUSTRIAL APPLICABILITY

Since the aniline compound represented by the formula (1) of the present invention, or a pharmaceutically acceptable salt thereof, demonstrates superior bronchodilatory action based on potent EP2 agonistic action, while also having superior properties as a pharmaceutical composition in terms of tissue distribution, bioavailability (BA), fast-acting pharmaceutical effect, sustained pharmaceutical effect, solubility, physical stability, drug interaction, toxicity and the like, it is preferably useful as a pharmaceutical for treatment or prevention of respiratory diseases (such as asthma, COPD, bronchitis, emphysema, pulmonary fibrosis, acute respiratory distress syndrome (ARDS), cystic fibrosis and pulmonary hypertension), and moreover, is also useful as a pharmaceutical for treatment and/or prevention of diseases for which EP2 agonistic action is thought to be useful (such as bone diseases, gastric ulcer, hypertension and glaucoma).

**Claims**

**1.** An aniline compound represented by the formula (I):

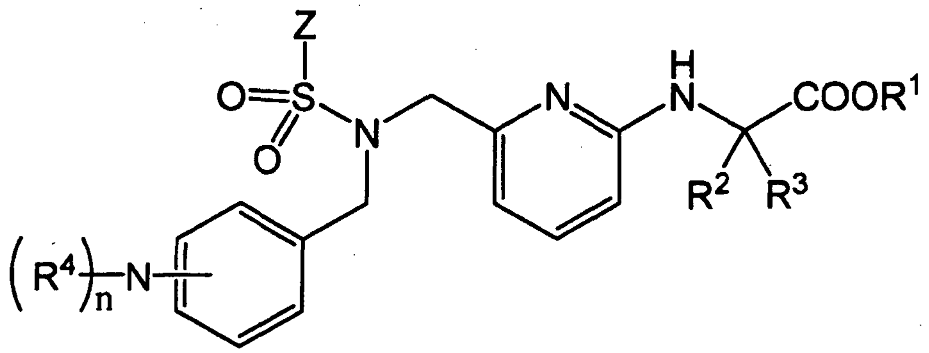

(I)

wherein

$R^1$ represents a hydrogen atom or a $C_1$-$C_6$ alkyl group which may be substituted by a halogeno group,
$R^2$ and $R^3$ each independently represent a hydrogen atom or a $C_1$-$C_6$ alkyl group,
$R^4$ represents a hydrogen atom, or, a $C_1$-$C_6$ alkyl group, a $C_2$-$C_6$ alkenyl group, a $C_2$-$C_6$ alkynyl group, a $C_3$-$C_8$ cycloalkyl group, a $C_2$-$C_6$ alkanoyl group, an arylcarbonyl group, a heteroarylcarbonyl group, a $C_1$-$C_6$ alkylsulfonyl group, an arylsulfonyl group, a heteroarylsulfonyl group, an aminosulfonyl group, a $C_1$-$C_6$ alkylaminosulfonyl group, an arylaminosulfonyl group, a heteroarylaminosulfonyl group, an aminocarbonyl group, a $C_1$-$C_6$ alkylaminocarbonyl group, an arylaminocarbonyl group, a heteroarylaminocarbonyl group, a $C_1$-$C_6$ alkoxycarbonyl group, an aryloxycarbonyl group, a heteroaryloxycarbonyl group, an aryl group, a heteroaryl group or a heterocyclic group, each of which may be substituted by the same or different 1 to 3 groups selected from a substituent group $\alpha$,
n is an integer of 2 or 3, $R^4$s may be the same or different from each other,
when n is 3, $R^4$s are $C_1$-$C_6$ alkyl groups,
Z represents a $C_1$-$C_6$ alkyl group, a $C_2$-$C_6$ alkenyl group, a $C_2$-$C_6$ alkynyl group, a $C_3$-$C_8$ cycloalkyl group, a $C_5$-$C_8$ cycloalkenyl group, an aryl group, a heteroaryl group or a heterocyclic group, each of which may be substituted by the same or different 1 to 3 groups selected from a substituent group $\alpha$,
a substituent contained in the substituent group $\alpha$ are a hydroxy group, an oxo group, a halogeno group, a cyano group, a nitro group, a formyl group, a $C_1$-$C_6$ alkyl group, a $C_7$-$C_{12}$ aralkyl group, a $C_2$-$C_6$ alkenyl group, a $C_2$-$C_6$ alkynyl group, a $C_1$-$C_6$ alkoxy group, a $C_1$-$C_6$ alkoxy-$C_1$-$C_6$ alkyl group, a $C_7$-$C_{12}$ aralkyloxy group, a $C_3$-$C_8$ cycloalkyl group, a $C_3$-$C_8$ cycloalkyl-$C_1$-$C_6$ alkyl group, a $C_5$-$C_8$ cycloalkenyl group, a $C_2$-$C_6$ alkanoyl group, a $C_3$-$C_8$ cycloalkyl-$C_2$-$C_6$ alkanoyl group, a $C_2$-$C_6$ alkanoyl-$C_1$-$C_6$ alkyl group, an arylcarbonyl group, a heteroarylcarbonyl group, a carboxy group, a $C_1$-$C_6$ alkoxycarbonyl group, a $C_1$-$C_6$ alkylthio group, an arylthio group, a heteroarylthio group, a $C_1$-$C_6$ alkylsulfinyl group, an arylsulfinyl group, a heteroarylsulfinyl group, a $C_1$-$C_6$ alkylsulfonyl group, an arylsulfonyl group, a heteroarylsulfonyl group, an aminosulfonyl group, a $C_1$-$C_6$ alkylaminosulfonyl group, a di-($C_1$-$C_6$ alkyl)aminosulfonyl group (two alkyl groups may form a 4- to 8-membered ring in combination), an arylaminosulfonyl group, a heteroarylaminosulfonyl group, an amino group, a $C_1$-$C_6$ alkylamino group, a di-($C_1$-$C_6$ alkyl)amino group (two alkyl groups may form a 4- to 8-membered ring in combination), a tri-($C_1$-$C_6$ alkyl)ammonium group (two alkyl groups among three may be combined to form a 4- to

8-membered ring), a $C_2$-$C_6$ alkenylamino group, a $C_2$-$C_6$ alkynylamino group, a $C_3$-$C_8$cyclo alkylamino group, an arylamino group, a heteroarylamino group, a $C_1$-$C_6$ alkylsulfonylamino group, an arylsulfonylamino group, a heteroarylsulfonylamino group, an aminosulfonylamino group, a $C_1$-$C_6$ alkylaminosulfonylamino group, a di-($C_1$-$C_6$ alkyl)aminosulfonylamino group (two alkyl groups may form a 4- to 8-membered ring in combination), an arylaminosulfonylamino group, a heteroarylaminosulfonylamino group, a $C_2$-$C_6$ alkanoylamino group, an arylcarbonylamino group, a heteroarylcarbonylamino group, an aminocarbonylamino group, a $C_1$-$C_6$ alkylaminocarbonylamino group, a di-($C_1$-$C_6$ alkyl)aminocarbonylamino group (two alkyl groups may form a 4- to 8-membered ring in combination), an arylaminocarbonylamino group, a heteroarylaminocarbonylamino group, a $C_1$-$C_6$ alkoxycarbonylamino group, an aryloxycarbonylamino group, a heteroaryloxycarbonylamino group, a carbamoyl group, a $C_1$-$C_6$ alkylcarbamoyl group, a di-($C_1$-$C_6$ alkyl)carbamoyl group (two alkyl groups may form a 4- to 8-membered ring in combination), an arylcarbamoyl group, a heteroarylcarbamoyl group, an aryl group, a heteroaryl group, an aryloxy group, a heteroaryloxy group and a heterocyclic group,

in the substituents in the substituent group $\alpha$, when it contains an alkyl group, an alkenyl group, an alkynyl group, a cycloalkyl group, a cycloalkenyl group, an aryl group, a heteroaryl group or a heterocyclic group portion, it may be further substituted by the same or different 1 to 3 substituents selected from the substituent group $\alpha$.

or a pharmaceutically acceptable salt thereof.

**2.** The aniline compound or a pharmaceutically acceptable salt thereof according to Claim 1, wherein $R^2$ and $R^3$ each independently represent a hydrogen atom or a $C_1$-$C_4$ alkyl group.

**3.** The aniline compound or a pharmaceutically acceptable salt thereof according to Claim 1 or 2 wherein $R^4$ is a hydrogen atom, or, a $C_1$-$C_6$ alkyl group, a $C_3$-$C_5$ alkenyl group, a $C_3$-$C_5$ alkynyl group, a $C_3$-$C_6$ cycloalkyl group, a $C_2$-$C_4$ alkanoyl group, a benzoyl group, a $C_1$-$C_4$ alkylsulfonyl group, a phenylsulfonyl group, an aminosulfonyl group, a $C_1$-$C_4$ alkylaminosulfonyl group, a phenylsulfamoyl group, a $C_1$-$C_4$ alkylaminocarbonyl group, a phenylcarbamoyl group, a $C_1$-$C_4$ alkoxycarbonyl group, a phenoxycarbonyl group, a phenyl group, a thienyl group, a pyrazolyl group, a thiazolyl group, a pyridyl group, a benzofuryl group, a benzothienyl group or a completely saturated heterocyclic group, each of which may be substituted by a group(s) selected from the group consisting of a halogeno group, a $C_1$-$C_4$ alkoxy group, a $C_3$-$C_6$ cycloalkyl group, a $C_1$-$C_4$ alkoxycarbonyl group, a $C_1$-$C_4$ alkylthio group, a $C_1$-$C_4$ alkylsulfinyl group, a $C_1$-$C_4$ alkylsulfonyl group, a $C_1$-$C_4$ alkylamino group, a di-($C_1$-$C_4$ alkyl)amino group, a $C_2$-$C_4$ alkanoylamino group, a $C_1$-$C_4$ alkoxycarbonylamino group, an aryl group, a heteroaryl group and a completely saturated heterocyclic group.

**4.** The aniline compound or a pharmaceutically acceptable salt thereof according to any one of Claims 1 to 3, wherein Z is a $C_1$-$C_4$ alkyl group, a $C_3$-$C_6$ cycloalkyl group, a $C_5$-$C_6$ cycloalkenyl group, a $C_3$-$C_5$ alkenyl group, a $C_3$-$C_5$ alkynyl group, a completely saturated heterocyclic group, each of which may be substituted by a group(s) selected from the group consisting of a halogeno group, a $C_7$-$C_{12}$ aralkyl group, a $C_1$-$C_4$ alkoxy group, a $C_7$-$C_{12}$ aralkyloxy group, a $C_3$-$C_6$ cycloalkyl group, a $C_5$-$C_6$ cycloalkenyl group, an arylcarbonyl group, a $C_1$-$C_4$ alkylamino group, a di-($C_1$-$C_4$ alkyl)amino group, an arylamino group, an aryl group, a heteroaryl group, an aryloxy group and a completely saturated heterocyclic group, or, a phenyl group or a heteroaryl group, each of which may be substituted by a group (s) selected from the group consisting of a halogeno group, a cyano group, a nitro group, a formyl group, a $C_1$-$C_4$ alkyl group, halogeno-$C_1$-$C_4$ alkyl group, a $C_7$-$C_{12}$ aralkyl group, a $C_7$-$C_{12}$ aralkyloxy group, a $C_3$-$C_6$ cycloalkyl group, a $C_1$-$C_4$ alkoxy group, a $C_1$-$C_4$ alkoxycarbonyl group, a $C_1$-$C_4$ alkylthio group, a $C_1$-$C_4$ alkylamino group, a di-($C_1$-$C_4$ alkyl)amino group, an arylamino group, a $C_2$-$C_4$ alkanoylamino group, a $C_1$-$C_4$ alkoxycarbonylamino group, an aryl group, a heteroaryl group, an aryloxy group and a completely saturated heterocyclic group.

**5.** The aniline compound or a pharmaceutically acceptable salt thereof according to Claim 3, wherein $R^4$ is a hydrogen atom, or, a $C_1$-$C_4$ alkyl group, a 2-propenyl group, a 2-propynyl group, a cyclopropyl group, a $C_2$-$C_4$ alkanoyl group, a benzoyl group, a $C_1$-$C_4$ alkylsulfonyl group, a $C_1$-$C_4$ alkylaminosulfonyl group, a $C_1$-$C_4$ alkylaminocarbonyl group, a phenylcarbamoyl group, a $C_1$-$C_4$ alkoxycarbonyl group, a phenoxycarbonyl group, a phenyl group, a thienyl group, a pyridyl group, a pyrrolidinyl group or a piperidinyl group, each of which may be substituted by a group(s) selected from the group consisting of a fluoro group, a chloro group, a methoxy group, a cyclopropyl group, a methoxycarbonyl group, a $C_1$-$C_4$ alkylsulfonyl group, a dimethylamino group, a $C_2$-$C_4$ alkanoylamino group, a methoxycarbonylamino group, a phenyl group, a thienyl group, a pyridyl group, a pyrrolidinyl group and a piperidinyl group.

**6.** The aniline compound or a pharmaceutically acceptable salt thereof according to Claim 5, wherein $R^4$ is a hydrogen atom, a trifluoromethyl group, a 2,2,2-trifluoroethyl group, a benzyl group, a 1-phenylethyl group, a 2-phenylethyl group, a methyl group, an ethyl group, a propyl group, a butyl group, a cyclopropyl group, an acetyl group, a propanoyl

group, a butanoyl group, a benzoyl group, a methylsulfonyl group, an ethylsulfonyl group, a propylsulfonyl group, a methylaminosulfonyl group, a methylaminocarbonyl group, a methoxycarbonyl group, a phenoxycarbonyl group, a phenyl group or a piperidinyl group.

**7.** The aniline compound or a pharmaceutically acceptable salt thereof according to Claim 4, wherein Z is a $C_1$-$C_4$ alkyl group, a 2-propynyl group, a 2-butynyl group, a cyclopropyl group, a cyclobutyl group, a cyclopentenyl group, each of which may be substituted by a group(s) selected from the group consisting of a fluoro group, a chloro group, a benzyl group, a methoxy group, a benzyloxy group, a cyclopropyl group, a cyclopentenyl group, a benzoyl group, a methylamino group, a dimethylamino group, an anilino group, a phenyl group, a thienyl group, a pyridyl group, a phenoxy group, a pyrrolidinyl group and a piperidinyl group, or, a phenyl group, a thienyl group, a pyrazolyl group, a thiazolyl group or a pyridyl group, each of which may be substituted by a group(s) selected from the group consisting of a fluoro group, a chloro group, a cyano group, a nitro group, a methyl group, a ethyl group, a trifluoromethyl group, a benzyl group, a benzyloxy group, a cyclopropyl group, a methoxy group, a methoxycarbonyl group, a methylthio group, a methylamino group, a dimethylamino group, an anilino group, an acetylamino group, a methoxycarbonylamino group, a phenyl group, a phenoxy group, an azetidinyl group, a pyrrolidinyl group and a piperidinyl group.

**8.** The aniline compound or a pharmaceutically acceptable salt thereof according to Claim 1, wherein
$R^1$ is a hydrogen atom, a methyl group, an ethyl group, a propyl group, an isopropyl group or a tert-butyl group,
$R^2$ and $R^3$ each independently represent a hydrogen atom or a methyl group, $R^4$ is a hydrogen atom, or, a $C_1$-$C_6$ alkyl group, a $C_3$-$C_5$ alkenyl group, a $C_3$-$C_5$ alkynyl group, a $C_3$-$C_6$ cycloalkyl group, a $C_2$-$C_4$ alkanoyl group, a benzoyl group, a $C_1$-$C_4$ alkylsulfonyl group, a phenylsulfonyl group, an aminosulfonyl group, a $C_1$-$C_4$ alkylaminosulfonyl group, a phenylsulfamoyl group, a $C_1$-$C_4$ alkylaminocarbonyl group, a phenylcarbamoyl group, a $C_1$-$C_4$ alkoxycarbonyl group, a phenoxycarbonyl group, a phenyl group, a thienyl group, a pyrazolyl group, a thiazolyl group, a pyridyl group, a benzofuryl group, a benzothienyl group or a completely saturated heterocyclic group, each of which may be substituted by a group(s) selected from the group consisting of a halogeno group, a $C_1$-$C_4$ alkoxy group, a $C_3$-$C_6$ cycloalkyl group, a $C_1$-$C_4$ alkoxycarbonyl group, a $C_1$-$C_4$ alkylthio group, a $C_1$-$C_4$ alkylsulfinyl group, a $C_1$-$C_4$ alkylsulfonyl group, a $C_1$-$C_4$ alkylamino group, a di-($C_1$-$C_4$ alkyl)amino group, a $C_2$-$C_4$ alkanoylamino group, a $C_1$-$C_4$ alkoxycarbonylamino group, an aryl group, a heteroaryl group and a completely saturated heterocyclic group,
Z is a $C_1$-$C_4$ alkyl group, a $C_3$-$C_6$ cycloalkyl group, a $C_5$-$C_6$ cycloalkenyl group, a $C_3$-$C_5$ alkenyl group, a $C_3$-$C_5$ alkynyl group, a completely saturated heterocyclic group, each of which may be substituted by a group(s) selected from the group consisting of a halogeno group, a $C_7$-$C_{12}$ aralkyl group, a $C_1$-$C_4$ alkoxy group, a $C_7$-$C_{12}$ aralkyloxy group, a $C_3$-$C_6$ cycloalkyl group, a $C_5$-$C_6$ cycloalkenyl group, an arylcarbonyl group, a $C_1$-$C_4$ alkylamino group, a di-($C_1$-$C_4$ alkyl)amino group, an arylamino group, an aryl group, a heteroaryl group, an aryloxy group and a completely saturated heterocyclic group, or, a phenyl group or a heteroaryl group, each of which may be substituted by a group (s) selected from the group consisting of a halogeno group, a cyano group, a nitro group, a formyl group, a $C_1$-$C_4$ alkyl group, a halogeno-$C_1$-$C_4$ alkyl group, a $C_7$-$C_{12}$ aralkyl group, a $C_7$-$C_{12}$ aralkyloxy group, a $C_3$-$C_6$ cycloalkyl group, a $C_1$-$C_4$ alkoxy group, a $C_1$-$C_4$ alkoxycarbonyl group, a $C_1$-$C_4$ alkylthio group, a $C_1$-$C_4$ alkylamino group, a di-($C_1$-$C_4$ alkyl)amino group, an arylamino group, a $C_2$-$C_4$ alkanoylamino group, a $C_1$-$C_4$ alkoxycarbonylamino group, an aryl group, a heteroaryl group, a aryloxy group and a completely saturated heterocyclic group.

**9.** The aniline compound or a pharmaceutically acceptable salt thereof according to Claim 1, wherein
$R^1$ is a hydrogen atom, a methyl group, a ethyl group, a propyl group, an isopropyl group or a tert-butyl group,
$R^2$ and $R^3$ each independently represent a hydrogen atom or a methyl group,
$R^4$ is a hydrogen atom, or, a $C_1$-$C_4$ alkyl group, a 2-propenyl group, a 2-propynyl group, a cyclopropyl group, a $C_2$-$C_4$ alkanoyl group, a benzoyl group, a $C_1$-$C_4$ alkylsulfonyl group, a $C_1$-$C_4$ alkylaminosulfonyl group, a $C_1$-$C_4$ alkylaminocarbonyl group, a phenylcarbamoyl group, a $C_1$-$C_4$ alkoxycarbonyl group, a phenoxycarbonyl group, a phenyl group, a thienyl group, a pyridyl group, a pyrrolidinyl group or a piperidinyl group, each of which may be substituted by a group(s) selected from the group consisting of a fluoro group, a chloro group, a methoxy group, a cyclopropyl group, a methoxycarbonyl group, a $C_1$-$C_4$ alkylsulfonyl group, a dimethylamino group, a $C_2$-$C_4$ alkanoylamino group, a methoxycarbonylamino group, a phenyl group, a thienyl group, a pyridyl group, a pyrrolidinyl group and a piperidinyl group,
Z is a $C_1$-$C_4$ alkyl group, a 2-propynyl group, a 2-butynyl group, a cyclopropyl group, a cyclobutyl group, a cyclopentenyl group, each of which may be substituted by a group(s) selected from the group consisting of a fluoro group, a chloro group, a benzyl group, a methoxy group, a benzyloxy group, a cyclopropyl group, a cyclopentenyl group, a benzoyl group, a methylamino group, a dimethylamino group, an anilino group, a phenyl group, a thienyl group, a pyridyl group, a phenoxy group, a pyrrolidinyl group and a piperidinyl group, or, a phenyl group, a thienyl group, a pyrazolyl group, a thiazolyl group or a pyridyl group, each of which may be substituted by a group(s) selected from the group consisting of a fluoro group, a chloro group, a cyano group, a nitro group, a methyl group, an ethyl group,

a trifluoromethyl group, a benzyl group, a benzyloxy group, a cyclopropyl group, a methoxy group, a methoxycarbonyl group, a methylthio group, a methylamino group, a dimethylamino group, an anilino group, an acetylamino group, a methoxycarbonylamino group, a phenyl group, a phenoxy group, an azetidinyl group, a pyrrolidinyl group and a piperidinyl group.

**10.** The aniline compound or a pharmaceutically acceptable salt thereof according to Claim 1, wherein
$R^1$ is a hydrogen atom, a methyl group, an ethyl group or an isopropyl group,
$R^2$ and $R^3$ are both hydrogen atoms,
$R^4$ is a hydrogen atom, a trifluoromethyl group, a 2,2,2-trifluoroethyl group, a benzyl group, a 1-phenylethyl group, a 2-phenylethyl group, a methyl group, an ethyl group, a propyl group, a butyl group, a cyclopropyl group, an acetyl group, a propanoyl group, a butanoyl group, a benzoyl group, a methylsulfonyl group, an ethylsulfonyl group, a propylsulfonyl group, a methylaminosulfonyl group, a methylaminocarbonyl group, a methoxycarbonyl group, a phenoxycarbonyl group, a phenyl group or a piperidinyl group, and
Z is a methyl group, an ethyl group, a trifluoromethyl group, a 2-phenylethyl group, a cyclopropyl group, a phenyl group, a 2-fluorophenyl group, a 3-fluorophenyl group, a 4-fluorophenyl group, a 2-chlorophenyl group, a 3-chlorophenyl group, a 4-chlorophenyl group, a 4-cyanophenyl group, a 4-cyclopropylphenyl group, a 4-methoxyphenyl group, a thiophen-2-yl group, a thiophen-3-yl group, a pyridin-2-yl group, a pyridin-3-yl group or a pyridin-4-yl group.

**11.** The aniline compound or a pharmaceutically acceptable salt thereof according to Claim 1, wherein
$R^1$ is a hydrogen atom, a methyl group, an ethyl group or an isopropyl group,
$R^2$ and $R^3$ are both hydrogen atoms,
$R^4$ is a hydrogen atom, a methyl group, an ethyl group, a propyl group, a butyl group, a 2,2,2-trifluoroethyl group, a benzyl group, a butanoyl group, a propylsulfonyl group or a phenyl group, and
Z is a phenyl group, a 4-fluorophenyl group, a pyridin-2-yl group or a pyridin-3-yl group.

**12.** The aniline compound or a pharmaceutically acceptable salt thereof according to Claim 1, wherein the aniline compound is
{6-[(4-methylaminobenzyl)(pyridin-3-ylsulfonyl)aminomethyl]pyridin-2-ylamino}-acetic acid,
[6-({4-[methyl(propylsulfonyl)amino]benzyl}(pyridin-3-ylsulfonyl)aminomethyl)-pyridin-2-ylamino]acetic acid,
{6-[(4-diphenylaminobenzyl)(pyridin-3-ylsulfonyl)aminomethyl]pyridin-2-ylamino}-acetic acid,
[6-({4-[diethyl(methyl)ammonio]benzyl}(pyridin-3-ylsulfonyl)aminomethyl)pyridin-2-ylamino]acetic acid,
[6-({4-[butyryl(methyl)amino]benzyl}(pyridin-3-ylsulfonyl)aminomethyl)pyridin-2-yl-amino]acetic acid,
[6-({4-[benzyl(methyl)amino]benzyl}(pyridin-3-ylsulfonyl)aminomethyl)pyridin-2-yl-amino]acetic acid,
[6-({4-[butyl(methyl)amino]benzyl}(pyridin-2-ylsulfonyl)aminomethyl)pyridin-2-yl-amino]acetic acid,
(6- {[4-(phenylamino)benzyl](pyridin-2-ylsulfonyl)aminomethyl}pyridin-2-ylamino)-acetic acid, or
[6-({4-[butyl(2,2,2-trifluoroethyl)amino]benzyl}(pyridin-2-ylsulfonyl)aminomethyl)-pyridin-2-ylamino]acetic acid.

**13.** A pharmaceutical composition comprising the aniline compound according to any one of claims 1 to 12 or a pharmaceutically acceptable salt thereof as an active ingredient.

**14.** The pharmaceutical composition according to claim 13 for preventing or treating a respiratory disease.

**Patentansprüche**

**1.** Anilinverbindung, dargestellt durch die Formel (I):

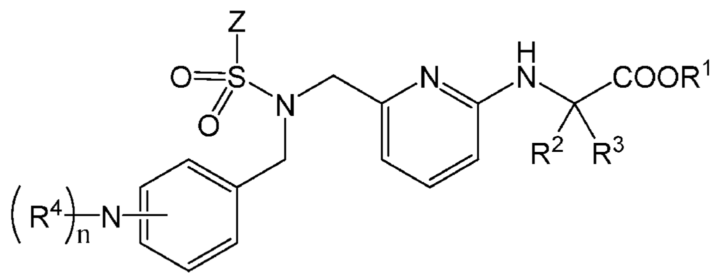

(I)

wobei

$R^1$ ein Wasserstoffatom oder eine $C_1$-$C_6$-Alkylgruppe, welche durch eine Halogengruppe substituiert sein kann, darstellt,
$R^2$ und $R^3$ jeweils unabhängig ein Wasserstoffatom oder eine $C_1$-$C_6$-Alkylgruppe darstellen,
$R^4$ ein Wasserstoffatom oder eine $C_1$-$C_6$-Alkylgruppe, eine $C_2$-$C_6$-Alkenylgruppe, eine $C_2$-$C_6$-Alkinylgruppe, eine $C_3$-$C_8$-Cycloalkylgruppe, eine $C_2$-$C_6$-Alkanoylgruppe, eine Arylcarbonylgruppe, eine Heteroarylcarbonylgruppe, eine $C_1$-$C_6$-Alkylsulfonylgruppe, eine Arylsulfonylgruppe, eine Heteroarylsulfonylgruppe, eine Aminosulfonylgruppe, eine $C_1$-$C_6$-Alkylaminosulfonylgruppe, eine Arylaminosulfonylgruppe, eine Heteroarylaminosulfonylgruppe, eine Aminocarbonylgruppe, eine $C_1$-$C_6$-Alkylaminocarbonylgruppe, eine Arylaminocarbonylgruppe, eine Heteroarylaminocarbonylgruppe, eine $C_1$-$C_6$-Alkoxycarbonylgruppe, eine Aryloxycarbonylgruppe, eine Heteroaryloxycarbonylgruppe, eine Arylgruppe, eine Heteroarylgruppe oder eine heterocyclische Gruppe darstellt, die jeweils durch die gleichen oder verschiedenen 1 bis 3 Gruppen ausgewählt aus einer Substituentengruppe $\alpha$ substituiert sein kann,
n eine ganze Zahl von 2 oder 3 ist, $R^4$s die gleichen oder verschieden voneinander sein können, wenn n 3 ist, $R^4$s $C_1$-$C_6$-Alkylgruppen sind,
Z eine $C_1$-$C_6$-Alkylgruppe, eine $C_2$-$C_6$-Alkenylgruppe, eine $C_2$-$C_6$-Alkinylgruppe, eine $C_3$-$C_8$-Cycloalkylgruppe, eine $C_5$-$C_8$-Cycloalkenylgruppe, eine Arylgruppe, eine Heteroarylgruppe oder eine heterocyclische Gruppe darstellt, die jeweils durch die gleichen oder verschiedenen 1 bis 3 Gruppen ausgewählt aus einer Substituentengruppe $\alpha$ substituiert sein kann,
ein Substituent, enthalten in der Substituentengruppe $\alpha$, eine Hydroxygruppe, eine Oxogruppe, eine Halogengruppe, eine Cyanogruppe, eine Nitrogruppe, eine Formylgruppe, eine $C_1$-$C_6$-Alkylgruppe, eine $C_7$-$C_{12}$-Aralkylgruppe, eine $C_2$-$C_6$-Alkenylgruppe, eine $C_2$-$C_6$-Alkinylgruppe, eine $C_1$-$C_6$-Alkoxygruppe, eine $C_1$-$C_6$-Alkoxy-$C_1$-$C_6$-alkylgruppe, eine $C_7$-$C_{12}$-Aralkyloxygruppe, eine $C_3$-$C_8$-Cycloalkylgruppe, eine $C_3$-$C_8$-Cycloalkyl-$C_1$-$C_6$-alkylgruppe, eine $C_5$-$C_8$-Cycloalkenylgruppe, eine $C_2$-$C_6$-Alkanoylgruppe, eine $C_3$-$C_8$-Cycloalkyl-$C_2$-$C_6$-alkanoylgruppe, eine $C_2$-$C_6$-Alkanoyl-$C_1$-$C_6$-alkylgruppe, eine Arylcarbonylgruppe, eine Heteroarylcarbonylgruppe, eine Carboxygruppe, eine $C_1$-$C_6$-Alkoxycarbonylgruppe, eine $C_1$-$C_6$-Alkylthiogruppe, eine Arylthiogruppe, eine Heteroarylthiogruppe, eine $C_1$-$C_6$-Alkylsulfinylgruppe, eine Arylsulfinylgruppe, eine Heteroarylsulfinylgruppe, eine $C_1$-$C_6$-Alkylsulfonylgruppe, eine Arylsulfonylgruppe, eine Heteroarylsulfonylgruppe, eine Aminosulfonylgruppe, eine $C_1$-$C_6$-Alkylaminosulfonylgruppe, eine Di-($C_1$-$C_6$-alkyl)aminosulfonylgruppe (zwei Alkylgruppen können in Kombination einen 4- bis 8-gliedrigen Ring bilden), eine Arylaminosulfonylgruppe, eine Heteroarylaminosulfonylgruppe, eine Aminogruppe, eine $C_1$-$C_6$-Alkylaminogruppe, eine Di-($C_1$-$C_6$-alkyl) aminogruppe (zwei Alkylgruppen können in Kombination einen 4- bis 8-gliedrigen Ring bilden), eine Tri-($C_1$-$C_6$-alkyl)ammoniumgruppe (zwei Alkylgruppen von drei können kombiniert sein, um einen 4- bis 8-gliedrigen Ring zu bilden), eine $C_2$-$C_6$-Alkenylaminogruppe, eine $C_2$-$C_6$-Alkinylaminogruppe, eine $C_3$-$C_8$-Cycloalkylaminogruppe, eine Arylaminogruppe, eine Heteroarylaminogruppe, eine $C_1$-$C_6$-Alkylsulfonylaminogruppe, eine Arylsulfonylaminogruppe, eine Heteroarylsulfonylaminogruppe, eine Aminosulfonylaminogruppe, eine $C_1$-$C_6$-Alkylaminosulfonylaminogruppe, eine Di-($C_1$-$C_6$-alkyl)aminosulfonylaminogruppe (zwei Alkylgruppen können in Kombination einen 4- bis 8-gliedrigen Ring bilden), eine Arylaminosulfonylaminogruppe, eine Heteroarylaminosulfonylaminogruppe, eine $C_2$-$C_6$-Alkanoylaminogruppe, eine Arylcarbonylaminogruppe, eine Heteroarylcarbonylaminogruppe, eine Aminocarbonylaminogruppe, eine $C_1$-$C_6$-Alkylaminocarbonylaminogruppe, eine Di-($C_1$-$C_6$-alkyl)aminocarbonylaminogruppe (zwei Alkylgruppen können in Kombination einen 4- bis 8-gliedrigen Ring bilden), eine Arylaminocarbonylaminogruppe, eine Heteroarylaminocarbonylaminogruppe, eine $C_1$-$C_6$-Alkoxycarbonylaminogruppe, eine Aryloxycarbonylaminogruppe, eine Heteroaryloxycarbonylaminogruppe, eine Carbamoylgruppe, eine $C_1$-$C_6$-Alkylcarbamoylgruppe, eine Di-($C_1$-$C_6$-alkyl)carbamoylgruppe (zwei Alkylgruppen können in Kombination einen 4- bis 8-gliedrigen Ring bilden), eine Arylcarbamoylgruppe, eine Heteroarylcarbamoylgruppe, eine Arylgruppe, eine Heteroarylgruppe, eine Aryloxygruppe, eine Heteroaryloxygruppe und eine heterocyclische Gruppe ist,
die Substituenten in der Substituentengruppe $\alpha$, wenn sie eine Alkylgruppe, eine Alkenylgruppe, eine Alkinylgruppe, eine Cycloalkylgruppe, eine Cycloalkenylgruppe, eine Arylgruppe, eine Heteroarylgruppe oder einen heterocyclischen Gruppenteil enthalten, weiter durch die gleichen oder verschiedenen 1 bis 3 Substituenten ausgewählt aus der Substituentengruppe $\alpha$ substituiert sein können,

oder ein pharmazeutisch annehmbares Salz davon.

2. Anilinverbindung oder ein pharmazeutisch annehmbares Salz davon nach Anspruch 1, wobei $R^2$ und $R^3$ jeweils unabhängig ein Wasserstoffatom oder eine $C_1$-$C_4$-Alkylgruppe darstellen.

3. Anilinverbindung oder ein pharmazeutisch annehmbares Salz davon nach Anspruch 1 oder 2, wobei $R^4$ ein Wasserstoffatom oder eine $C_1$-$C_6$-Alkylgruppe, eine $C_3$-$C_5$-Alkenylgruppe, eine $C_3$-$C_5$-Alkinylgruppe, eine $C_3$-$C_6$-Cycloalkylgruppe, eine $C_2$-$C_4$-Alkanoylgruppe, eine Benzoylgruppe, eine $C_1$-$C_4$-Alkylsulfonylgruppe, eine Phenylsulfonylgruppe, eine Aminosulfonylgruppe, eine $C_1$-$C_4$-Alkylaminosulfonylgruppe, eine Phenylsulfamylgruppe, eine $C_1$-$C_4$-Alkylaminocarbonylgruppe, eine Phenylcarbamoylgruppe, eine $C_1$-$C_4$-Alkoxycarbonylgruppe, eine Phenoxycarbonylgruppe, eine Phenylgruppe, eine Thienylgruppe, eine Pyrazolylgruppe, eine Thiazolylgruppe, eine Pyridylgruppe, eine Benzofurylgruppe, eine Benzothienylgruppe oder eine vollständig gesättigte heterocyclische Gruppe ist, die jeweils durch eine Gruppe(n) ausgewählt aus der Gruppe bestehend aus einer Halogengruppe, einer $C_1$-$C_4$-Alkoxygruppe, einer $C_3$-$C_6$-Cycloalkylgruppe, einer $C_1$-$C_4$-Alkoxycarbonylgruppe, einer $C_1$-$C_4$-Alkylthiogruppe, einer $C_1$-$C_4$-Alkylsulfinylgruppe, einer $C_1$-$C_4$-Alkylsulfonylgruppe, einer $C_1$-$C_4$-Alkylaminogruppe, einer Di-($C_1$-$C_4$-alkyl)aminogruppe, einer $C_2$-$C_4$-Alkanoylaminogruppe, einer $C_1$-$C_4$-Alkoxycarbonylaminogruppe, einer Arylgruppe, einer Heteroarylgruppe und einer vollständig gesättigten heterocyclischen Gruppe substituiert sein kann.

4. Anilinverbindung oder ein pharmazeutisch annehmbares Salz davon nach einem der Ansprüche 1 bis 3, wobei Z eine $C_1$-$C_4$-Alkylgruppe, eine $C_3$-$C_6$-Cycloalkylgruppe, eine $C_5$-$C_6$-Cycloalkenylgruppe, eine $C_3$-$C_5$-Alkenylgruppe, eine $C_3$-$C_5$-Alkinylgruppe, eine vollständig gesättigte heterocyclische Gruppe, die jeweils durch eine Gruppe(n) ausgewählt aus der Gruppe bestehend aus einer Halogengruppe, einer $C_7$-$C_{12}$-Aralkylgruppe, einer $C_1$-$C_4$-Alkoxygruppe, einer $C_7$-$C_{12}$-Aralkyloxygruppe, einer $C_3$-$C_6$-Cycloalkylgruppe, einer $C_5$-$C_6$-Cycloalkenylgruppe, einer Arylcarbonylgruppe, einer $C_1$-$C_4$-Alkylaminogruppe, einer Di-($C_1$-$C_4$-alkyl)aminogruppe, einer Arylaminogruppe, einer Arylgruppe, einer Heteroarylgruppe, einer Aryloxygruppe und einer vollständig gesättigten heterocyclischen Gruppe substituiert sein kann, oder eine Phenylgruppe oder eine Heteroarylgruppe, die jeweils durch eine Gruppe(n) ausgewählt aus der Gruppe bestehend aus einer Halogengruppe, einer Cyanogruppe, einer Nitrogruppe, einer Formylgruppe, einer $C_1$-$C_4$-Alkylgruppe, Halogen-$C_1$-$C_4$-alkylgruppe, einer $C_7$-$C_{12}$-Aralkylgruppe, einer $C_7$-$C_{12}$-Aralkyloxygruppe, einer $C_3$-$C_6$-Cycloalkylgruppe, einer $C_1$-$C_4$-Alkoxygruppe, einer $C_1$-$C_4$-Alkoxycarbonylgruppe, einer $C_1$-$C_4$-Alkylthiogruppe, einer $C_1$-$C_4$-Alkylaminogruppe, einer Di($C_1$-$C_4$-alkyl)aminogruppe, einer Arylaminogruppe, einer $C_2$-$C_4$-Alkanoylaminogruppe, einer $C_1$-$C_4$-Alkoxycarbonylgruppe, einer Arylgruppe, einer Heteroarylgruppe, einer Aryloxygruppe und einer vollständig gesättigten heterocyclischen Gruppe substituiert sein kann, ist.

5. Anilinverbindung oder ein pharmazeutisch annehmbares Salz davon nach Anspruch 3, wobei $R^4$ ein Wasserstoffatom oder eine $C_1$-$C_4$-Alkylgruppe, eine 2-Propenylgruppe, eine 2-Propinylgruppe, eine Cyclopropylgruppe, eine $C_2$-$C_4$-Alkanoylgruppe, eine Benzoylgruppe, eine $C_1$-$C_4$-Alkylsulfonylgruppe, eine $C_1$-$C_4$-Alkylaminosulfonylgruppe, eine $C_1$-$C_4$-Alkylaminocarbonylgruppe, eine Phenylcarbamoylgruppe, eine $C_1$-$C_4$-Alkoxycarbonylgruppe, eine Phenoxycarbonylgruppe, eine Phenylgruppe, eine Thienylgruppe, eine Pyridylgruppe, eine Pyrrolidinylgruppe oder eine Piperidinylgruppe ist, die jeweils durch eine Gruppe(n) ausgewählt aus der Gruppe bestehend aus einer Fluorgruppe, einer Chlorgruppe, einer Methoxygruppe, einer Cyclopropylgruppe, einer Methoxycarbonylgruppe, einer $C_1$-$C_4$-Alkylsulfonylgruppe, einer Dimethylaminogruppe, einer $C_2$-$C_4$-Alkanoylaminogruppe, einer Methoxycarbonylaminogruppe, einer Phenylgruppe, einer Thienylgruppe, einer Pyridylgruppe, einer Pyrrolidinylgruppe und einer Piperidinylgruppe substituiert sein kann.

6. Anilinverbindung oder ein pharmazeutisch annehmbares Salz davon nach Anspruch 5, wobei $R^4$ ein Wasserstoffatom, eine Trifluormethylgruppe, eine 2,2,2-Trifluorethylgruppe, eine Benzylgruppe, eine 1-Phenylethylgruppe, eine 2-Phenylethylgruppe, eine Methylgruppe, eine Ethylgruppe, eine Propylgruppe, eine Butylgruppe, eine Cyclopropylgruppe, eine Acetylgruppe, eine Propanoylgruppe, eine Butanoylgruppe, eine Benzoylgruppe, eine Methylsulfonylgruppe, eine Ethylsulfonylgruppe, eine Propylsulfonylgruppe, eine Methylaminosulfonylgruppe, eine Methylaminocarbonylgruppe, eine Methoxycarbonylgruppe, eine Phenoxycarbonylgruppe, eine Phenylgruppe oder eine Piperidinylgruppe ist.

7. Anilinverbindung oder ein pharmazeutisch annehmbares Salz davon nach Anspruch 4, wobei Z eine $C_1$-$C_4$-Alkylgruppe, einen 2-Propinylgruppe, eine 2-Butinylgruppe, eine Cyclopropylgruppe, eine Cyclobutylgruppe, eine Cyclopentenylgruppe, die jeweils durch eine Gruppe(n) ausgewählt aus der Gruppe bestehend aus einer Fluorgruppe, einer Chlorgruppe, einer Benzylgruppe, einer Methoxygruppe, einer Benzyloxygruppe, einer Cyclopropylgruppe, einer Cyclopentenylgruppe, einer Benzoylgruppe, einer Methylaminogruppe, einer Dimethylaminogruppe, einer Anilingruppe, einer Phenylgruppe, einer Thienylgruppe, einer Pyridylgruppe, einer Phenoxygruppe, einer Pyrrolidinylgruppe und einer Piperidinylgruppe substituiert sein kann, oder eine Phenylgruppe, eine Thienylgruppe, eine Pyrazolylgruppe, eine Thiazolylgruppe oder eine Pyridiylgruppe, die jeweils durch eine Gruppe(n) ausgewählt aus der Gruppe bestehend aus einer Fluorgruppe, einer Chlorgruppe, einer Cyanogruppe, einer Nitrogruppe, einer Methylgruppe, einer Ethylgruppe, einer Trifluormethylgruppe, einer Benzylgruppe, einer Benzyloxygruppe, einer Cyclopropylgruppe, einer Methoxygruppe, einer Methoxycarbonylgruppe, einer Methylthiogruppe, einer Methylaminogruppe, einer Dimethylaminogruppe, einer Anilingruppe, einer Acetylaminogruppe, einer Methoxycarbonalygruppe, einer Phenylgruppe, einer Phenoxygruppe, einer Azetidinylgruppe, einer Pyrrolidinylgruppe und einer Piperidinylgruppe substituiert sein kann, ist.

**8.** Anilinverbindung oder ein pharmazeutisch annehmbares Salz davon nach Anspruch 1, wobei
$R^1$ ein Wasserstoffatom, eine Methylgruppe, eine Ethylgruppe, eine Propylgruppe, eine Isopropylgruppe oder eine tert-Butylgruppe ist,
$R^2$ und $R^3$ jeweils unabhängig ein Wasserstoffatom oder eine Methylgruppe darstellen,
$R^4$ ein Wasserstoffatom oder eine $C_1$-$C_6$-Alkylgruppe, eine $C_3$-$C_5$-Alkenylgruppe, eine $C_3$-$C_5$-Alkinylgruppe, eine $C_3$-$C_6$-Cycloalkylgruppe, eine $C_2$-$C_4$-Alkanoylgruppe, eine Benzoylgruppe, eine $C_1$-$C_4$-Alkylsulfonylgruppe, eine Phenylsulfonylgruppe, eine Aminosulfonylgruppe, eine $C_1$-$C_4$-Alkylaminosulfonylgruppe, eine Phenylsulfamylgruppe, eine $C_1$-$C_4$-Alkylaminocarbonylgruppe, eine Phenylcarbamoylgruppe, eine $C_1$-$C_4$-Alkoxycarbonylgruppe, eine Phenoxycarbonylgruppe, eine Phenylgruppe, eine Thienylgruppe, eine Pyrazolylgruppe, eine Thiazolylgruppe, eine Pyridylgruppe, eine Benzofurylgruppe, eine Benzothienylgruppe oder eine vollständig gesättigte heterocyclische Gruppe ist, die jeweils durch eine Gruppe(n) ausgewählt aus der Gruppe bestehend aus einer Halogengruppe, einer $C_1$-$C_4$-Alkoxygruppe, einer $C_3$-$C_6$-Cycloalkylgruppe, einer $C_1$-$C_4$-Alkoxycarbonylgruppe, einer $C_1$-$C_4$-Alkylthiogruppe, einer $C_1$-$C_4$-Alkylsulfinylgruppe, einer $C_1$-$C_4$-Alkylsulfonylgruppe, einer $C_1$-$C_4$-Alkylaminogruppe, einer Di-($C_1$-$C_4$-alkyl)aminogruppe, einer $C_2$-$C_4$-Alkanoylgruppe, einer $C_1$-$C_4$-Alkoxycarbonylaminogruppe, einer Arylgruppe, einer Heteroarylgruppe und einer vollständig gesättigten heterocyclischen Gruppe substituiert sein kann,
Z eine $C_1$-$C_4$-Alkylgruppe, eine $C_3$-$C_6$-Cycloalkylgruppe, einer $C_5$-$C_6$-Cycloalkenylgruppe, einer $C_3$-$C_5$-Alkenylgruppe, eine $C_3$-$C_5$-Alkinylgruppe, eine vollständig gesättigte heterocyclische Gruppe, die jeweils durch eine Gruppe(n) ausgewählt aus der Gruppe bestehend aus einer Halogengruppe, einer $C_7$-$C_{12}$-Aralkylgruppe, einer $C_1$-$C_4$-Alkoxygruppe, einer $C_7$-$C_{12}$-Aralkyloxygruppe, einer $C_3$-$C_6$-Cycloalkylgruppe, einer $C_5$-$C_6$-Cycloalkenylgruppe, einer Arylcarbonylgruppe, einer $C_1$-$C_4$-Alkylaminogruppe, einer Di-($C_1$-$C_4$-alkyl)aminogruppe, einer Arylaminogruppe, einer Arylgruppe, einer Heteroarylgruppe, einer Aryloxygruppe und einer vollständig gesättigten heterocyclischen Gruppe substituiert sein kann, oder eine Phenylgruppe oder eine Heteroarylgruppe, die jeweils durch eine Gruppe(n) ausgewählt aus der Gruppe bestehend aus einer Halogengruppe, einer Cyanogruppe, einer Nitrogruppe, einer Formylgruppe, einer $C_1$-$C_4$-Alkylgruppe, einer Halogen-$C_1$-$C_4$-alkylgruppe, einer $C_7$-$C_{12}$-Aralkylgruppe, einer $C_7$-$C_{12}$-Aralkyloxygruppe, einer $C_3$-$C_6$-Cycloalkylgruppe, einer $C_1$-$C_4$-Alkoxygruppe, einer $C_1$-$C_4$-Alkoxycarbonylgruppe, einer $C_1$-$C_4$-Alkylthiogruppe, einer $C_1$-$C_4$-Alkylaminogruppe, einer Di-($C_1$-$C_4$-alkyl)aminogruppe, einer Arylaminogruppe, einer $C_2$-$C_4$-Alkanoylaminogruppe, einer $C_1$-$C_4$-Alkoxycarbonylaminogruppe, einer Arylgruppe, einer Heteroarylgruppe, einer Aryloxygruppe und einer vollständig gesättigten heterocyclischen Gruppe substituiert sein kann, ist.

**9.** Anilinverbindung oder ein pharmazeutisch annehmbares Salz davon nach Anspruch 1, wobei
$R^1$ ein Wasserstoffatom, eine Methylgruppe, eine Ethylgruppe, eine Propylgruppe, eine Isopropylgruppe oder eine tert-Butylgruppe ist,
$R^2$ und $R^3$ jeweils unabhängig ein Wasserstoffatom oder eine Methylgruppe darstellen,
$R^4$ ein Wasserstoffatom oder eine $C_1$-$C_4$-Alkylgruppe, eine 2-Propenylgruppe, eine 2-Propinylgruppe, eine Cyclopropylgruppe, eine $C_2$-$C_4$-Alkanoylgruppe, eine Benzoylgruppe, eine $C_1$-$C_4$-Alkylsulfonylgruppe, eine $C_1$-$C_4$-Alkylaminosulfonylgruppe, eine $C_1$-$C_4$-Alkylaminocarbonylgruppe, eine Phenylcarbamoylgruppe, eine $C_1$-$C_4$-Alkoxycarbonylgruppe, eine Phenoxycarbonylgruppe, eine Phenylgruppe, eine Thienylgruppe, eine Pyridylgruppe, eine Pyrrolidinylgruppe oder Piperidinylgruppe ist, die jeweils durch eine Gruppe(n) ausgewählt aus der Gruppe bestehend aus einer Fluorgruppe, einer Chlorgruppe, einer Methoxygruppe, einer Cyclopropylgruppe, einer Methoxycarbonylgruppe, einer $C_1$-$C_4$-Alkylsulfonylgruppe, einer Dimethylaminogruppe, einer $C_2$-$C_4$-Alkanoylaminogruppe, Methoxycarbonylaminogruppe, einer Phenylgruppe, einer Thienylgruppe, einer Pyridylgruppe, einer Pyrrolidinylgruppe oder einer Piperidinylgruppe substituiert sein kann,
Z eine $C_1$-$C_4$-Alkylgruppe, eine 2-Propinylgruppe, eine 2-Butinylgruppe, eine Cyclopropylgruppe, eine Cyclobutylgruppe, eine Cyclopentenylgruppe, die jeweils durch eine Gruppe(n) ausgewählt aus der Gruppe bestehend aus einer Fluorgruppe, einer Chlorgruppe, einer Benzylgruppe, einer Methoxygruppe, einer Benzyloxygruppe, einer Cyclopropylgruppe, einer Cyclopentenylgruppe, einer Benzoylgruppe, einer Methylaminogruppe, einer Dimethylaminogruppe, einer Anilingruppe, einer Phenylgruppe, einer Thienylgruppe, einer Pyridylgruppe, einer Phenoxygruppe, einer Pyrrolidinylgruppe und einer Piperidinylgruppe substituiert sein kann, oder eine Phenylgruppe, eine Thienylgruppe, eine Pyrazolylgruppe, eine Thiazolylgruppe oder eine Pyridiylgruppe, die jeweils durch eine Gruppe (n) ausgewählt aus der Gruppe bestehend aus einer Fluorgruppe, einer Chlorgruppe, einer Cyanogruppe, einer Nitrogruppe, einer Methylgruppe, einer Ethylgruppe, einer Trifluormethylgruppe, einer Benzylgruppe, einer Benzyloxygruppe, einer Cyclopropylgruppe, einer Methoxygruppe, einer Methoxycarbonylgruppe, einer Methylthiogruppe, einer Methylaminogruppe, einer Dimethylaminogruppe, einer Anilingruppe, einer Acetylaminogruppe, einer Methoxycarbonylaminogruppe, einer Phenylgruppe, einer Phenoxygruppe, einer Azetidinylgruppe, einer Pyrrolidinylgruppe und einer Piperdinylgruppe substituiert sein kann, ist.

**10.** Anilinverbindung oder ein pharmazeutisch annehmbares Salz davon nach Anspruch 1, wobei
$R^1$ ein Wasserstoffatom, eine Methylgruppe, eine Ethylgruppe oder eine Isopropylgruppe ist,
$R^2$ und $R^3$ beide Wasserstoffatom sind,
$R^4$ ein Wasserstoffatom, eine Trifluormethylgruppe, 2,2,2-Trifluorethylgruppe, eine Benzylgruppe, einer 1-Phenylethylgruppe, eine 2-Phenylethylgruppe, eine Methylgruppe, eine Ethylgruppe, eine Propylgruppe, einer Butylgruppe, einer Cyclopropylgruppe, eine Acetylgruppe, eine Propanoylgruppe, eine Butanoylgruppe, eine Benzoylgruppe, eine Methylsulfonylgruppe, eine Ethylsulfonylgruppe, einer Propylsulfonylgruppe, eine Methylaminosulfonylgruppe, eine Methylaminorcarbonylgruppe, eine Methoxycarbonylgruppe, eine Phenoxycarbonylgruppe, eine Phenylgruppe oder eine Piperidinylgruppe ist, und
Z eine Methylgruppe, eine Ethylgruppe, eine Trifluormethylgruppe, eine 2-Phenylethylgruppe, eine Cyclopropylgruppe, eine Phenylgruppe, eine 2-Fluorphenylgruppe, eine 3-Fluorphenylgruppe, eine 4-Fluorphenylgruppe, eine 2-Chlorphenylgruppe, eine 3-Chlorphenylgruppe, eine 4-Chlorphenylgruppe, eine 4-Cyanophenylgruppe, eine 4-Cyclopropylphenylgruppe, eine 4-Methoxyphenylgruppe, eine Thiophen-2-ylgruppe, eine Thiophen-3-ylgruppe, eine Pyridin-2-ylgruppe, eine Pyridin-3-ylgruppe oder eine Pyridin-4-ylgruppe ist.

**11.** Anilingverbindung oder ein pharmazeutisch annehmbares Salz davon nach Anspruch 1, wobei
$R^1$ ein Wasserstoffatom, eine Methylgruppe, eine Ethylgruppe oder eine Isopropylgruppe ist,
$R^2$ und $R^3$ beide Wasserstoffatome sind,
$R^4$ ein Wasserstoffatom, eine Methylgruppe, eine Ethylgruppe, eine Propylgruppe, eine Butylgruppe, eine 2,2,2-Trifluorethylgruppe, eine Benzylgruppe, eine Butanoylgruppe, eine Propylsulfonylgruppe oder eine Phenylgruppe ist, und
Z eine Phenylgruppe, eine 4-Fluorphenylgruppe, eine Pyridin-2-ylgruppe oder eine Pyridin-3-ylgruppe ist.

**12.** Anilinverbindung oder ein pharmazeutisch annehmbares Salz davon nach Anspruch 1, wobei die Anilinverbindung
{6-[(4-Methylaminobenzyl)(pyridin-3-ylsulfonyl)aminomethyl]pyridin-2-ylamino}-essigsäure,
[6-({4-[Methyl(propylsulfonyl)amino]benzyl}(pyridin-3-ylsulfonyl)aminomethyl)-pyridin-2-ylamino]essigsäure,
{6-[(4-Diphenylaminobenzyl)(pyridin-3-ylsulfonyl)aminomethyl]pyridin-2-ylamino}-essigsäure,
[6-({4-[Diethyl(methyl)ammonio]benzyl} (pyridin-3-ylsulfonyl)aminomethyl)pyridin-2-ylamino]essigsäure,
[6-({4-[Butyryl(methyl)amino]benzyl}(pyridin-3-ylsulfonyl)aminomethyl)pyridin-2-ylamino]essigsäure,
[6-({4-[Benzyl(methyl)amino]benzyl}(pyridin-3-ylsulfonyl)aminomethyl)pyridin-2-ylamino]essigsäure,
[6-({4-[Butyl(methyl)amino]benzyl}(pyridin-2-ylsulfonyl)aminomethyl)pyridin-2-yl-amino]essigsäure,
(6-{[4-(Phenylamino)benzyl](pyridin-2-ylsulfonyl)aminomethyl}pyridin-2-ylamino)-essigsäure, oder
[6-({4-[Butyl(2,2,2-trifluorethyl)amino]benzyl}(pyridin-2-ylsulfonyl)aminomethyl)-pyridin-2-ylamino]essigsäure ist.

**13.** Pharmazeutische Zusammensetzung, umfassend die Anilinverbindung nach einem der Ansprüche 1 bis 12 oder ein pharmazeutisch annehmbares Salz davon als einen aktiven Bestandteil.

**14.** Pharmazeutische Zusammensetzung nach Anspruch 13 zur Prävention oder Behandlung einer Atemwegserkrankung.

## Revendications

**1.** Composé aniline représenté par la formule (I) :

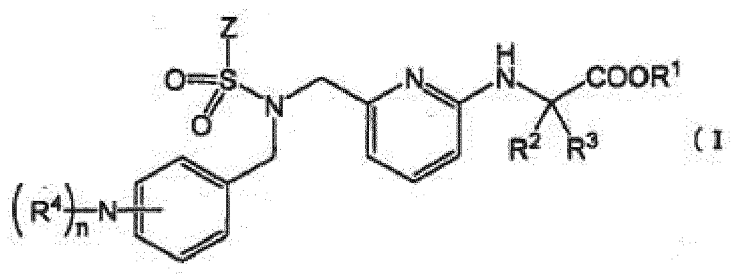

(I)

dans laquelle

$R^1$ représente un atome d'hydrogène ou un groupe alkyle en $C_1$ à $C_6$ qui peut être substitué par un groupe halogéno,
$R^2$ et $R^3$ représentent chacun indépendamment un atome d'hydrogène ou un groupe alkyle en $C_1$ à $C_6$,
$R^4$ représente un atome d'hydrogène ou un groupe alkyle en $C_1$ à $C_6$, un groupe alkényle en $C_2$ à $C_6$, un groupe alkynyle en $C_2$ à $C_6$, un groupe cycloalkyle en $C_3$ à $C_8$, un groupe alcanoyle en $C_2$ à $C_6$, un groupe arylecarbonyle, un groupe hétéroarylcarbonyle, un groupe alkylsulfonyle en $C_1$ à $C_6$, un groupe arylsulfonyle, un groupe hétéroarylsulfonyle, un groupe alkyle en $C_1$ à $C_6$-aminosulfonyle, un groupe arylaminosulfonyle, un groupe hétéroarylaminosulfonyle, un groupe aminocarbonyle, un groupe alkylaminocarbonyle en $C_1$ à $C_6$, un groupe arylaminocarbonyle, un groupe hétéroarylaminocarbonyle, un groupe alcoxycarbonyle en $C_1$ à $C_6$, un groupe aryloxycarbonyle, un groupe hétéroaryloxycarbonyle, un groupe aryle, un groupe hétéroaryle ou un groupe hétérocyclique, chacun pouvant être substitué par 1 à 3 groupes identiques ou différents sélectionnés parmi un groupe substituant $\alpha$,
n représente un entier dont la valeur est de l'ordre de 2 ou 3, $R^4$s peuvent être identiques ou différents l'un de l'autre,
quand n représente 3, $R^4$s sont des groupes alkyle en $C_1$ à $C_6$,
Z représente un groupe alkyle en $C_1$ à $C_6$, un groupe alkényle en $C_2$ à $C_6$, un groupe alkynyle en $C_2$ à $C_6$, un groupe cycloalkyle en $C_3$ à $C_8$, un groupe cycloalkényle en $C_5$ à $C_8$, un groupe aryle, un groupe hétéroaryle ou un groupe hétérocyclique, chacun pouvant être substitués par 1 à 3 groupes identiques ou différents sélectionnés parmi un groupe substituant $\alpha$,
un substituant contenu dans le groupe substituant $\alpha$ est un groupe hydroxy, un groupe oxo, un groupe halogéno, un groupe cyano, un groupe nitro, un groupe formyle, un groupe alkyle en $C_1$ à $C_6$, un groupe aralkyle en $C_7$ à $C_{12}$, un groupe alkényle en $C_2$ à $C_6$, un groupe alkynyle en $C_2$ à $C_6$, un groupe alcoxy en $C_1$ à $C_6$, un groupe alcoxy en $C_1$ à $C_6$-alkyle en $C_1$ à $C_6$, un groupe aralkyloxy en $C_7$ à $C_{12}$, un groupe cycloalkyle en $C_3$ à $C_8$, un groupe cycloalkyle en $C_3$ à $C_8$-alkyle en $C_1$ à $C_6$, un groupe cycloalkényle en $C_5$ à $C_8$, un groupe alcanoyle en $C_2$ à $C_6$, un groupe cycloalkyle en $C_3$ à $C_8$- alcanoyle en $C_2$ à $C_6$, un groupe alcanoyle en $C_2$ à $C_6$-alkyle en $C_1$ à $C_6$, un groupe arylcarbonyle, un groupe hétéroarylcarbonyle, un groupe carboxy, un groupe alcoxycarbonyle en $C_1$ à $C_6$, un groupe alkylthio en $C_1$ à $C_6$, un groupe arylthio, un groupe hétéroarylthio, un groupe alkylsulfinyle en $C_1$ à $C_6$, un groupe arylsulfinyle, un groupe hétéroarylsulfinyle, un groupe alkylsulfonyle en $C_1$ à $C_6$, un groupe arylsulfonyle, un groupe hétéroarylsulfonyle, un groupe aminosulfonyle, un groupe alkylaminosulfonyle en $C_1$ à $C_6$, un groupe di(alkyle en $C_1$ à $C_6$)aminosulfonyle (deux groupes alkyle peuvent former un cycle de 4 à 8 chaînons conjointement), un groupe arylaminosulfonyle, un groupe hétéroarylaminosulfonyle, un groupe amino, un groupe alkylamino en $C_1$ à $C_6$, un groupe di(alkyle en $C_1$ à $C_6$)amino (deux groupes alkyle peuvent former un cycle de 4 à 8 chaînons conjointement), un groupe tri-(alkyle en $C_1$ à $C_6$)ammonium (deux groupes alkyle parmi trois peuvent être associés pour former un cycle de 4 à 8 chaînons), un groupe alkénylamino en $C_2$ à $C_6$, un groupe alkynylamino en $C_2$ à $C_6$, un groupe cyclo alkylamino en $C_3$ à $C_8$, un groupe arylamino, un groupe hétéroarylamino, un groupe hétéroarylamino, un groupe alkylsulfonylamino en $C_1$ à $C_6$, un groupe arylsulfonylamino, un groupe hétéroarylsulfonylamino en $C_1$ à $C_6$, un groupe aminosulfonylamino, un groupe alkylaminosulfonylamino, un groupe di-(alkyle en $C_1$ à $C_6$)aminosulfonylamino (deux groupes alkyle peuvent former un cycle de 4 à 8 chaînons conjointement), un groupe arylaminosulfonylamino, un groupe hétéroarylaminosulfonylamino, un groupe alcanoylamino en $C_2$ à $C_6$, un groupe arylcarbonyle, un groupe hétéroarylcarbonylamino, un groupe aminocarbonylamino, un groupe alkylaminocarbonylamino en $C_1$ à $C_6$, un groupe dialkylaminocarbonylamino en $C_1$ à $C_6$ (deux groupes alkyle peuvent former un cycle de 4 à 8 chaînons conjointement), un groupe arylaminocarbonylamino, un groupe hétéroarytaminocarbonylamino, un groupe alcoxycarbonylamino en $C_1$ à $C_6$, un groupe aryloxycarbonylamino, un groupe hétéroaryloxycarbonylamino, un groupe carbamoyle, un groupe alkylcarbamoyle en $C_1$ à $C_6$, un groupe di-(alkyle en $C_1$ à $C_6$)carbamoyle (deux groupes alkyle peuvent former un cycle de 4 à 8 chaînons conjointement), un groupe arylcarbamoyle, un groupe hétéroarylcarbamoyle, un groupe aryle, un groupe hétéroaryle, un groupe aryloxy, un groupe hétéroaryloxy et un groupe hétérocyclique,
dans les substituants du groupe de substituants $\alpha$, lorsqu'il contient un groupe alkyle, un groupe alkényle, un groupe alkynyle, un groupe cycloalkyle, un groupe cycloalkényle, un groupe aryle, un groupe hétéroaryle ou une partie de groupe hétérocyclique, il peut être substitué plus avant par 1 à 3 substituants identiques ou différents sélectionnés parmi le groupe substituant $\alpha$ ou un sel pharmaceutiquement acceptable de celui-ci.

**2.** Composé aniline ou un sel pharmaceutiquement acceptable de celui-ci selon la Revendication 1, dans lequel $R^2$ et $R^3$ représentent chacun indépendamment un atome d'hydrogène ou un groupe alkyle en $C_1$ à $C_4$.

**3.** Composé anile ou un sel pharmaceutiquement acceptable de celui-ci conformément à la Revendication 1 ou 2, dans lequel $R^4$ représente un atome d'hydrogène, ou un groupe alkyle en $C_1$ à $C_6$, un groupe alkényle en $C_3$ à $C_5$, un groupe alkynyle en $C_3$ à $C_5$, un groupe cycloalkyle en $C_3$ à $C_6$, un groupe alcanoyle en $C_2$ à $C_4$, un groupe benzoyle, un groupe alkylsulfonyle en $C_1$ à $C_4$, un groupe phénylsulfonyle, un groupe aminosulfonyle, un groupe alkylaminosulfonyle en $C_1$ à $C_4$, un groupe phénylsulfamoyle, un groupe alkylaminocarbonyle en $C_1$ à $C_4$, un groupe phénylcarbamoyle, un groupe alcoxycarbonyle en $C_1$ à $C_4$, un groupe phénoxycarbonyle, un groupe phényle, un groupe thiényle, un groupe pyrazolyle, un groupe thiazolyle, un groupe pyridyle, un groupe benzofuryle, un groupe benzothiényle ou un groupe hétérocyclique complètement saturé, chacun desquels peut être substitué par un/des groupe(s) sélectionné(s) parmi le groupe constitué d'un groupe halogéno, d'un groupe alcoxy en $C_1$ à $C_4$, d'un groupe cycloalkyle en $C_3$ à $C_6$, d'un groupe alcoxycarbonyle en $C_1$ à $C_4$, d'un groupe alkylthio en $C_1$ à $C_4$, d'un groupe alkylsulfinyle en $C_1$ à $C_4$, d'un groupe alkylsulfonyle en $C_1$ à $C_4$, d'un groupe alkylamino en $C_1$ à $C_4$, d'un groupe di-(alkyle en $C_1$ à $C_4$)amino, d'un groupe alkanoylamino en $C_2$ à $C_4$, d'un groupe alcoxycarbonylamino en $C_1$ à $C_4$, d'un groupe aryle, d'un groupe hétéroaryle et d'un groupe hétérocyclique complètement saturé.

**4.** Composé aniline ou un sel pharmaceutiquement acceptable de celui-ci selon l'une quelconque des Revendications 1 à 3, dans lequel Z représente un groupe alkyle en $C_1$ à $C_4$, un groupe cycloalkyle en $C_3$ à $C_6$, un groupe cycloalkényle en $C_5$ à $C_6$, un groupe alkényle en $C_3$ à $C_5$, un groupe alkynyle en $C_3$ à $C_5$, un groupe hétérocyclique complètement saturé, chacun desquels pouvant être substitué par un/des groupe(s) sélectionné(s) parmi le groupe constitué d'un groupe halogéno, d'un groupe aralkyle en $C_7$ à $C_{12}$, d'un groupe alcoxy en $C_1$ à $C_4$, d'un groupe aralkyloxy en $C_7$ à $C_{12}$, d'un groupe cycloalkyle en $C_3$ à $C_6$, d'un groupe cycloalkényle en $C_5$ à $C_6$, d'un groupe arylcarbonyle, d'un groupe alkylamino en $C_1$ à $C_4$, d'un groupe di-(alkyle en $C_1$ à $C_4$)amino, d'un groupe arylamino, d'un groupe aryle, d'un groupe hétéroaryle, d'un groupe aryloxy et d'un groupe hétérocyclique complètement saturé, ou d'un groupe phényle ou d'un groupe hétéroaryle, chacun desquels pouvant être substitué par un/des groupe(s) sélectionné(s) parmi le groupe constitué d'un groupe halogéno, d'un groupe cyano, d'un groupe nitro, d'un groupe formyle, d'un groupe alkyle en $C_1$ à $C_4$, d'un groupe halogéno-alkyle en $C_1$ à $C_4$, d'un groupe aralkyle en $C_7$ à $C_{12}$, d'un groupe aralkyloxy en $C_7$ à $C_{12}$, d'un groupe cycloalkyle en $C_3$ à $C_6$, d'un groupe alcoxy en $C_1$ à $C_4$, d'un groupe alcoxycarbonyle en $C_1$ à $C_4$, d'un groupe alkylthio en $C_1$ à $C_4$, d'un groupe alkylamino en $C_1$ à $C_4$, d'un groupe di-(alkyle en $C_1$ à $C_4$)amino, d'un groupe arylamino, d'un groupe alkanoylamino en $C_2$ à $C_4$, d'un groupe alcoxycarbonylamino en $C_1$ à $C_4$, d'un groupe aryle, d'un groupe hétéroaryle, d'un groupe aryloxy et d'un groupe hétérocyclique complètement saturé.

**5.** Composé aniline ou un sel pharmaceutiquement acceptable de celui-ci selon la revendication 3, dans lequel $R^4$ représente un atome d'hydrogène, ou un groupe alkyle en $C_1$ à $C_4$, un groupe 2-propényle, un groupe 2-propynyle, un groupe cyclopropyle, un groupe alcanoyle en $C_2$ à $C_4$, un groupe benzoyle, un groupe alkylsulfonyle en $C_1$ à $C_4$, un groupe alkylaminosulfonyle en $C_1$ à $C_4$, un groupe alkylaminocarbonyle en $C_1$ à $C_4$, un groupe phénylcarbamoyle, un groupe alcoxycarbonyle $C_1$ à $C_4$, un groupe phénoxycarbonyle, un groupe phényle, un groupe thiényle, un groupe pyridyle, un groupe pyrrolidinyle ou un groupe pipéridinyle, chacun desquels peut être substitué par un/des groupe (s) sélectionné(s) parmi le groupe constitué d'un groupe fluoro, d'un groupe chloro, d'un groupe méthoxy, d'un groupe cyclopropyle, d'un groupe méthoxycarbonyle, d'un groupe alkylsulfonyle en $C_1$ à $C_4$, d'un groupe diméthylamino, d'un groupe alcanoylamino en $C_2$ à $C_4$, d'un groupe méthoxycarbonylamino, d'un groupe phényle, d'un groupe thiényle, d'un groupe pyridyle, d'un groupe pyrrolidinyle et d'un groupe pipéridinyle.

**6.** Composé aniline ou un sel pharmaceutiquement acceptable de celui-ci selon la Revendication 5, dans lequel $R^4$ représente un atome d'hydrogène, un groupe trifluorométhyle, un groupe 2,2,2-trifluoroéthyle, un groupe benzyle, un groupe 1-phényléthyle, un groupe 2-phényléthyle, un groupe méthyle, un groupe éthyle, un groupe propyle, un groupe butyle, un groupe cyclopropyle, un groupe acétyle, un groupe propanoyle, un groupe butanoyle, un groupe benzoyle, un groupe méthylsulfonyle, un groupe éthylsulfonyle, un groupe propylsulfonyle, un groupe méthylaminosulfonyle, un groupe méthylaminocarbonyle, un groupe méthoxycarbonyle, un groupe phénoxycarbonyle, un groupe phényle ou un groupe pipéridinyle.

**7.** Composé aniline ou un sel pharmaceutiquement acceptable de celui-ci selon la Revendication 4, dans lequel Z représente un groupe alkyle en $C_1$ à $C_4$, un groupe 2-propynyle, un groupe 2-butynyle, un groupe cyclopropyle, un groupe cyclobutyle, un groupe cyclopentényle, chacun desquels pouvant être substitué par un/des groupe(s) sélectionné(s) parmi le groupe constitué d'un groupe fluoro, d'un groupe chloro, d'un groupe benzyle, d'un groupe méthoxy, d'un groupe benzyloxy, d'un groupe cyclopropyle, d'un groupe cyclopentényle, d'un groupe benzoyle, d'un groupe méthylamino, d'un groupe diméthylamino, d'un groupe anilino, d'un groupe phényle, d'un groupe thiényle, d'un groupe pyridyle, d'un groupe phénoxy, d'un groupe pyrrolidinyle et d'un groupe pipéridinyle, ou d'un groupe phényle, d'un groupe thiényle, d'un groupe pyrazolyle, d'un groupe thiazolyle ou d'un groupe pyridyle, chacun

desquels pouvant être substitué par un/des groupe(s) sélectionné(s) parmi le groupe constitué d'un groupe fluoro, d'un groupe chloro, d'un groupe cyano, d'un groupe nitro, d'un groupe méthyle, d'un groupe éthyle, d'un groupe trifluorométhyle, d'un groupe benzyle, d'un groupe benzyloxy, d'un groupe cyclopropyle, d'un groupe méthoxy, d'un groupe méthoxycarbonyle, d'un groupe méthylthio, d'un groupe méthylamino, d'un groupe diméthylamino, d'un groupe anilino, d'un groupe acétylamino, d'un groupe méthoxycarbonylamino, d'un groupe phényle, d'un groupe phénoxy, d'un groupe azétidinyle, d'un groupe pyrrolidinyle et d'un groupe pipéridinyle.

**8.** Composé d'aniline ou un sel pharmaceutiquement acceptable de celui-ci selon la revendication 1, dans lequel
$R^1$ représente un atome d'hydrogène, un groupe méthyle, un groupe éthyle, un groupe propyle, un groupe isopropyle ou un groupe tert-butyle,
$R^2$ and $R^3$ représentent chacun indépendamment un atome d'hydrogène ou un groupe méthyle,
$R^4$ représente un atome d'hydrogène, ou un groupe alkyle en $C_1$ à $C_6$, un groupe alkényle en $C_3$ à $C_5$, un groupe alkynyle en $C_3$ à $C_5$, un groupe cycloalkyle en $C_3$ à $C_6$, un groupe alcanoyle en $C_2$ à $C_4$, un groupe benzoyle, un groupe alkylsulfonyle en $C_1$ à $C_4$, un groupe phénylsulfonyle, un groupe aminosulfonyle, un groupe alkylaminosulfonyle en $C_1$ à $C_4$, un groupe phénylsulfamoyle, un groupe alkylaminocarbonyle en $C_1$ à $C_4$, un groupe phénylcarbamoyle, un groupe alcoxycarbonyle en $C_1$ à $C_4$, un groupe phénoxycarbonyle, un groupe phényle, un groupe thiényle, un groupe pyrazolyle, un groupe thiazolyle, un groupe pyridyle, un groupe benzofuryle, un groupe benzothiényle ou un groupe hétérocyclique complètement saturé, chacun desquels pouvant être substitué par un/des groupe(s) sélectionné(s) parmi le groupe constitué d'un groupe halogéno, d'un groupe alcoxy en $C_1$ à $C_4$, d'un groupe cycloalkyle en $C_3$ à $C_6$, d'un groupe alcoxycarbonyle en $C_1$ à $C_4$, d'un groupe alkylthio en $C_1$ à $C_4$, d'un groupe alkylsulfinyle en $C_1$ à $C_4$, d'un groupe alkylsulfonyle en $C_1$ à $C_4$, d'un groupe alkylamino en $C_1$ à $C_4$, d'un groupe di-(alkyle en $C_1$ à $C_4$)amino, d'un groupe alcanoylamino en $C_2$ à $C_4$, d'un groupe alcoxycarbonylamino en $C_1$ à $C_4$, d'un groupe aryle, d'un groupe hétéroaryle et d'un groupe hétérocyclique complètement saturé,
Z représente un groupe alkyle en $C_1$ à $C_4$, un groupe cycloalkyle en $C_3$ à $C_6$, un groupe cycloalkényle en $C_5$ à $C_6$, un groupe alkényle en $C_3$ à $C_5$, un groupe alkynyle en $C_3$ à $C_5$, un groupe hétérocyclique complètement saturé, chacun desquels pouvant être substitué par un/des groupe(s) sélectionné(s) parmi le groupe constitué d'un groupe halogéno, d'un groupe aralkyle en $C_7$ à $C_{12}$, d'un groupe alcoxy en $C_1$ à $C_4$, d'un groupe aralkyloxy en $C_7$ à $C_{12}$, d'un groupe cycloalkyle en $C_3$ à $C_6$, d'un groupe cycloalkényle en $C_5$ à $C_6$, d'un groupe arylcarbonyle, d'un groupe alkylamino en $C_1$ à $C_4$, d'un groupe di-(alkyle en $C_1$ à $C_4$)amino, d'un groupe arylamino, d'un groupe aryle, d'un groupe hétéroaryle, d'un groupe aryloxy et d'un groupe hétérocyclique complètement saturé, ou d'un groupe phényle ou d'un groupe hétéroaryle, chacun desquels pouvant être substitué par un/des groupe(s) sélectionné(s) parmi le groupe constitué d'un groupe halogéno, d'un groupe cyano, d'un groupe nitro, d'un groupe formyle, d'un groupe alkyle en $C_1$ à $C_4$, d'un groupe halogéno-alkyle en $C_1$ à $C_4$, d'un groupe aralkyle en $C_7$ à $C_{12}$, d'un groupe aralkyloxy en $C_7$ à $C_{12}$, d'un groupe cycloalkyle en $C_3$ à $C_6$, d'un groupe alcoxy en $C_1$ à $C_4$, d'un groupe alcoxycarbonyle en $C_1$ à $C_4$, d'un groupe alkylthio en $C_1$ à $C_4$, d'un groupe alkylamino en $C_1$ à $C_4$, d'un groupe di-(alkyle en $C_1$ à $C_4$) amino, d'un groupe arylamino, d'un groupe alcanoylamino en $C_2$ à $C_4$, d'un groupe alcoxycarbonylamino en $C_1$ à $C_4$, d'un groupe aryle, d'un groupe hétéroaryle, d'un groupe aryloxy et d'un groupe hétérocyclique complètement saturé.

**9.** Composé aniline ou un sel pharmaceutiquement acceptable de celui-ci selon la revendication 1, dans lequel
$R^1$ représente un atome d'hydrogène, un groupe méthyle, un groupe éthyle, un groupe propyle, un groupe isopropyle ou un groupe tert-butyle.
$R^2$ et $R^3$ représentent chacun indépendamment un atome d'hydrogène ou un groupe méthyle,
R4 représente un atome d'hydrogène, ou un groupe alkyle en $C_1$ à $C_4$, un groupe 2-propényle, un groupe 2-propynyle, un groupe cyclopropyle, un groupe acanoyle en $C_2$ à $C_4$, un groupe benzoyle, un groupe alkylsulfonyle en $C_1$ à $C_4$, un groupe alkylaminosulfonyle en $C_1$ à $C_4$, un groupe alkylaminocarbonyle en $C_1$ à $C_4$, un groupe phénylcarbamoyle, un groupe alcoxycarbonyle en $C_1$ à $C_4$, un groupe phénoxycarbonyle, un groupe phényle, un groupe thiényle, un groupe pyridyle, un groupe pyrrolidinyle ou un groupe pipéridinyle, chacun desquels pouvant être substitué par un/des groupe(s) sélectionné(s) parmi le groupe constitué d'un groupe fluoro, d'un groupe chloro, d'un groupe méthoxy, d'un groupe cyclopropyle, d'un groupe méthoxycarbonyle, d'un groupe alkylsulfonyle en $C_1$ à $C_4$, d'un groupe diméthylamino, d'un groupe alcanoylamino en $C_2$ à $C_4$, d'un groupe méthoxycarbonylamino, d'un groupe phényle, d'un groupe thiényle, d'un groupe pyridyle, d'un groupe pyrrolidinyle et d'un groupe pipéridinyle,
Z représente un groupe alkyle en $C_1$-$C_4$, un groupe 2-propynyle, un groupe 2-butynyle, un groupe cyclopropyle, un groupe cyclobutyle, un groupe cyclopentényle, chacun desquels pouvant être substitué par un/des groupe(s) sélectionné(s) parmi le groupe constitué d'un groupe fluoro, d'un groupe chloro, d'un groupe benzyle, d'un groupe méthoxy, d'un groupe benzyloxy, d'un groupe cyclopropyle, d'un groupe cyclopentényle, d'un groupe benzoyle, d'un groupe méthylamino, d'un groupe diméthylamino, d'un groupe anilino, d'un groupe phényle, d'un groupe thiényle, d'un groupe pyridyle, d'un groupe phénoxy, d'un groupe pyrrolidinyle et d'un groupe pipéridinyle, ou d'un

groupe phényle, d'un groupe thiényle, d'un groupe pyrazolyle, d'un groupe thiazolyle ou d'un groupe pyridyle, chacun desquels pouvant être substitué par un/des groupe(s) sélectionné(s) parmi le groupe constitué d'un groupe fluoro, d'un groupe chloro, d'un groupe cyano, d'un groupe nitro, d'un groupe méthyle, d'un groupe éthyle, d'un groupe trifluorométhyle, d'un groupe benzyle, d'un groupe benzyloxy, d'un groupe cyclopropyle, d'un groupe méthoxy, d'un groupe méthoxycarbonyle, d'un groupe méthylthio, d'un groupe méthylamino, d'un groupe diméthylamino, d'un groupe anilino, d'un groupe acétylamino, d'un groupe méthoxycarbonylamino, d'un groupe phényle, d'un groupe phénoxy, d'un groupe azétidinyle, d'un groupe pyrrolidinyle et d'un groupe pipéridinyle.

**10.** Composé d'aniline ou un sel pharmaceutiquement acceptable de celui-ci selon la Revendication 1, dans lequel
$R^1$ représente un atome d'hydrogène, un groupe éthyle ou un groupe isopropyle,
$R^2$ et $R^3$ représentent tous les deux des atomes d'hydrogène.
$R^4$ représente un atome d'hydrogène, un groupe trifluorométhyle, un groupe 2,2,2-trifluoroéthyle, un groupe benzyle, un groupe 1-phényléthyle, un groupe 2-phényléthyle, un groupe méthyle, un groupe éthyle, un groupe propyle, un groupe butyle, un groupe cyclopropyle, un groupe acétyle, un groupe propanoyle, un groupe butanoyle, un groupe benzoyle, un groupe méthylsulfonyle, un groupe éthylsulfonyle, un groupe propylsulfonyle, un groupe méthylaminosulfonyle, un groupe méthylaminocarbonyle, un groupe méthoxycarbonyle, un groupe phénoxycarbonyle, un groupe phényle ou un groupe pipéridinyle et
Z représente un groupe méthyle, un groupe éthyle, un groupe trifluorométhyle, un groupe 2-phényléthyle, un groupe cyclopropyle, un groupe phényle, un groupe 2-fluorophényle, un groupe 3-fluorophényle, un groupe 4-fluorophényle, un groupe 2-chlorophényle, un groupe 3-chlorophényle, un groupe 4-chlorophényle, un groupe 4-cyanophényle, un groupe 4-cyclopropylphényle, un groupe 4-méthoxyphényle, un groupe thiophen-2-yl, un groupe thiophen-3-yl, un groupe pyridin-2-yl, un groupe pyridin-3-yl ou un groupe pyridin-4-yl.

**11.** Composé d'aniline ou un sel pharmaceutiquement acceptable de celui-ci selon la Revendication 1, dans lequel
$R^1$ représente un atome d'hydrogène, un groupe méthyle, un groupe éthyle ou un groupe isopropyle,
$R^2$ et $R^3$ sont tous deux des atomes d'hydrogène,
$R^4$ représente un atome d'hydrogène, un groupe méthyle, un groupe éthyle, un groupe propyle, un groupe butyle, un groupe 2,2,2-trifluoroéthyle, un groupe benzyle, un groupe butanoyle, un groupe propylsulfonyle ou un groupe phényle, et
Z représente un groupe phényle, un groupe 4-fluorophényle, un groupe pyridin-2-yl ou un groupe pyridin-3-yl.

**12.** Composé aniline ou un sel pharmaceutiquement acceptable de celui-ci selon la Revendication 1, dans lequel le composé aniline est
acide {6-[(4-méthylaminobenzyl)(pyridin-3-ylsulfonyl)aminométhyl]pyridin-2-ylamino}-acétique,
acide [6-({4-[méthyl(propylsulfonyl)amino]benzyl}(pyridin-3-ylsulfonyl)aminométhyl)-pyridin-2-ylamino]-acétique,
acide {6-[(4-diphénylaminobenzyl)(pyridin-3-ylsulfonyl)aminométhyl]pyridin-2-ylamino}-acétique,
acide [6-({4-[diéthyl(méthyl)ammonio]benzyl}(pyridin-3 ylsulfonyl)aminométhyl)pyridin-2-ylamino]-acétique,
acide[6-({4-[butyryl(méthyl)amino]benzyl}(pyridin-3-ylsulfonyl)aminométhyl)pyridin-2-yl-amino]-acétique,
acide [6-({4-[benzyl(méthyl)amino]benzyl}(pyridin-3-ylsulfonyl)aminométhyl)pyridin-2-yl-amino]-acétique,
acide [6-({4-[butyl(méthyl)amino]benzyl}(pyridin-2-ylsulfonyl)aminométhyl)pyridin-2-ylamino]-acétique,
acide (6-{[4-(phénylamino)benzyl](pyridin-2-ylsulfonyl)aminométhy}pyridin-2-ylamonol]-acétique, ou
acide [6-({4-[butyl(2,2,2-trifluoroéthyl)amino]benzyl}(pyridin-2-ylsulfonyl)aminométhyl)-pyridin-2-ylamino]-acétique.

**13.** Composition pharmaceutique comprenant le composé aniline selon l'une quelconque des revendications 1 à 12 ou un sel pharmaceutiquement acceptable de celui-ci.

**14.** Composition pharmaceutique selon la revendication 13 destinée à la prévention ou au traitement d'une maladie respiratoire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9828264 A
- WO 9919300 A
- WO 2004078169 A
- WO 2008015517 A
- US 200387127 A
- WO 2006074884 A


### Non-patent literature cited in the description


- *American Journal of Respiratory and Critical Care Medicine,* 1999, vol. 159, 31
- *American Journal of Physiology-Lung Cellular and Molecular Physiology,* 2003, vol. 284, L599
- *Proceedings of the National Academy of Sciences of the United States of America,* 2003, vol. 100, 6736
- *Farmaco,* 1989, vol. 44, 1167
- *Journal of Molecular Structure,* 2007, vol. 829, 202
- **ABRAMOVITZ et al.** *Biochimica et Biophysica Acta,* 2000, vol. 1483, 285
- **WILSON et al.** *European Journal of Pharmacology,* 2004, vol. 501, 49